# EUROPEAN PATENT APPLICATION

(11) **EP 4 545 797 A1**
(43) Date of publication of application: **30.04.2025**
(21) Application number: 23803067.0
(22) Date of filing: 09.06.2023
(51) Int. Cl.: F04D 25/08, F04D 29/00, F04D 29/66, F04D 27/00, H02K 11/33

(54) **PORTABLE FAN**

(30) Priority: 14.06.2022 CN 202221486507 U; 25.07.2022 CN 202221950040 U; 21.10.2022 CN 202222795438 U; 02.11.2022 CN 202222928106 U; 02.11.2022 CN 202222918924 U; 09.11.2022 CN 202222989400 U; 15.11.2022 CN 202223038810 U; 19.12.2022 CN 202223413732 U; 08.02.2023 CN 202320231044 U; 16.02.2023 CN 202320295274 U; 17.04.2023 CN 202320882604 U; 17.04.2023 CN 202320958823 U
(71) Applicant: Shenzhen Jisu Technology Co., Ltd, Shenzhen, Guangdong (CN)
(72) Inventor: ZHENG, Guanzheng, Shenzhen, Guangdong (CN); XIE, Jiahang, Shenzhen, Guangdong (CN); YUAN, Shuiyong, Shenzhen, Guangdong (CN); LI, Xiangfu, Shenzhen, Guangdong (CN); GAO, Haijun, Shenzhen, Guangdong (CN); YE, Cheng, Shenzhen, Guangdong (CN); HUANG, Cunfu, Shenzhen, Guangdong (CN); ZHENG, Weijie, Shenzhen, Guangdong (CN)
(74) Representative: Herrero & Asociados, S.L.
(86) International application number: PCT/CN2023/099539
(87) International publication number: WO 2023/217299

(57) **Abstract**

A portable fan includes: a housing, defining an air inlet, a receiving cavity, an air duct, and an air outlet, which are sequentially communicated with each other. The housing includes a first side wall defining the air outlet; a fan assembly, received in the receiving cavity and configured to blow an airflow at the air inlet to flow through the air duct to reach the air outlet; and a negative-ion module, arranged in the housing. The negative-ion module includes a negative-ion generator, a positive-electrode release portion, and a negative-electrode release portion; when the negative-ion generator is operating, the positive-electrode release portion and the negative-electrode release portion discharge to a space therebetween to ionize air in the space to produce negative ions, and the negative ions are capable of being discharged out of the housing.

## Description

### TECHNICAL FIELD

The present disclosure relates to the field of fans, and in particular to a portable fan generating negative ions.

### BACKGROUND

As living standards improve, people have increased demands for air quality and wish that air in the environment can be purified. However, most portable fans on the market can blow air only and cannot purify the air. Some portable fans are arranged with a negative-ion module that generates negative ions, and the negative ions are taken to kill bacteria to purify the air.

However, for the negative-ion module in the art, a voltage from a battery is directly provided to a negative-ion generator to enable the negative-ion generator to generate the negative ions to purify the air. When the battery provides a high voltage to the negative-ion generator, electric leakage and static electricity may occur. In this case, when human or objects approach the negative-ion generator, safety issues due to electric shocks may occur.

### SUMMARY OF THE DISCLOSURE

Therefore, the present disclose provides a portable fan, arranged with a positive-electrode release portion and a negative-electrode release portion, such that a short circuit with a human body is prevented, and a micro-current is prevented. Therefore, safety performance of the portable fan is improved.

The present disclose provides a portable fan, including: a housing, defining an air inlet, a receiving cavity, an air duct, and an air outlet, wherein the air inlet, the receiving cavity, the air duct, and the air outlet are sequentially communicated with each other; and the housing comprises a first side wall defining the air outlet; a fan assembly, received in the receiving cavity and configured to blow an airflow at the air inlet to flow through the air duct to reach the air outlet; and a negative-ion module, arranged in the housing, wherein the negative-ion module comprises a negative-ion generator, a positive-electrode release portion, and a negative-electrode release portion; when the negative-ion generator is operating, the positive-electrode release portion and the negative-electrode release portion are configured to discharge to a space between the positive-electrode release portion and the negative-electrode release portion to ionize air in the space to produce negative ions, and the negative ions are capable of being discharged out of the housing.

According to the present disclosure, the negative-ion module is arranged on the portable fan and provides the additional function of sterilizing and disinfecting the air. In addition, both the positive-electrode release portion and the negative-electrode release portion are arranged. In this way, a short circuit with a human body is prevented, a micro-current is prevented, and therefore, safety performance of the portable fan is improved.

### BRIEF DESCRIPTION OF THE DRAWINGS

FIG. 1 is a perspective view of a portable fan according to a first implementation of an Embodiment 1 of the present disclosure.
FIG. 2 is a cross-sectional view of the portable fan shown in FIG. 1.
FIG. 3 is a left-side disassembled view of a first portion of the portable fan according to the first implementation of the Embodiment 1 of the present disclosure.
FIG. 4 is another disassembled view of the first portion of the portable fan according to the first implementation of the Embodiment 1 of the present disclosure.
FIG. 5 is a top-side disassembled view of a third portion of the portable fan according to the first implementation of the Embodiment 1 of the present disclosure.
FIG. 6 is a bottom-side view of the portable fan according to the first implementation of the Embodiment 1 of the present disclosure.
FIG. 7 is a bottom-side disassembled view of a support portion and the third portion of the portable fan according to the first implementation of the Embodiment 1 of the present disclosure.
FIG. 8 is a perspective view of a portable fan according to a second implementation of the Embodiment 1 of the present disclosure.
FIG. 9 is a cross-sectional view of the portable fan shown in FIG. 8.
FIG. 10 is a perspective view of a portable fan according to a third implementation of the Embodiment 1 of the present disclosure.
FIG. 11 is a cross-sectional view of the portable fan shown in FIG. 10.
FIG. 12 is a perspective view of a portable fan according to an Embodiment 2 of the present disclosure.
FIG. 13 is a left-side disassembled view of a first portion of the portable fan according to the Embodiment 2 of the present disclosure.
FIG. 14 is an enlarged view of a portion A shown in FIG. 13.
FIG. 15 is another disassembled view of the first portion of the portable fan according to the Embodiment 2 of the present disclosure.
FIG. 16 is a top-side disassembled view of the third portion of the portable fan according to the Embodiment 2 of the present disclosure.
FIG. 17 is a bottom-side view of the portable fan according to the Embodiment 2 of the present disclosure.
FIG. 18 is a bottom-side disassembled view of the support portion and the third portion of the portable fan according to the Embodiment 2 of the present disclosure.
FIG. 19 is a perspective view of a portable fan according to an Embodiment 3 of the present disclosure.
FIG. 20 is a perspective view of the portable fan, being viewed from another viewing angle, according to the Embodiment 3 of the present disclosure.
FIG. 21 is a cross-sectional view of the portable fan according to the Embodiment 3 of the present disclosure.
FIG. 22 is an enlarged view of a portion A shown in FIG. 21.
FIG. 23 is a disassembled view of the support portion according to the embodiments of the present disclosure.
FIG. 24 is a disassembled view of a portion of the portable fan according to the Embodiment 3 of the present disclosure.
FIG. 25 is a schematic view of a side wall of a clamp arm being disassembled off, according to the embodiments of the present disclosure.
FIG. 26 is a disassembled view of a portion of the portable fan according to the Embodiment 3 of the present disclosure.
FIG. 27 is a disassembled view of a portion of the portable fan according to the Embodiment 3 of the present disclosure.
FIG. 28 is a disassembled view of a rotatable connection member of the portable fan according to the Embodiment 3 of the present disclosure.
FIG. 29 is a perspective view of a portable fan according to an Embodiment 4 of the present disclosure.
FIG. 30 is a perspective view of the portable fan, being viewed from another viewing angle, according to the Embodiment 4 of the present disclosure.
FIG. 31 is a cross-sectional view of the portable fan according to the Embodiment 4 of the present disclosure.
FIG. 32 is an enlarged view of a portion A shown in FIG. 31.
FIG. 33 is a disassembled view of the support portion of the portable fan according to the Embodiment 4 of the present disclosure.
FIG. 34 is a schematic view of a second side wall of the clamp arm being disassembled off, according to the Embodiment 4 of the present disclosure.
FIG. 35 is a circuit diagram of a master chip of a master control circuit of a fan drive circuit according to a first implementation of the present disclosure.
FIG. 36 is a structural schematic view of a three-phase drive circuit of the fan drive circuit and a current detection circuit according to the first implementation of the present disclosure.
FIG. 37 is a structural schematic view of an inverted-phase electric potential detection circuit of the fan drive circuit according to the first implementation of the present disclosure.
FIG. 38 is a structural schematic view of an interface circuit of the fan drive circuit and a charge management circuit according to the first implementation of the present disclosure.
FIG. 39 is a structural schematic view of an auxiliary chip of a master control circuit of a fan drive circuit according to an Embodiment 5 of the present disclosure.
FIG. 40 is a structural schematic view of an indicator lamp branch of the fan drive circuit and a circuit of a keypad according to the Embodiment 5 of the present disclosure.
FIG. 41 is a structural schematic view of a first speed control member of the fan drive circuit according to the Embodiment 5 of the present disclosure.
FIG. 42 is a structural schematic view of a second speed control member of the fan drive circuit according to the Embodiment 5 of the present disclosure.
FIG. 43 is a structural schematic view of the master control circuit of the fan drive circuit according to the Embodiment 5 of the present disclosure.
FIG. 44 is a structural schematic view of a three-phase drive circuit of the fan drive circuit and a current detection circuit according to the Embodiment 5 of the present disclosure.
FIG. 45 is a structural schematic view of an inverted-phase electric potential detection circuit of the fan drive circuit according to the Embodiment 5 of the present disclosure.
FIG. 46 is a structural schematic view of a transistor temperature detection circuit of the fan drive circuit according to the Embodiment 5 of the present disclosure.
FIG. 47 is a structural schematic view of a battery voltage detection circuit of the fan drive circuit according to the Embodiment 5 of the present disclosure.
FIG. 48 is a schematic view of a burner interface of the fan drive circuit according to the Embodiment 5 of the present disclosure.
FIG. 49 is a schematic view of a master chip of a master control circuit of a fan drive circuit according to the Embodiment 5 of the present disclosure.
FIG. 50 is a structural schematic view of a three-phase drive circuit of the fan drive circuit and a battery voltage and current detection circuit according to the Embodiment 5 of the present disclosure.
FIG. 51 is a structural schematic view of three three-phase control chips of the master control circuit of the fan drive circuit according to the Embodiment 5 of the present disclosure.
FIG. 52 is a structural schematic view of a signal amplification circuit of the fan drive circuit according to the Embodiment 5 of the present disclosure.
FIG. 53 is a structural schematic view of the transistor temperature detection circuit of the fan drive circuit according to the Embodiment 5 of the present disclosure.
FIG. 54 is a structural schematic view of a light control circuit of the fan drive circuit according to the Embodiment 5 of the present disclosure.
FIG. 55 is a structural schematic view of a Hall detection circuit of the fan drive circuit according to the Embodiment 5 of the present disclosure.
FIG. 56 is a structural schematic view of a switch control circuit of the fan drive circuit according to the Embodiment 5 of the present disclosure.
FIG. 57 is a structural schematic view of a direct-current conversion circuit of the fan drive circuit according to the Embodiment 5 of the present disclosure.
FIG. 58 is a structural block diagram of a portable fan according to an embodiment of the present disclosure.
FIG. 59 is an exploded view of a portable fan according to an Embodiment 6 of the present disclosure.
FIG. 60 is a cross-sectional view of a portion of the portable fan according to the Embodiment 6 of the present disclosure.
FIG. 61 is a structural schematic view of a shaft tube of the portable fan according to the Embodiment 6 of the present disclosure.
FIG. 62 is a structural schematic view of an annular ring of the portable fan according to the Embodiment 6 of the present disclosure.
FIG. 63 is a structural schematic view of a disassembling member of the portable fan according to the Embodiment 6 of the present disclosure.
FIG. 64 is a structural schematic view of a housing of the portable fan according to the Embodiment 6 of the present disclosure.
FIG. 65 is a structural schematic view of another housing of the portable fan according to the Embodiment 6 of the present disclosure.
FIG. 66 is a perspective view of a portable fan according to an Embodiment 7 of the present disclosure.
FIG. 67 is a perspective view of the portable fan, being viewed from another viewing angle, according to the Embodiment 7 of the present disclosure.
FIG. 68 is a cross-sectional view of the portable fan according to the Embodiment 7 of the present disclosure.
FIG. 69 is an enlarged view of a portion A shown in FIG. 68.
FIG. 70 is a structural schematic view of a support member being disassembled off, according to the Embodiment 7 of the present disclosure.
FIG. 71 is a cross-sectional view of a portion of the portable fan according to the Embodiment 7 of the present disclosure.
FIG. 72 is a schematic view of a second side wall of a clamp arm being disassembled off, according to the Embodiment 7 of the present disclosure.
FIG. 73 is a disassembled view of a portion of the portable fan according to the Embodiment 7 of the present disclosure.
FIG. 74 is a disassembled view of a portion of the portable fan according to the Embodiment 7 of the present disclosure.
FIG. 75 is a disassembled view of a rotatable connection member of the portable fan according to the Embodiment 7 of the present disclosure.
FIG. 76 is a perspective view of a portable fan according to an Embodiment 8 of the present disclosure.
FIG. 77 is a perspective view of the portable fan, being viewed from another viewing angle, according to the Embodiment 8 of the present disclosure.
FIG. 78 is a perspective disassembled view of the portable fan according to the Embodiment 8 of the present disclosure.
FIG. 79 is a perspective view of a rotatable connection member of the portable fan according to the Embodiment 8 of the present disclosure.
FIG. 80 is a disassembled view of the rotatable connection member shown in FIG. 79.
FIG. 81 is a perspective disassembled view of the portable fan according to the Embodiment 8 of the present disclosure.
FIG. 82 is a disassembled view of the portable fan shown in FIG. 81.
FIG. 83 is an exploded view of the portable fan according to the embodiments of the present disclosure.
FIG. 84 is a cross-sectional view of a portion of the portable fan according to the embodiments of the present disclosure.
FIG. 85 is a structural schematic view of a shaft tube of the portable fan according to the embodiments of the present disclosure.
FIG. 86 is a structural schematic view of an annular ring of the portable fan according to the embodiments of the present disclosure.
FIG. 87 is a structural schematic view of a rotation plate of the portable fan according to the embodiments of the present disclosure.
FIG. 88 is a structural schematic view of a housing of the portable fan according to the embodiments of the present disclosure.
FIG. 89 is a structural schematic view of another housing of the portable fan according to the embodiments of the present disclosure.
FIG. 90 is a front view of a portable fan according to a first implementation of an Embodiment 10 of the present disclosure.
FIG. 91 is a structural schematic view of translating two clamp arms shown in FIG. 90.
FIG. 92 is a perspective view of an upper cover of a connection member, shown in FIG. 91, being moved away.
FIG. 93 is a front view of the two clamp arms, shown in FIG. 90, reaching extreme positions for approaching each other.
FIG. 94 is a front view of the two clamp arms, shown in FIG. 90, reaching extreme positions for moving away from each other.
FIG. 95 is a perspective view of one clamp arm removing a second housing and a shielding plate according to an Embodiment 10 of the present disclosure.
FIG. 96 is a perspective view of a partition plate, shown in FIG. 95, being partially removed.
FIG. 97 is a perspective view of the two shielding plates on the first housing and a support pad on the connection member being removed, according to the Embodiment 10 of the present disclosure.
FIG. 98 is a disassembled schematic view of a portion of the portable fan according to the Embodiment 10 of the present disclosure.
FIG. 99 is a cross-sectional view of the portable fan shown in FIG. 90.
FIG. 100 is a cross-sectional view of the portable fan according to the Embodiment 10 of the present disclosure.
FIG. 101 is a schematic view of a portion of a portable fan according to an Embodiment 11 of the present disclosure.
FIG. 102 is a partial circuit diagram view of the portable fan according to the Embodiment 11 of the present disclosure.
FIG. 103 is a perspective view of the portable fan according to the Embodiment 11 of the present disclosure.
FIG. 104 is an exploded view of a portion of the portable fan shown in FIG. 103.
FIG. 105 is a perspective view of a portable fan according to a first implementation of an Embodiment 12 of the present disclosure.
FIG. 106 is a cross-sectional view of a portion of the portable fan shown in FIG. 105.
FIG. 107 is a perspective view of the portable fan according to a second implementation of the Embodiment 12 of the present disclosure.
FIG. 108 is a cross-sectional view of a portion of the portable fan shown in FIG. 107.
FIG. 109 is a perspective view of the portable fan according to a third implementation of the Embodiment 12 of the present disclosure.
FIG. 110 is a cross-sectional view of a portion of the portable fan shown in FIG. 109.
FIG. 111 is a structural schematic view of a fan according to an Embodiment 13 of the present disclosure.
FIG. 112 is a structural schematic view of the fan, being viewed from another viewing angle, according to the Embodiment 13 of the present disclosure.
FIG. 113 is a structural schematic view of the fan, having partial elements omitted, according to the Embodiment 13 of the present disclosure.
FIG. 114 is a structural schematic view of the fan, having partial elements omitted, according to the Embodiment 13 of the present disclosure.
FIG. 115 is a structural schematic view of the fan, having partial elements omitted, according to the Embodiment 13 of the present disclosure.
FIG. 116 is a structural schematic view of the fan, having partial elements omitted, according to the Embodiment 13 of the present disclosure.
FIG. 117 is a cross-sectional view of a portion of the portable fan according to the Embodiment 13 of the present disclosure.
FIG. 118 is an enlarged view of a portion B shown in FIG. 117.
FIG. 119 is a cross-sectional view of a portion of the portable fan according to the Embodiment 13 of the present disclosure.
FIG. 120 is an enlarged view of a portion A shown in FIG. 119.

### DETAILED DESCRIPTION

In order to facilitate better understanding of purposes, structures, features and efficacies of the present disclosure, a portable fan of the present disclosure will be further described by referring to the accompanying drawings and specific embodiments.

### Embodiment 1

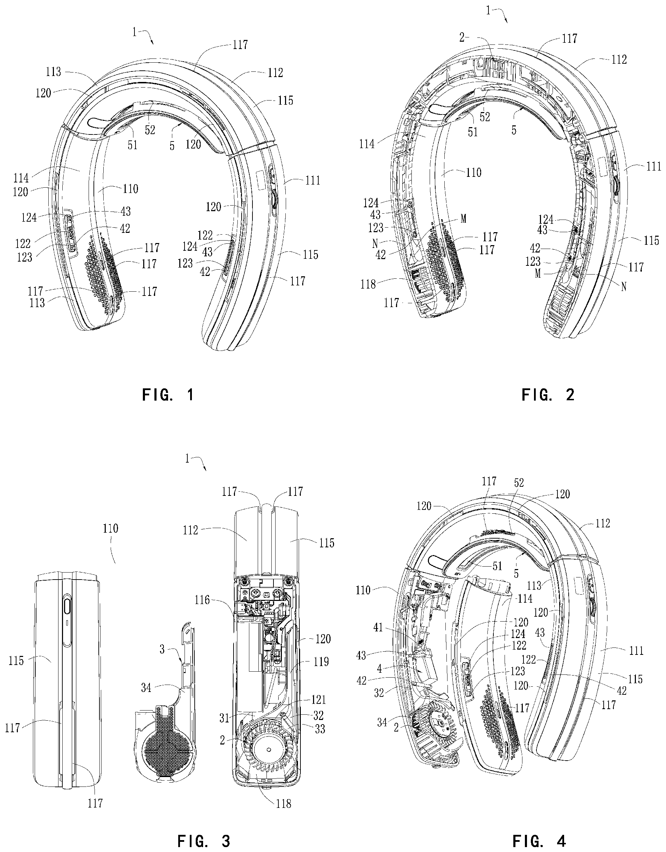

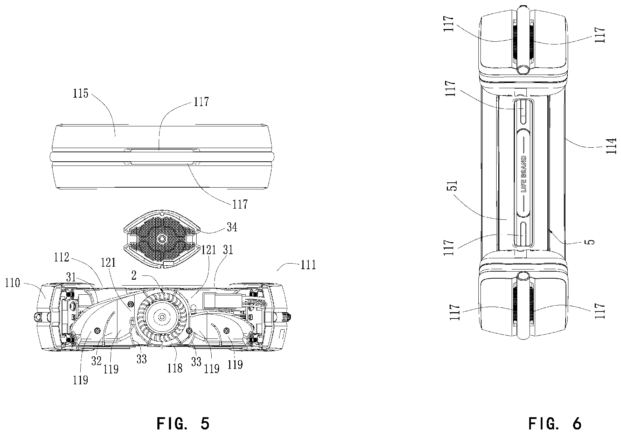

As shown in FIGS. 1 to 7, schematic views of a first implementation of a portable fan of the present disclosure are shown. The portable fan includes a housing 1, a fan assembly 2 and an negative-ion module 4. The fan assembly 2 is received in a receiving cavity 118. The negative-ion module 4 is arranged on the housing 1. The housing 1 defines, in sequence, an air inlet 117, the receiving cavity 118, an air duct 119, and an air outlet 120. The fan assembly 2 is configured to blow air from the air inlet 117 to flow along the air duct 119 to reach the air outlet 120. The negative-ion module 4 includes a negative-ion generator 41, a positive-electrode release portion 43 and a negative-electrode release portion 42. When the negative-ion generator 41 is operating, discharging occurs between the positive-electrode release portion 43 and the negative-electrode release portion 42, such that air is ionized to produce negative ions, and the negative ions are discharged out of the housing 1.

As shown in FIGS. 1 to 3, the housing 1 includes a first side wall 113 defining the air outlet 120, a second side wall 114 facing towards a neck of a user, and a third side wall 115 facing away from the neck of the user. The first side wall 113 is connected with the second side wall 114 and the third side wall 115. The housing 1 further includes a fourth side wall 116 that is opposite to the first side wall 113 and connects the second side wall 114 to the third side wall 115.

As shown in FIGS. 1 to 3, in the present embodiment, the housing 1 includes a first portion 110, a second portion 111, and a third portion 112 pivotally connected to the first portion 110 and the second portion 111. The first portion 110 and the second portion 111 are disposed symmetrically to each other. The third portion 112 is pivotally connected to the first portion 110 and the second portion 111, respectively. In this way, the first portion 110 and/or the second portion 111 may be rotated to change a size of an opening at a free end of the portable fan, enabling the portable fan to be easily worn to the neck.

As shown in FIGS. 2, 3, and 5, each of the first portion 110, the second portion 111, and the third portion 112 defines the air inlet 117, the receiving cavity 118, the air duct 119, and the air outlet 120; and the air inlet 117, the receiving cavity 118, the air duct 119, and the air outlet 120 are sequentially located and are communicated with each other. The fan assembly 2 is received in each respective receiving cavity 118. The portable fan is divided into three portions. Each of the three portions is arranged with the fan assembly 2 respectively. Each of the three portions discharges the air uniformly, such that an air flowing effect is better. In other embodiments, any two or one of the first portion 110, the second portion 111, and the third portion 112 define the air inlet 117, the receiving cavity 118, the air duct 119, and the air outlet 120.

As shown in FIGS. 2, 3 and 5, each of the receiving cavity 118 of the first portion 110 and the receiving cavity 118 of the second portion 111 are located at a respective free end. The receiving cavity 118 of the third portion 112 is located at a middle portion of the third portion. Each of two sides of the receiving cavity 118 of the third portion 112 is arranged with one respective air duct 119. Each of the first portion 110 and the second portion 111 is arranged with one respective negative-ion module 4. In other embodiments, either the first portion 110 or the second portion 111 is arranged with the negative-ion module 4. By arranging the negative-ion module 4 on the portable fan, an additional function of sterilizing and disinfecting the air is provided, and the user may have better usage experience.

As shown in FIGS. 2 to 5, in the first portion 110, each of the second side wall 114 and the third side wall 115 defines the respective air inlet 117 corresponding to the fan assembly 2. The first portion 110 further includes a partition assembly 3. The partition assembly 3 includes a longitudinal plate 31, a first cross plate 32, and a second cross plate 34. The longitudinal plate 31, the first cross plate 32, the second cross plate 34, and an inner wall of the housing 1 cooperatively define the receiving cavity 118 and the air duct 119. A first space M is defined between the first cross plate 32 and the inner wall of the housing 1. The negative-ion module 4 is received in the first space M. In the present embodiment, the first portion 110 is arranged with a recess 122 exposed outwardly at a position corresponding to the first space M. A first release port 123 and a second release port 124, which communicate the first space M to the outside environment, are defined in the recess 122. The negative-electrode release portion 42 is received in the first release port 123, and the positive-electrode release portion 43 is received in the second release port 124. By arranging both the positive-electrode release portion 43 and the negative-electrode release portion 42, a short circuit with the user is prevented, and a micro-current is prevented, and therefore, safety performance of the portable fan is improved.

As shown in FIGS. 2 to 4, in the present embodiment, the first cross plate 32 of the first portion 110 does not define a first through hole 321 communicating the air duct 119 to the first space M. Of course, in other embodiments, the first cross plate 32 may define the first through hole 321 communicating the air duct 119 with the first space M. A portion of wind generated by the fan assembly 2 is blown to flow through the first through hole 321 towards the first space M.

As shown in FIGS. 2 to 4, the first cross plate 32 of the first portion 110 includes a shielding plate 33 that extends longitudinally and abuts against the second cross plate 34. The shielding plate 33 is located between the receiving cavity 118 and the air duct 119. An end of the shielding plate 33 abuts against the first side wall 113, and an air vent 121 is defined between the other end of the shielding plate 33 and the longitudinal plate 31. The wind generated by the fan assembly 2 flows through the air vent 121 to enter the air duct 119. The air outlet 120 may be defined in a projecting area of the shielding plate 33 onto the first side wall 113. By arranging the shielding plate 33, the air vent 121 having a smaller-sized outlet is formed, such that an airflow pressure is increased, and an airflow path is further. Therefore, the airflow may flow to the air outlet 120 located further away from the fan assembly 2, and a difference in airflow strengths at air outlets 120 at various locations may be reduced. In addition, the air outlet 120 may be defined in the projecting area of the shielding plate 33 onto the first side wall 113, and therefore, a large range is available for defining the air outlet 120, a range of outputting the airflow is increased, a pressure relief effect is achieved, such that the airflow is prevented from being blocked, and noise due to airflow blockage may be reduced. The shielding plate 33 also prevents hair from entering the receiving cavity 118, danger due to hair intaken is prevented. The second portion 111 and the first portion 110 are symmetrically disposed to each other, and therefore, the second portion 111 has symmetrical structures with respect to the first portion 110 and will not be repeated herein.

As shown in FIGS. 2, 5, 6 and 7, in the third portion 112, each of the second side wall 114 and the third side wall 115 of the third portion 112 defines the air inlet 117. The receiving cavity 118 is located at a middle portion. Each of two sides of the receiving cavity 118 of the third portion 112 is arranged with one respective air duct 119. That is, the third portion 112 has two air ducts 119. The wind generated by the fan assembly 2 in the receiving cavity 118 located at the middle portion is blown to flow towards the two air ducts 119 at the two sides. Similar to the first portion 110, the third portion 112 is arranged with a longitudinal plate 31, a first cross plate 32, and a second cross plate 34. The first cross plate 32 includes a shielding plate 33 which is extending longitudinally and abuts against the second cross plate 34. Unlike the first portion 110 and the second portion 111, both ends of the shielding plate 33 in the third portion 112 abut the first side wall 113. The shielding plate 33 extends around the fan assembly 2 to form an "M" shape. The shielding plate 33 and the longitudinal plate 31 cooperatively define air vents 121 corresponding to the two air ducts 119.

As shown in FIGS. 2, 5, 6, and 7, the third portion 112 is further arranged with a support portion 5 facing towards a rear of the neck of the user. The support portion 5 defines a first notch 51 and a second notch 52. An extending direction of the first notch 51 is the same as that of the air inlet 117. An extending direction of the second notch 52 is the same as that of the air outlet 120. By defining the first notch 51 and the second notch 52, the amount of air intaken through the air inlet 117 in the second side wall 114 is ensured. In addition, air at the rear of the neck of the user is intaken into the first notch 51 and the second notch 52, such that sweat at the rear of the neck is absorbed, a better user experience is provided.

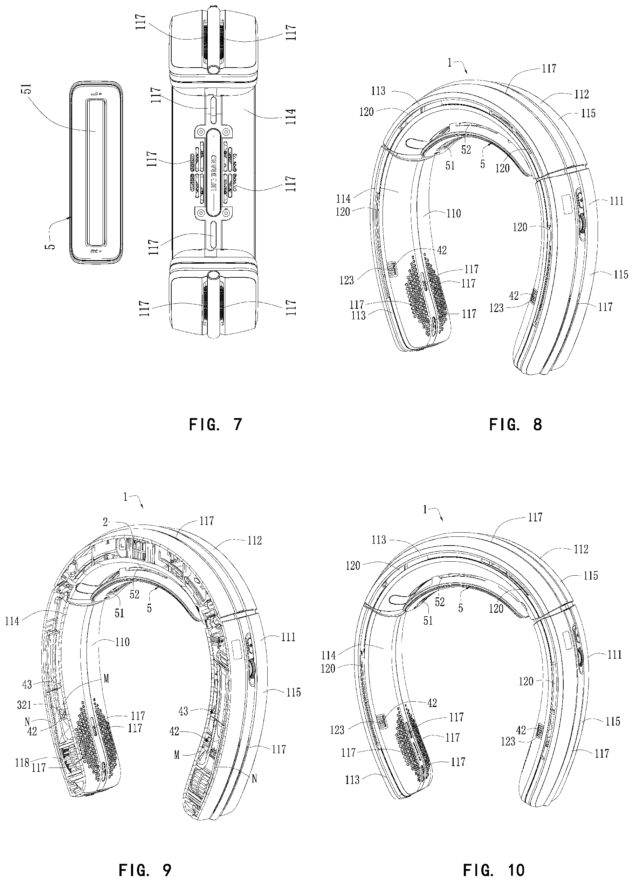

As shown in FIGS. 8 and 9, schematic views of a second implementation of the portable fan of the present disclosure are shown. In the present implementation, in the first portion 110 and the second portion 111, the first space M is formed between the first cross plate 32 and the inner wall of the housing 1, and the negative-ion module 4 is received in the first space M. The positive-electrode release portion 43 and the negative-electrode release portion 42 are both received in the first space M. The housing 1 defines the first release port 123 that communicates the first space M with the outside environment. The negative-electrode release portion 42 is received in the first release port 123. In the first space M, the positive-electrode release portion 43 and the negative-electrode release portion 42 ionize a space therebetween to produce the negative ions which are output out of the housing 1 through the first release port 123. The first cross plate 32 defines the first through hole 321, which communicates the air duct 119 with the first space M. A part of the wind generated by the fan assembly 2 is blown from the first through hole 321 to the first space M. Alternatively, the first cross plate 32 does not define the first through hole 321 communicating the air duct 119 with the first space M, and other structures and performance are essentially the same as those of the first implementation, which will not be repeated herein.

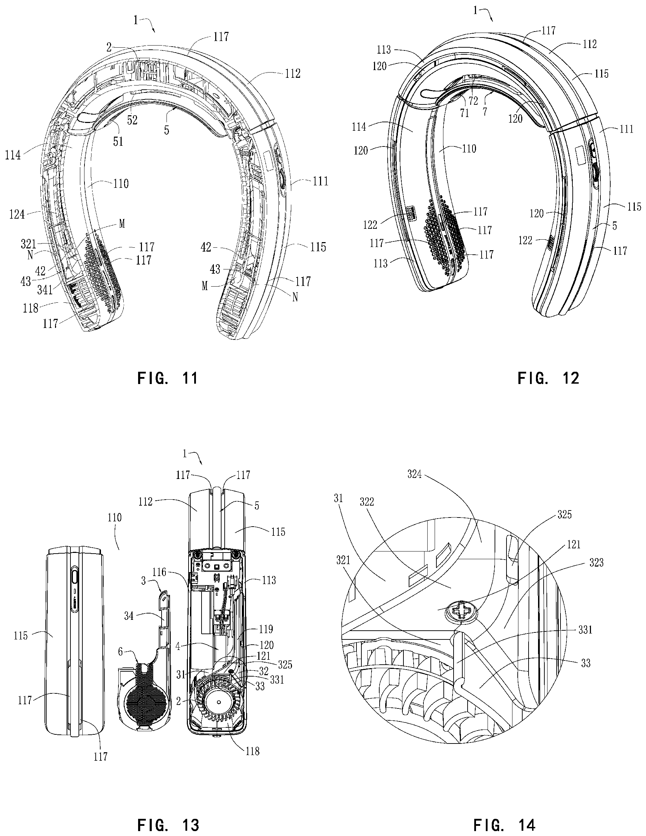

As shown in FIGS. 10 and 11, schematic views of the portable fan of a third implementation of the present disclosure are shown. In the present implementation, for the first portion 110 and the second portion 111, the first space M is defined between the first cross plate 32 and the inner wall of the housing 1, the negative-electrode release portion 42 is received in the first space M, the first cross plate 32 defines the first through hole 321 that communicates the air duct 119 with the first space M. The negative-electrode release portion 42 is disposed corresponding to the first through hole 321. A second space N is defined between the second cross plate 34 and the housing 1. The positive-electrode release portion 43 is received in the second space N. The second cross plate 34 defines a second through hole 341 that communicates the air duct 119 with the second space N. The positive-electrode release portion 43 is disposed corresponding to the second through hole 341. The first through hole 321 and the second through hole 341 are located staggered to each other in a front-to-rear direction. The positive-electrode release portion 43 and the negative-electrode release portion 42 are disposed staggered to each other in a front-to-rear direction. The positive-electrode release portion 43 and the negative-electrode release portion 42 ionize air between the first through hole 321 and the second through hole 341, i.e., ionize air in the air duct 119 to generate negative ions, and the wind of the fan assembly 2 blows the generated negative ions out of the housing. In the present embodiment, the housing 1 defines the first release port 123 that communicates the first space M with the outside environment, and the negative-electrode release portion 42 is located close to the first release port 123. A part of the wind generated by the fan assembly 2 flows through the first through hole 321 to enter the first space M to blow a part of the negative ions out of the housing through the air outlet 120. In other embodiments, the housing 1 may not define the first release port 123 that communicates the first space M with the outside environment, and all generated negative ions are output directly from the air outlet 120. Other structures and performances are essentially the same as those in the first implementation and will not be repeated herein.

### Embodiment 2

As shown in FIGS. 12 and 13, schematic views of the portable fan of the present disclosure are shown. The portable fan includes a housing 1, a fan assembly 2 arranged inside the housing 1, and a partition assembly 3 arranged inside the housing 1. In the present embodiment, the housing 1 includes a first portion 110, a second portion 111, and a third portion 112 pivotally connected to the first portion 110 and the second portion 111. The first portion 110 and the second portion 111 are disposed symmetrically to each other. The third portion 112 is pivotally connected to the first portion 110 and the second portion 111. Therefore, the first portion 110 and/or the second portion 111 may be rotated to change a size of an opening at a free end of the portable fan, and the portable fan can be easily worn to the user.

As shown in FIG. 12, FIG. 13 and FIG. 15, each of the first portion 110, the second portion 111, and the third portion 112 defines the air inlet 117, the receiving cavity 118, the air duct 119, and the air outlet 120; and the air inlet 117, the receiving cavity 118, the air duct 119, and the air outlet 120 are sequentially located and are communicated with each other. The fan assembly 2 is received in each respective receiving cavity 118. The portable fan is divided into three portions. Each of the three portions is arranged with the fan assembly 2 respectively. Each of the three portions discharges the air uniformly, such that an air flowing effect is better. In other embodiments, any two or one of the first portion 110, the second portion 111, and the third portion 112 define the air inlet 117, the receiving cavity 118, the air duct 119, and the air outlet 120.

As shown in FIGS. 12, 13 and 15, the housing 1 includes a first side wall 113 defining the air outlet 120, a second side wall 114 facing towards a neck of a user, and a third side wall 115 facing away from the neck of the user. The first side wall 113 is connected with the second side wall 114 and the third side wall 115. The housing 1 further includes a fourth side wall 116 that is opposite to the first side wall 113 and connects the second side wall 114 to the third side wall 115.

As shown in FIGS. 13 and 14, in the first portion 110, the partition assembly 3 includes a longitudinal plate 31, a first cross plate 32, and a second cross plate 34. The longitudinal plate 31, the first cross plate 32, the second cross plate 34, and an inner wall of the housing 1 cooperatively define the receiving cavity 118 and the air duct 119. The first cross plate 32 includes a shielding plate 33 that extends longitudinally and abuts against the second cross plate 34. The shielding plate 33 is located between the receiving cavity 118 and the air duct 119. An end of the shielding plate 33 abuts against the first side wall 113, and an air vent 121 is defined between the other end of the shielding plate 33 and the longitudinal plate 31. The wind generated by the fan assembly 2 flows through the air vent 121 to enter the air duct 119. The air outlet 120 may be defined in the projecting area of the shielding plate 33 onto the first side wall 113.

By arranging the shielding plate 33, the air vent 121 having a smaller-sized outlet is formed, such that an airflow pressure is increased, and an airflow path is further. Therefore, the airflow may flow to the air outlet 120 located further away from the fan assembly 2, and a difference in airflow strengths at air outlets 120 at various locations may be reduced. In addition, the air outlet 120 may be defined in the projecting area of the shielding plate 33 onto the first side wall 113, and therefore, a large range is available for defining the air outlet 120, a range of outputting the airflow is increased, a pressure relief effect is achieved, such that the airflow is prevented from being blocked, and noise due to airflow blockage may be reduced. The shielding plate 33 also prevents hair from entering the receiving cavity 118, danger due to hair intaken is prevented. The second portion 111 and the first portion 110 are symmetrically disposed to each other, and therefore, the second portion 111 has symmetrical structures with respect to the first portion 110 and will not be repeated herein.

By arranging the shielding plate 33, the air vent 121 having a smaller-sized outlet is formed, such that an airflow pressure is increased, and an airflow path is further. Therefore, the airflow may flow to the air outlet 120 located further away from the fan assembly 2, and a difference in airflow strengths at air outlets 120 at various locations may be reduced. In addition, the air outlet 120 may be defined in the projecting area of the shielding plate 33 onto the first side wall 113, and therefore, a large range is available for defining the air outlet 120, a range of outputting the airflow is increased, a pressure relief effect is achieved, such that the airflow is prevented from being blocked, and noise due to airflow blockage may be reduced. The shielding plate 33 also prevents hair from entering the receiving cavity 118, danger due to hair intaken is prevented.

As shown in FIGS. 13 and 14, in the first portion 110, the first cross plate 32 includes a first flat plate portion 321 received in the receiving cavity 118, a first inclined surface 322 received in the air duct 119, a second inclined surface 323 received in the air duct 119, and a second flat plate portion 324 received in the air duct 119. The second inclined surface 323 is connected to both the first inclined surface 322 and the second flat plate portion 324. The second inclined surface 323 is disposed adjacent to the air outlet 120. The shielding plate 33 is disposed between the first flat plate portion 321 and the second inclined surface 323. The shielding plate 33 includes a curved portion 331 located at the free end, and an end of the curved portion 331 is disposed on the second inclined surface 323. An inner diameter of the curved portion 331 is in a range of 0.25mm to 1.25 mm. The curved portion 331 is located at an end of the free end of the shielding portion 33, and that is, the curved portion 331 is located at the air outlet 121. A part of the airflow is affected by the Coanda Effect generated by the curved portion 331 flows out of the housing through the air outlet 120 defined in the projecting area of the shielding plate 33 on the first side wall 113. The curved portion 331 is arranged to further reduce airflow noise. It is understood that, within a certain value range, as the inner diameter of the curved portion 331 increases, the curved portion 331 has a stronger noise reduction capability.

As shown in FIGS. 13 and 14, in the first portion 110, the wind generated by the fan assembly 2 flows through the air vent 121 to enter the first inclined surface 322, and subsequently, enters the second flat plate portion 324, along which a cross-sectional area of the air duct 119 decreases. In addition, the longitudinal plate 31 extends curvedly. In a direction extending from the air vent 121 towards the air duct 119, a spacing between the longitudinal plate 31 and the first side wall 113 is decreasing until a certain spacing is reached, and the certain spacing is maintained. Therefore, the cross-sectional area of the air duct 119 decreases. Since the cross-sectional area of the air duct 119 decreases in various dimensions, the airflow pressure increases, the airflow strength increases, the airflow path is longer, such that the airflow outputting effect is better at the air outlet 120, and a better user experience is provided.

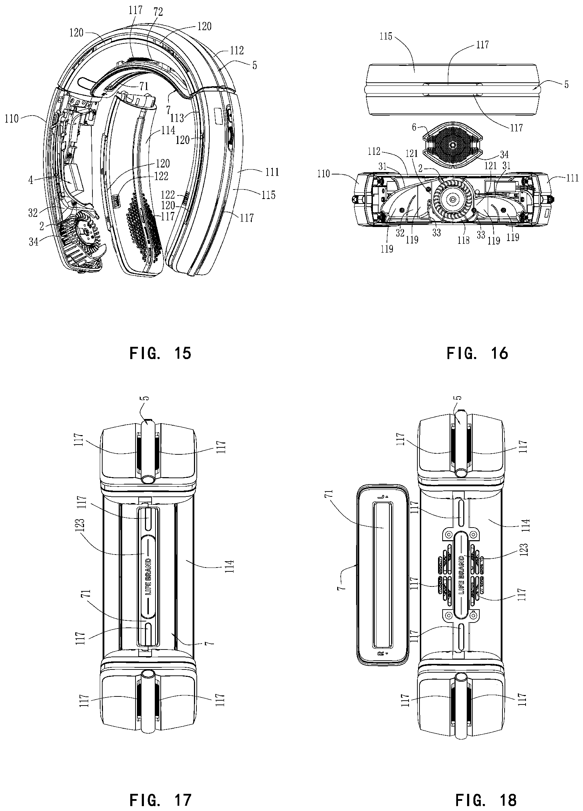

As shown in FIGS. 12 to 14, in the first portion 110, each of the second side wall 114 and the third side wall 115 defines the respective air inlet 117 corresponding to the fan assembly 2. As shown in FIG. 15, a negative-ion cavity is defined between the first cross plate 32 and the second side wall 114. The first side wall 113 defines a release port 122 communicated with the negative-ion cavity. The negative-ion generator 4 is arranged inside the housing 1. A release end of the negative-ion generator 4 directly faces the release port 122. The release port 122 is disposed adjacent to and side-by-side with the air outlet 120. In the present embodiment, the first cross plate 32 defines a through hole 325 communicated with the negative-ion cavity. A part of the wind generated by the fan assembly 2 flows through the through hole 325 to reach the release end of the negative-ion generator 4 and flows out of the housing through the release port 122. It is understood that, in other embodiments, the first cross plate 32 may not define the through hole 325 communicated with the negative-ion cavity. The release end of the negative-ion generator 4 is disposed close to or faces towards the release port 122. The negative ions released from the release end are output out of the housing directly from the release port 122.

As shown in FIG. 13, in the first portion 110, a light strip 5 is embedded in the third side wall 115. The light strip 5 and the third side wall 115 extend along a same direction. The air inlet 117 defined in the third side wall 115 is disposed on two sides of the light strip 5. The second cross plate 34 is arranged with a filter member 6 that faces towards the air inlet 117 defined in the third side wall 115. The filter member 6 defines a plurality of small holes. The filter member 6 prevents some impurities from entering the receiving cavity 118, such that performance of the fan assembly 2 is prevented from being affected. The second portion 111 is arranged symmetrically with the first portion 110 and has structures substantially the same as the first portion 110, which will not be repeated herein.

As shown in FIG. 16, in the third portion 112, the receiving cavity 118 is located at the middle portion. Each of two sides of the receiving cavity 118 of the third portion 112 is arranged with one respective air duct 119. That is, the third portion 112 has two air ducts 119. The wind generated by the fan assembly 2 in the receiving cavity 118 located at the middle portion flows towards the two air ducts 119 at the two sides. Similar to the first portion 110 and the second portion 111, the third portion 112 is arranged with a longitudinal plate 31, a first cross plate 32, and a second cross plate 34. The first cross plate 32 includes a shielding plate 33 which is extending longitudinally and abuts against the second cross plate 34. Unlike the first portion 110 and the second portion 111, both ends of the shielding plate 33 in the third portion 112 abut the first side wall 113. The shielding plate 33 extends around the fan assembly 2 to form an "M" shape. The shielding plate 33 and the longitudinal plate 31 cooperatively define two air vents 121 corresponding to the two air ducts 119 respectively.

As shown in FIGS. 16 to 18, in the present embodiment, the third portion 112 is not arranged with the negative-ion generator 4. An air intaking cavity is defined between the first cross plate 32 and the second side wall 114. The third portion 112 is further arranged with a support portion 7 facing towards the rear of the neck of the user. The support portion 7 defines a first notch 71. A center of the second side wall 114 is arranged with a logo portion 123 that is exposed from the first notch 71 to enhance an appearance of the portable fan and create a brand image. The logo portion 123 is surrounded by the air inlet 117 to increase the amount of air intaken into the housing. A part of the air inlet 117 is exposed from the first notch 71. Specifically, a portion of the air inlet 117 disposed close to the fan assembly 2 is covered by the support portion 7, and a portion of the air inlet 117 disposed further away from the fan assembly 2 is exposed from the first notch 71. The air at the rear of the neck of the user may be intaken into the housing through the first notch 71 and the portion of the air inlet 117 exposed from the first notch 71, such that sweat at the rear of the neck can be absorbed, and a better user experience is provided. In addition, further as shown in FIGS. 12 and 15, the support portion 7 further defines a second notch 72 extending parallel to the air outlet 120. By defining the first notch 71 and the second notch 72, the amount of air intaken through the air inlet 117 in the second side wall 114 is ensured. Of course, in other embodiments, the third portion 112 may be arranged with the negative-ion generator 4, and correspondingly, the negative-ion cavity is defined between the first cross plate 32 and the second side wall 114.

As shown in FIGS. 12 and 16, similar to the first portion 110 and the second portion 111, in the third portion 112, a light strip 5 is embedded in the third side wall 115. The light strip 5 and the third side wall 115 extend in the same direction. The air inlet 117 defined in the third side wall 115 is disposed on two sides of the light strip 5. The second cross panel 34 is arranged with a filter member 6 that faces the air inlet 117 defined in the third side wall 115, and the filter member 6 defines a plurality of small holes.

### Embodiment 3

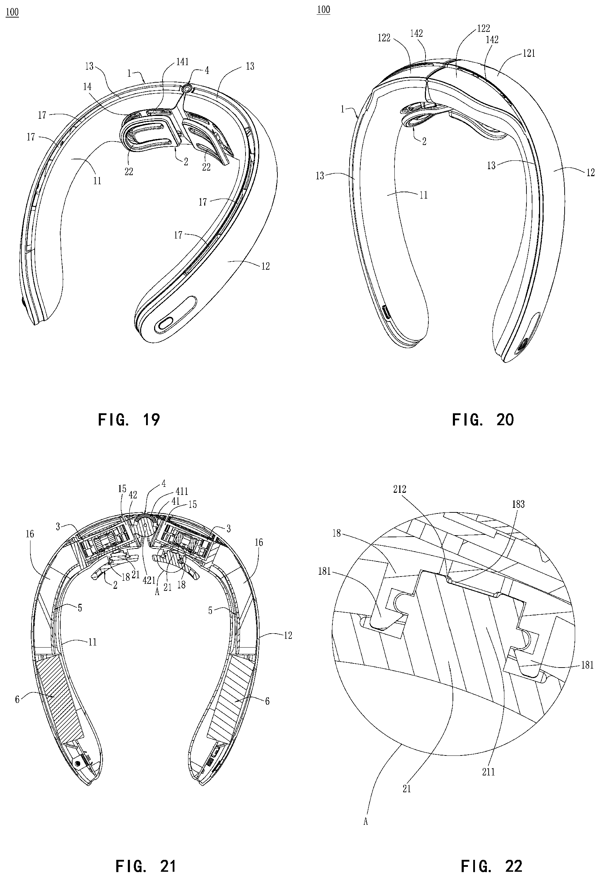

As shown in FIGS. 19 to 21, schematic views of the portable fan 100 of the present disclosure are shown. The portable fan 100 includes a housing 1, a support member 2 and a fan assembly 3. The support member 2 is mounted on a side of the housing 1 facing the neck of the user. The support member 2 is rotatably connected to the housing 1 and is supported to the rear of the neck of the user. The fan assembly 3 is arranged inside the housing 1. By arranging the support member 2 to be supported to the rear of the neck, the housing 1 is prevented from rubbing the neck. In addition, the support member 2 supports the housing 1 and the rear of the neck of the user, allowing a spacing to be defined between the housing 1 and the rear of the neck, such that a contact area between the housing 1 and the neck is reduced, and a cooling effect of the portable fan 100 is improved.

As shown in FIGS. 19 to 21, the housing 1 includes a first side wall 11 facing towards the neck of the user and a second side wall 12 facing away from the neck of the user. A first mating portion 18 is arranged on and protrudes from the first side wall 11. The support member 2 is arranged with a second mating portion 21 corresponding to the first mating portion 18. The second mating portion 21 is rotatably connected to the first mating portion 18. The support member 2 is rotatable relative to the first side wall 11.

As shown in FIGS. 21 to 23, in the present embodiment, the first mating portion 18 includes two pivot portions 181 and an elastic arm 182 disposed between the two pivot portions 181. A protrusion 183 is arranged at a free end of the elastic arm 182. The second mating portion 21 includes a pivot column 211. A plurality of recesses 212 are defined in a surface of the pivot column 211. Two ends of the pivot column 211 are respectively pivotally connected to the two pivot portions 181. When the support member 2 is rotating upwardly and downwardly, the protrusion 183 is received in any one of the plurality of recesses 212 in order to position the support member 2 at various angles.

As shown in FIGS. 19 to 21, the housing 1 includes two clamp arms 13 that are disposed symmetrically to each other. The two the clamp arms 13 are rotatably connected to each other by a rotation connection member 4. A pivot angle of each clamp arm 13 is in a range of ±10°. The two clamp arms 13 are rotatably connected to each other to allow the user to adjust a distance between free ends of the two clamp arms 13, such that the portable fan can be worn to the neck and fixed. Each clamp arm 13 defines sequentially an air inlet 14, a receiving cavity 15, an air duct 16, and an air outlet 17, and the air inlet 14, the receiving cavity 15, the air duct 16, and the air outlet 17 are communicated to each other. The fan assembly 3 is received in the receiving cavity 15 to drive an airflow to flow from the air inlet 14 to flow through the air duct 16 to reach the air outlet 17.

As shown in FIGS. 19 to 21, the portable fan 100 is arranged with two support members 2 corresponding to the two clamp arms 13, respectively. When the two clamp arms 13 are pivoted, positions of the two support members 2 in contact with the user are changed. However, since the support members 2 are rotatably connected to the clamp arms 13, each support member 2 can be adjusted independently to be attached to the neck of the user, such that wearing comfort is improved.

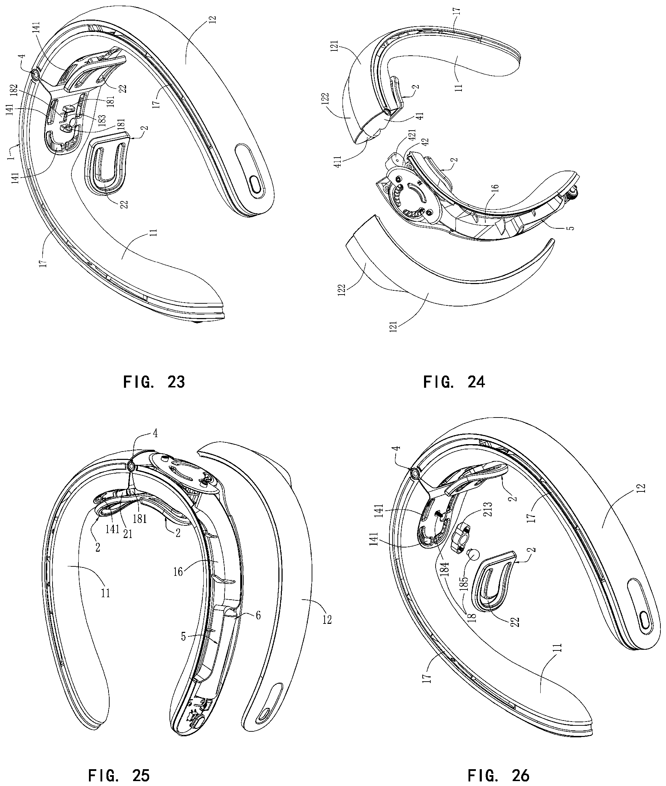

As shown in FIGS. 19 to 21, corresponding to each of fan assembly 3, each clamp arm 13 defines a first air inlet 141 defined in the first side wall 11 and a second air inlet 142 defined in the second side wall 12. The receiving cavity 15 is located at two ends of the two clamp arms 13 that are connected to each other. The support member 2 is disposed corresponding to the receiving cavity 15. A spacing is defined between at least a portion of the support member 2 and the first air inlet 141. Therefore, when the portable fan 100 is in use, the support member 2 does not affect air intaken through the first air inlet 141. Moreover, as shown in FIG. 23, the support member 2 defines a through hole 22 corresponding to the first air inlet 141, such that sweat, generated by contact between the support member 2 and the neck, can be absorbed and evaporated in time, ensuring that the neck of the user is dry and fresh, and a wearing experience is improved.

As shown in FIGS. 20 and 24, the second side wall 12 includes a main wall 121 and an auxiliary wall 122. The main wall 121 and the auxiliary wall 122 are arranged in a stepped manner. The second air inlet 142 is formed between the main wall 121 and the auxiliary wall 122. The second air inlet 142 is hiddenly formed between the main wall 121 and the auxiliary wall 122, and therefore, an aesthetic appearance is provided, and the hair of the user may not enter the second air inlet 142 easily, safety of the portable fan is improved. The main wall 121 is longer than the auxiliary wall 122. When the user is wearing the portable fan 100, the main wall 121 is located above the auxiliary wall 122, and the auxiliary wall 122 is located a radially inner side of the main wall 121. In this way, the second air inlet 142 defined in the second side wall 12 that faces away from the neck can be better hidden, and an opening of the second air inlet 142 faces downwards, such that the hair of the user may not be intaken into the air inlet easily. A cover plate having a through hole is disposed between the second air inlet 142 and the fan assembly 3. The cover plate having the through hole further prevents the hair from being sucked into the fan by the fan assembly 3.

As shown in FIGS. 21 and 24, the rotation connection member 4 includes a first connection member 41 and a second connection member 42. The first connection member 41 is fixedly connected to one of the two clamp arms 13, and the second connection member 42 is fixedly connected to the other one of the two clamp arms 13. The first connection member 41 includes a first fixation portion 411, and the second connection member 42 includes a second fixation portion 421. The first fixation portion 411 and the second fixation portion 421 are rotatably connected to each other. The first fixation portion 411 and the second fixation portion 421 cooperatively form a spindle shape. The two the clamp arms 13, during rotating, rotate along the first fixation portion 411 and the second fixation portion 421 that are configured in the spindle shape, such that an aesthetic appearance is provided.

As shown in FIGS. 21, 24 and 25, a partition assembly 5 is further arranged. The partition assembly 5 separates the receiving cavity 15 and the air duct 16, which are communicated with each other, from the other space inside the housing 1. A width of an end of the air duct 16 away from the receiving cavity 15 is less than a width of an end of the air duct 16 near the receiving cavity 15, such that a space is reserved to receive a battery 6. In the present embodiment, the battery 6 is arranged inside each of the two clamp arms 13, and two fan assemblies 3 may operate simultaneously or separately. The air outlet 17 is formed at and extends through a side of the air duct 16 and extends substantially to cover the entirety of the air duct 16. The width of the end of the air duct 16 away from the receiving cavity 15 is narrower, such that the air may be gathered easily at the narrower end, and various portions of the air outlet 17 may have an even airflow.

As shown in FIG. 26, a schematic view of the portable fan 100 of the present disclosure is shown. In the present embodiment, the second mating portion 21 includes a mounting bracket 213. The first mating portion 18 includes a fixation member 184 secured to the first side wall 11 and a rotation ball 185 that extends through the mounting bracket 213 and is secured to the fixation member 184. The mounting bracket 213 is rotated with respect to the rotation ball 185 to enable the support member 2 to rotate with respect to the rotation ball 185. The support member 2 may rotate up and down. In the present embodiment, the support member 2 may rotate spherically, such that the portable fan can be worn comfortably. Other structures and performance of the present embodiment are substantially the same as those in the above embodiments, and will not be repeated herein.

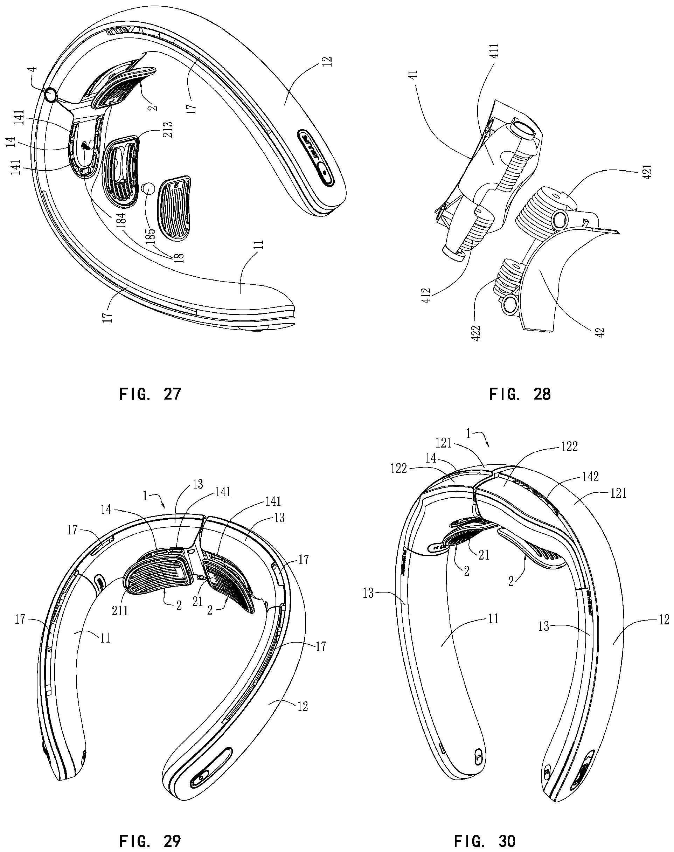

As shown in FIGS. 27 and 28, schematic views of the portable fan 100 of the present disclosure are shown. In the present embodiment, a shape of the mounting bracket 213 matches a shape of the second mating portion 21. In the rotation connection member 4, the first fixation portion 411 includes a plurality of first pieces 412, the second fixation portion 421 includes a plurality of second pieces 422. The plurality of the first pieces 412 and the plurality of the second pieces 422 are laminated and arranged alternately with each other. In this way, friction between the first fixation portion 411 and the second fixation portion 421 is greatly increased, such that, during rotating, the two clamp arms 13 may stop at any position, safety performance is improved. Other structures and performance of the present embodiment are substantially the same as those in the above embodiments, and will not be repeated herein.

It is understood that, in addition to the above embodiment, in other embodiments, the support member 2 is connected to the housing 1 by a bendable positioning member. The bendable positioning member may allow the support member 2 to be better attached to the rear of the neck.

### Embodiment 4

As shown in FIGS. 29 to 32, schematic views of the portable fan of the present disclosure are shown. The portable fan includes a housing 1, a support member 2, a cushion member 3, and a fan assembly 4. The housing 1 is worn around the neck of the user. The housing 1 defines sequentially an air inlet 14, a receiving cavity 15, an air duct 16, and an air outlet 17; and the air inlet 14, the receiving cavity 15, the air duct 16, and the air outlet 17 are sequentially communicated to each other. The housing 1 is in a "C" shape. The housing 1 includes a rear neck section located at a middle of the housing. The receiving cavity 15 is defined in the rear neck section. The rear neck section is arranged with a mounting portion 18 at a position corresponding to the receiving cavity 15 and facing towards the rear of the neck. The support member 2 is mounted on the mounting portion 18 and is configured to support the rear neck of the user. The cushion member 3 is arranged inside the mounting portion 18 and abuts against the support member 2. The cushion member 3 may be elastically deformable. The fan assembly 4 is received in the receiving cavity 15 and is configured to drive the air to flow from the air inlet 14 through the air duct 16 to reach the air outlet 17.

By arranging the support member 2 to support the rear of the neck, the housing of the portable fan is prevented from directly touching the rear of the neck. In addition, the support member 2 is supported between the housing 1 to be away from the rear of the neck to allow a spacing to be defined between the housing 1 and the rear of the neck. In this way, a contact area between the housing 1 and the rear of the neck is reduced, a cooling effect of the portable fan is improved. The cushion member 3 is arranged in the mounting portion 18 to abut against the support member 2. Since the cushion member 3 is elastically deformable, the support member 2 is more flexible and is movable to be attached to necks of various users.

As shown in FIGS. 30 to 33, the housing 1 includes a first side wall 11 facing towards the neck of the user and a second side wall 12 facing away from the neck of the user. The first side wall 11 is arranged with the mounting portion 18. The support member 2 includes a contact portion 21 and a connection member 22. The contact portion 21 having a curved surface to be better attached to the neck. The connection member 22 includes a first mating portion 221 mating with the contact portion 21 and a second mating portion 222 mating with the mounting portion 18. Two second mating portions 222 are arranged. When the two second mating portions 222 are pressed, the two second mating portions 222 are moved close to each other to extend into or move out of the mounting portion 18. When pressing on the two second mating portions 222 is released, the two second mating portions 222 are secured to the mounting portion 18 or moved out of the mounting portion 18.

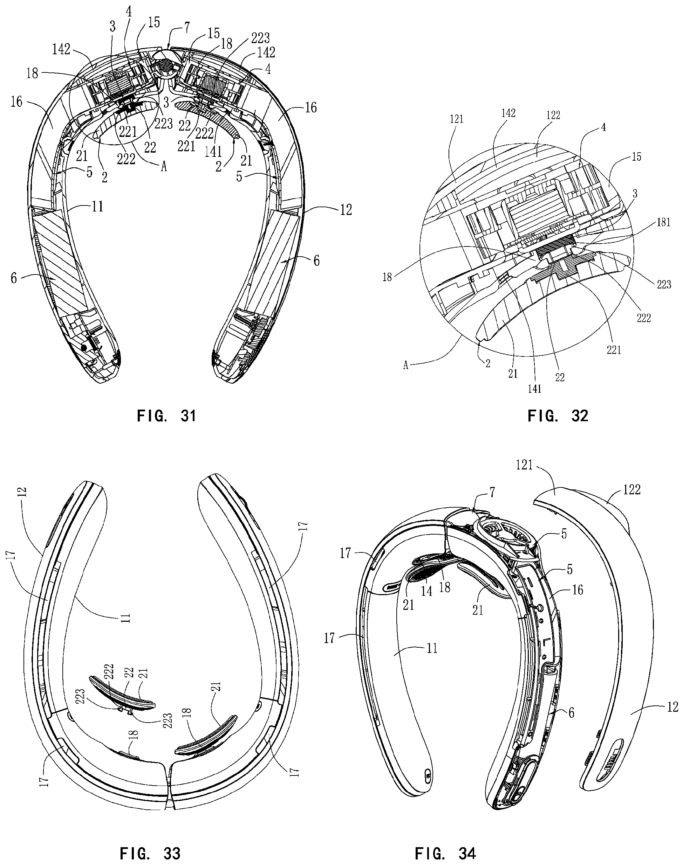

As shown in FIGS. 31 to 33, the mounting portion 18 includes a mounting cavity 181, the second mating portion 222 includes a hook portion 223 that enters the mounting cavity 181 and hooks with the mounting cavity 181. The cushion member 3 abuts against the hook portion 223. A height of the hook portion 223 and the cushion member 3 are greater than or equal to a height of the mounting cavity 181. In this way, the support member 2 may not be loosely shaken. The cushion member 3 is elastically deformable, and in the present embodiment, the cushion member 3 is made of silicone, and a size of the cushion member 3 may be compressed to be 15% to 25% of its original size. Therefore, the support member 2 is more flexible and can be better movable to be attached to necks of various users. Of course, in other embodiments, the cushion member 3 may be made of other materials having an elastic deformation capability. A spacing is formed between a surface of the first mating portion 221 facing towards the mounting portion 18 and the mounting portion 18, and the support member 2 may be flexibly rotated in all directions. Moreover, a mounting tool can access the spacing, such that the support member 2 can be mounted easily.

In the present embodiment, the support member 2 may be made of silicone. Properties of the silicone allow the support member 2 to be deformable, such that the support member 2 can be better attached to the neck. In addition, the silicone is more comfortable when being worn to the user, a better user experience is provided.

As shown in FIGS. 29 to 31, the housing 1 includes two clamp arms 13 that are disposed symmetrically to each other. The two clamp arms 13 are rotatably connected to each other by a rotation connection member 722. A pivot angle of each clamp arm 13 is in a range of ±10°. The two clamp arms 13 are rotatably connected to each other to allow the user to adjust a distance between free ends of the two clamp arms 13, such that the portable fan can be worn to the neck and fixed. Each clamp arm 13 defines sequentially an air inlet 14, a receiving cavity 15, an air duct 16, and an air outlet 17, and the air inlet 14, the receiving cavity 15, the air duct 16, and the air outlet 17 are communicated to each other. Two receiving cavities 15 are located close to each other and are both located at the rear neck section.

As shown in FIGS. 31 and 33, the portable fan is arranged with two support members 2 corresponding to the two clamp arms 13. When the two clamp arms 13 are pivoted, positions of the two support members 2 in contact with the user changes accordingly. However, since the cushion member 3 is arranged in the mounting cavity 181 to abut against the two support members 2, the two support members 2 can still be respectively attached well to the neck, and the portable fan can be worn comfortably.

As shown in FIGS. 30 to 32, corresponding to each fan assembly 4, each of the two clamp arms 13 defines a first air inlet 141 defined in the first side wall 11 and a second air inlet 142 defined in the second side wall 12. The receiving cavity 15 is located at two ends of the two clamp arms 13 that are connected to each other. The support member 2 is disposed at a position corresponding to the receiving cavity 15. A spacing is defined between at least a portion of the support member 2 and the first air inlet 141. Therefore, when the portable fan is in use, the support member 2 does not affect the air intaken through the first air inlet 141. Moreover, as shown in the drawings, the contact portion 21 of the support member 2 defines a through hole 211 corresponding to the first air inlet 141. In this way, the sweat, generated due to the contact portion 21 in contact with the neck, can be absorbed and evaporated in time, ensuring that the neck is dry and fresh, and a wearing experience is improved.

As shown in FIGS. 30 to 32, the second side wall 12 includes a main wall 121 and an auxiliary wall 122. The main wall 121 and the auxiliary wall 122 are arranged in a stepped manner. The second air inlet 142 is formed between the main wall 121 and the auxiliary wall 122. The second air inlet 142 is hiddenly formed between the main wall 121 and the auxiliary wall 122, and therefore, an aesthetic appearance is provided, and the hair of the user may not enter the second air inlet 142 easily, safety of the portable fan is improved.

As shown in FIGS. 31 and 34, a partition assembly 5 is further arranged. The partition assembly 5 separates the receiving cavity 15 and the air duct 16, which are communicated with each other, from the other space inside the housing 1. A width of an end of the air duct 16 away from the receiving cavity 15 is less than a width of an end of the air duct 16 near the receiving cavity 15, such that a space is reserved to receive a battery 6. In the present embodiment, the battery 6 is arranged inside each of the two clamp arms 13, and two fan assemblies 3 may operate simultaneously or separately. The air outlet 17 is formed at and extends through a side of the air duct 16 and extends substantially to cover the entirety of the air duct 16. The width of the end of the air duct 16 away from the receiving cavity 15 is narrower, such that the air may be gathered easily at the narrower end, and various portions of the air outlet 17 may have an even airflow.

### Embodiment 5

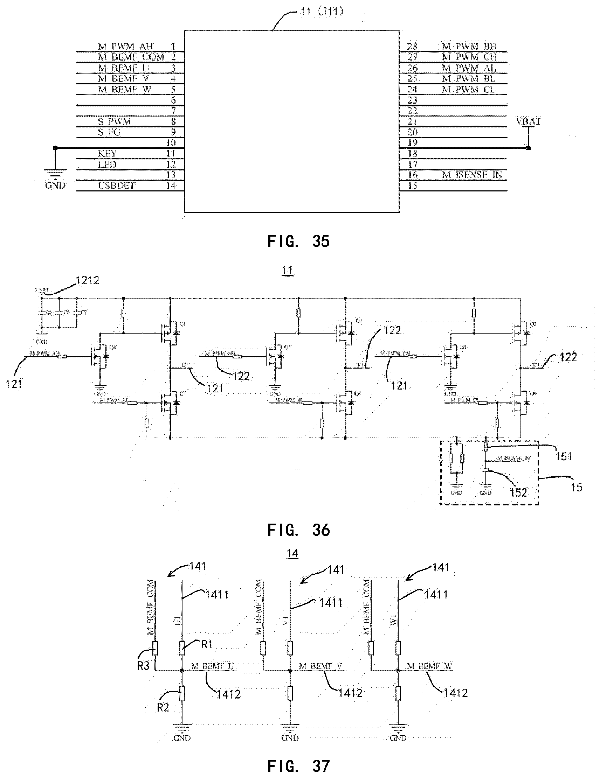

As shown in FIGS. 35 to 37, in a first implementation, a fan drive circuit is provided. The fan drive circuit is applicable for various types of fans. Specifically, the fan drive circuit includes: a master control circuit 11, a three-phase drive circuit 12, and an inverted-phase electric potential detection circuit 14.

The three-phase drive circuit 12 includes at least three signal input ends 121 and three drive signal output ends 122. Each of the at least three signal input ends 121 is electrically connected to the master control circuit 11 to receive control signals. The three drive signal output ends 122 are electrically connect to three signal ends (U, V, and W) of a direct-current (DC) brushless fan motor to output a three-phase drive signal to drive the DC brushless fan motor to rotate. The inverted-phase electric potential detection circuit 14 includes three detection branches 141. Each detection branch 141 includes a detection end 1411 and a detection output end 1412 electrically connected to the detection end. Three detection ends 1411 of the three detection branches 141 are respectively electrically connected to the three drive signal output ends 122. Three detection output ends 1412 of the three detection branches 141 are electrically connected to the master control circuit 11 to respectively output a first detection signal, a second detection signal, and a third detection signal. In this way, the master control circuit 11 is informed of a phase of the three-phase drive signal based on the first detection signal, the second detection signal, and the third detection signal to adjust the control signals.

As shown in FIG. 37, the detection branch 141 includes a first detection resistor R1, a second detection resistor R2, and a third detection resistor R3. The first detection resistor R1 and the second detection resistor are connected to each other in series. An end of the first detection resistor R1 away from the second detection resistor R2 is the detection end 1411, and an end of the second detection resistor R2 away from the first detection resistor R1 is grounded. A node between the first detection resistor R1 and the second detection resistor R2 is the detection output end 1412.

By arranging the three-phase drive circuit 12, energy-saving performance and control performance of the fan motor are improved, and a service life of the fan drive circuit and the portable fan is extended. By arranging the inverted-phase electric potential detection circuit 14, the master control circuit 11 may be easily informed of the phase of the DC brushless fan motor, such that the master control circuit 11 may send corresponding control signals to the three-phase drive circuit 12 to control driving of the DC brushless fan motor, and reliability and stability of the driving is improved.

As shown in FIG. 36, the three-phase drive circuit 12 includes a first transistor Q1, a second transistor Q2, a third transistor Q3, a fourth transistor Q4, a fifth transistor Q5, a sixth transistor Q6, a seventh transistor Q7, an eighth transistor Q8, and a ninth transistor Q9. A first conductive end 1211 of the first transistor Q1, a first conductive end 1211 of the second transistor Q2, and a first conductive end 1211 of the third transistor Q3 are connected to a power supply end 1212. A first conductive end 1211 of the fourth transistor Q4 is connected to the power supply end 1212. A first conductive end 1211 of the fifth transistor Q5 is connected to the power supply end 1212. A first conduction end 1211 of the sixth transistor Q6 is connected to the power supply end 1212. A control end of the fourth transistor Q4, a control end of the fifth transistor Q5, and a control end of the sixth transistor Q6 are electrically connected to the master control circuit 11. A control end of the seventh transistor Q7 is electrically connected to the control end of the fourth transistor Q4, a control end of the eighth transistor Q8 is electrically connected to the control end of the fifth transistor Q5, and a control end of the ninth transistor Q9 is electrically connected to the control end of the sixth transistor Q6, such that the control signals are received. Second conduction ends 1213 of the fourth transistor Q4, the fifth transistor Q5, and the sixth transistor Q6 are grounded. A first conductive end 1211 of the seventh transistor Q7 is connected to a second conductive end 1213 of the first transistor Q1. A second conductive end 1213 of the seventh transistor Q7 is grounded. A first conductive end 1211 of the eighth transistor Q8 is connected to a second conductive end 1213 of the second transistor Q2. A second conductive end 1213 of the eighth transistor Q8 is grounded. A first conductive end 1211 of the ninth transistor Q9 is connected to the second conductive end 1213 of the third transistor Q3. A second conductive end 1213 of the ninth transistor Q9 is grounded. A node between the first conductive 1211 of the seventh transistor Q7 and the second conductive 1213 of the first transistor Q1, a node between the first conductive 1211 of the eighth transistor Q8 and the second conductive 1213 of the second transistor Q2, and a node between the first conductive 1211 of the ninth transistor Q9 and the second conductive 1213 of the third transistor Q3 respectively serve as the three drive signal output ends 122. The at least three signal input ends 121 are three PWM signal input ends. The control signals include three PWM signals.

As shown in FIG. 36, the fan drive circuit further includes a current detection circuit 15. The second conductive ends 1213 of the seventh transistor Q7, the eighth transistor Q8, and the ninth transistor Q9 are all grounded via the current detection circuit 15. The current detection circuit 15 is electrically connected to the master control circuit 11. The current detection circuit 15 includes a sense resistor 151 and a sense capacitor 152. The second conductive ends 1213 of the seventh transistor Q7, the eighth transistor Q8, and the ninth transistor Q9 are grounded via the sense resistor 151 and the sense capacitor 152 sequentially. A node between the sense resistor 151 and the sense capacitor 152 is electrically connected to the master control circuit 11. By arranging the current detection circuit 15, when a current is abnormal, the master control circuit 11 may control the fan drive circuit to stop operating or to operate at a lower power, such that an overcurrent protection is provided for the fan drive circuit, and reliability and the service life of the fan drive circuit are improved.

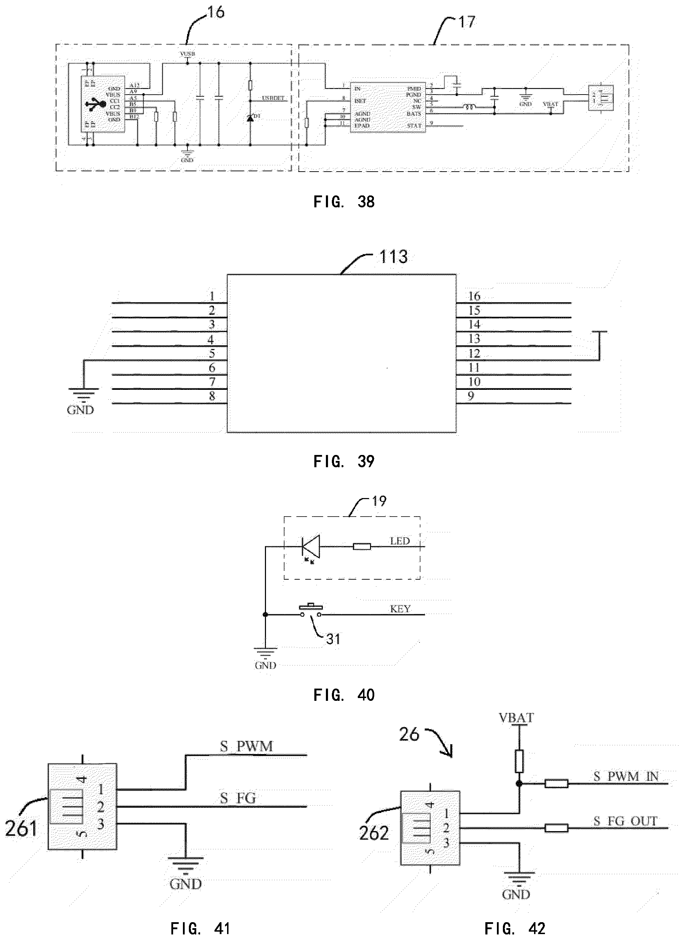

As shown in FIGS. 38 and 40, the fan drive circuit further includes an interface circuit 16 and a charge management circuit 17. The interface circuit 16 is configured to be electrically connected to an external power source to receive an external voltage. The charge management circuit 17 is electrically connected between the interface circuit 16 and a battery VBAT to receive the external voltage and to charge or output a power supply voltage to the battery VBAT. The fan drive circuit further includes a keypad 31. An end of the keypad 31 is connected to the master control circuit 11, and the other end of the keypad 31 is grounded. The fan drive circuit further includes an indicator branch 19. The indicator branch 19 includes a light-emitting diode and a resistor that is connected in series to the light-emitting diode. A positive-electrode of the light-emitting diode is electrically connected to the master control circuit 11, and a negative-electrode of the light-emitting diode is grounded.

Specifically, in the present embodiment, the fan drive circuit may be arranged for a neck fan, but is not limited to neck fans, the fan drive circuit may further be applied to other portable fans such as desktop table fans, floor fans, handheld fans, clip fans, folding fans, and the like. Two DC brushless fan motors are respectively arranged in a left side and a right side of the neck fan and are configured to respectively drive fan blades in the left side and fan blades in the right side of the neck fan to rotate.

As shown in FIGS. 35, 36 and 39, the master control circuit 11 includes a master control chip 111 and an auxiliary chip 113. The master control circuit 11 includes the master control chip 111 and the auxiliary chip 113. Two three-phase drive circuits 12, two inverted-phase electric potential detection circuits 14, and two DC brushless fan motors are arranged in one-to-one correspondence to each other. The master control chip 111 is electrically connected to one of the two three-phase drive circuits 12 to output the control signals to one of the two three-phase drive circuits 12 to drive the respective one of the two brushless DC fan motors. The inverted-phase electric potential detection circuit 14 is electrically connected to one three-phase drive circuit 12 and outputs the first detection signal, the second detection signal, and the third detection signal to the master control chip 111. In this way, the master control chip 111 is informed of the phase of the three-phase drive signal of the one three-phase drive circuit 12 to adjust the control signals output to the one three-phase drive circuit 12. The auxiliary chip 113 is electrically connected to the other three-phase drive circuit 12 to output the control signals to the other three-phase drive circuit 12 to drive the other one of the two DC brushless fan motors. The other inverted-phase electric potential detection circuit 14 is electrically connected to the respective one three-phase drive circuit 12 and outputs a corresponding first detection signal, a corresponding second detection signal and a corresponding third detection signal to the auxiliary chip 113. In this way, the auxiliary chip 113 is informed of the phase of the three-phase drive signal of the other three-phase drive circuit 12 to adjust the control signals output to the other three-phase drive circuit 12.

In this embodiment, the master control chip 111, the corresponding three-phase drive circuit 12, and the corresponding inverted-phase electric potential detection circuit 14 are arranged on one module (such as on a first circuit board) and may be arranged on a same side of the neck fan as the corresponding DC brushless fan motor. The auxiliary chip 113, the corresponding three-phase drive circuit 12, and the corresponding inverted-phase electric potential detection circuit 14 are arranged on another one module (such as on a second circuit board that is independent from the first circuit board) and may be arranged on the other side of the neck fan, together with the corresponding DC brushless fan motor. It is understood that the above configuration has better rationality and compactness, and reliability of connection and driving is improved. However, arrangement of the three-phase drive circuit 12, the inverted-phase electric potential detection circuit 14, the master control chip 111, and the auxiliary chip 113 may be arranged in various manners. For example, the three-phase drive circuit 12, the inverted-phase electric potential detection circuit 14, the master control chip 111, and the auxiliary chip 113 are arranged on a same circuit board; alternatively, the three-phase drive circuit 12 and the inverted-phase electric potential detection circuit 14 are arranged on one circuit board, and the master control chip 111 and the auxiliary chip 113 are arranged on another one circuit board. The arrangement may be determined according to the actual demands, which will not described here.

As shown in FIGS. 41 and 42, the fan drive circuit further includes a first connector 261 and a speed adjustment interface circuit 26 having a second connector 262. A first pin and a second pin of the first connector 261 are electrically connected to the master control chip 111. A third pin of the first connector 261 is grounded. A first pin of the second connector 262 is connected to the battery VBAT via a first connection resistor and is also connected to the auxiliary chip 113 via a second connection resistor. A second pin of the second connector 262 is connected to the auxiliary chip 113 via a third connection resistor, and a third pin of the second connector 262 is grounded. In addition, each pin of the first connector 261 and the second connector 262 may be electrically connected to each other in one-to-one correspondence with each other. In this way, rotation speeds of the two DC brushless fan motors may be synchronously adjusted.

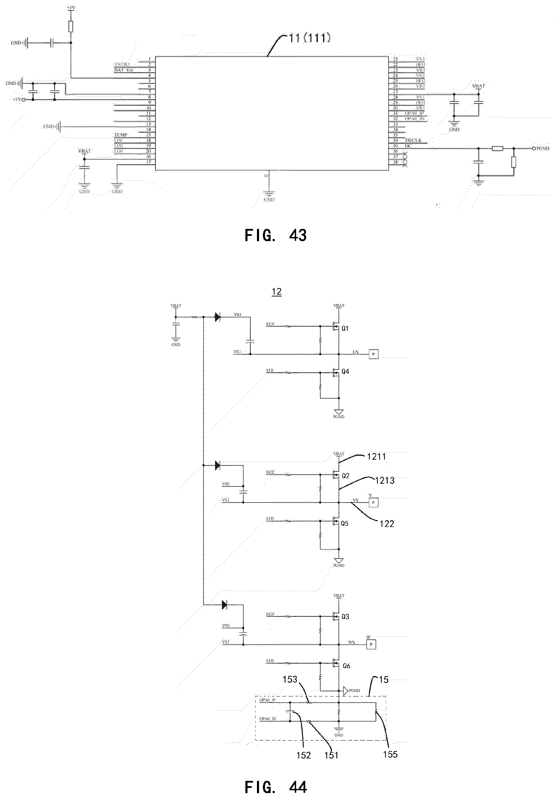

As shown in FIGS. 43 to 48, the fan drive circuit of a second implementation is shown. Portions of the fan drive circuit of the present implementation are the same as those in the first implementation and will not be repeated. Portions of the fan drive circuit of the present implementation that are different from those in the first implementation will be described in the following. Firstly, the master control circuit 11 of the present implementation is different from that of the first implementation, and the master control circuit 11 of the present implementation may substantially include the master control chip 111.

As shown in FIG. 44, in the second implementation, the three-phase drive circuit 12 includes a first transistor Q1, a second transistor Q2, a third transistor Q3, a fourth transistor Q4, a fifth transistor Q5, and a sixth transistor Q6. A first conductive end 1211 of the first transistor Q1, a first conductive end 1211 of the second transistor Q2, and a first conductive end 1211 of the third transistor Q3 are connected to a power supply end 1212. A first conductive end 1211 of the fourth transistor Q4 is connected to a second conductive end 1213 of the first transistor Q1. A first conductive end 1211 of the fifth transistor Q5 is connected to a second conductive end 1213 of the second transistor Q2. A first conduction end 1211 of the sixth transistor Q6 is connected to a second conductive end 1213 of the third transistor Q3. A node between the first conductive 1211 of the fourth transistor Q4 and the second conductive 1213 of the first transistor Q1, a node between the first conductive 1211 of the fifth transistor Q5 and the second conductive 1213 of the second transistor Q2, and a node between the first conductive 1211 of the sixth transistor Q6 and the second conductive 1213 of the third transistor Q3 respectively serve as the three drive signal output ends 122. Control ends of the first transistor Q1, the second transistor Q2, the third transistor Q3, the fourth transistor Q4, the fifth transistor Q5, and the sixth transistor Q6 are electrically connected to the master control circuit 11 to receive the control signals; and the control signals include six PWM signals.

As shown in FIG. 44, substantially the same as the first implementation, the second conductive end 1213 of the sixth transistor Q6 is grounded via the current detection circuit 15, and the current detection circuit 15 is electrically connected to the master control circuit 11. The current detection circuit 15 includes a sense resistor 151 and a sense capacitor 152. The second conductive end 1213 of the sixth transistor Q6 is grounded via the sense resistor 151. The sense capacitor 152 is connected in parallel with the sense resistor 151. A node between the sense resistor 151 and the second conduction end 1213 of the sixth transistor Q6 is electrically connected to the master control circuit 11. The current detection circuit 15 further includes a first series resistor 153, a second series resistor 154, a parallel resistor 155. The parallel resistor 155 is connected in parallel with the sense resistor 151. The first series resistance 153 is connected between an end of the sense capacitor 152 and an end of the sense resistor 151. The second series resistance 154 is connected between the other end of the sense capacitor 152 and the other end of the sense resistor 151. By arranging the current detection circuit 15, when the current is abnormal, the master control circuit 11 may control the fan drive circuit to stop operating or to operate at a lower power, such that an overcurrent protection is provided for the fan drive circuit, and reliability and the service life of the fan drive circuit are improved.

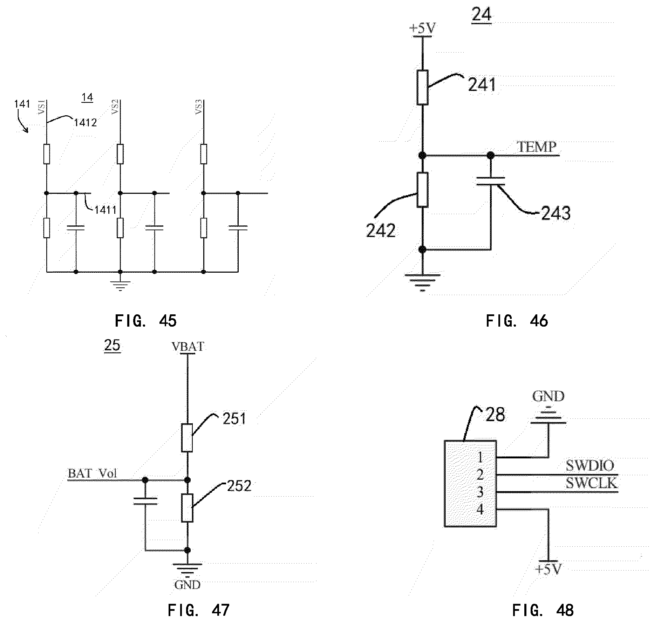

As shown in FIG. 45, the inverted-phase electric potential detection circuit 14 of the second implementation is substantially the same as that of the first implementation, and will not be repeated herein.

As shown in FIG. 46, the fan drive circuit further includes a transistor temperature detection circuit 24. The transistor temperature detection circuit 24 may be disposed adjacent to each transistor of the three-phase drive circuit 12 and includes a first voltage divider resistor 241 and a thermistor 242 connected in series with the first voltage divider resistor 241. The thermistor 242 is configured to sense a temperature of each transistor of the three-phase drive circuit 12. A node between the first voltage divider resistor 241 and the thermistor 242 is electrically connected to the master control circuit 11 and is configured to output a temperature signal, enabling the master control circuit 11 to control, based on the temperature signal, the fan drive circuit to enter or not enter a temperature protection state. The thermistor 242 is connected between the first voltage divider resistor 241 and the ground. The transistor temperature detection circuit 24 further includes a voltage regulated capacitor 243 connected in parallel with the thermistor 242. By arranging the transistor temperature detection circuit 24, the master control circuit 11 may be informed whether the temperature of each transistor of the three-phase drive circuit 12 is abnormal and may control the fan drive circuit to stop operating or to operate at a lower power when the temperature is abnormal. In this way, an over-temperature protection is provided for the fan drive circuit, and reliability and the service life of the fan drive circuit are improved.

As shown in FIG. 47, the fan drive circuit further includes a battery voltage detection circuit 25 that is electrically connected between the positive electrode of the battery VBAT and the ground. An output end of the battery voltage detection circuit 25 is electrically connected to the master control circuit 11. By arranging the battery voltage detection circuit 25, the master control circuit 11 may be informed whether a battery voltage is normal or not. When the battery voltage is abnormal, the master control circuit 11 may control the fan drive circuit to stop operating or to operate at a lower power. Therefore, reliability and the service life of the fan drive circuit are improved.

Specifically, the battery voltage detection circuit 25 includes a second voltage divider resistor 251 and a third voltage divider resistor 252 that is connected in series to the second voltage divider resistor 251. A node between the second voltage divider resistor 251 and the third voltage divider resistor 252 is electrically connected to the master control circuit 11. It is understood that the above-described battery voltage detection circuit 25 is simple in structure and has high reliability and a low cost.

As shown in FIG. 48, the fan drive circuit of the second implementation further includes a burner interface 28 to burn in a control program to the master control circuit 11. The burner interface 28 may be a SWD burner interface, but is not limited to the above.

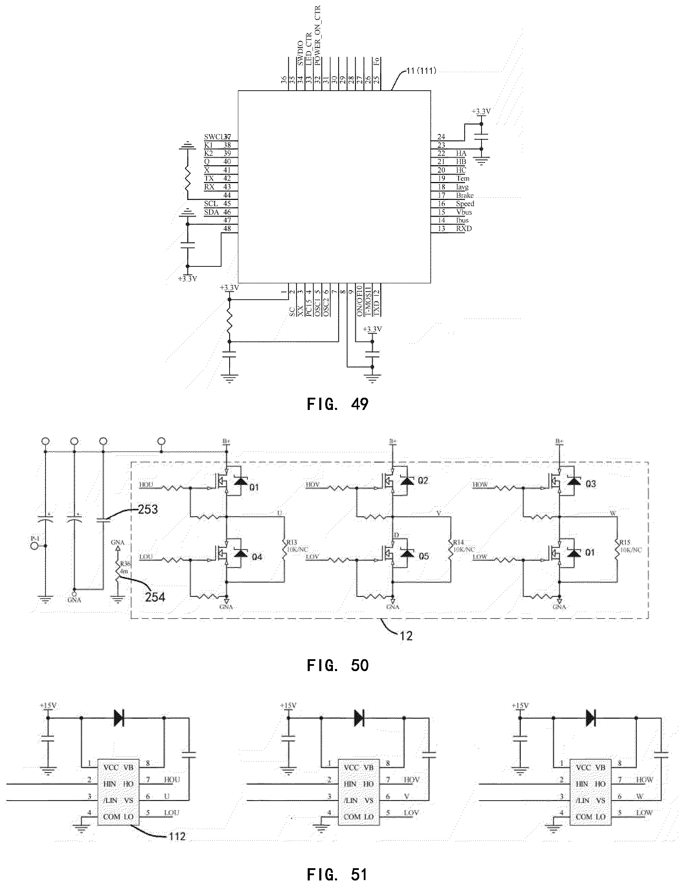

As shown in FIGS. 49 to 50, the fan drive circuit of a third implementation is provided. Portions of the fan drive circuit of the present implementation are the same as those in the second implementation and will not be repeated. Portions of the fan drive circuit of the present implementation that are different from those in the second implementation will be described in the following.

As shown in FIGS. 49-FIG. 51, the three-phase drive circuit 12 of the third implementation is essentially the same as the three-phase drive circuit 12 of the second implementation. The master control circuit 11 of the third implementation is different from that of the second implementation. In the present implementation, the master control circuit 11 includes a master control chip 111 and three three-phase control chips 112. Each of the three three-phase control chips 112 is electrically connected to the master control chip 111 and the three-phase drive circuit 12.

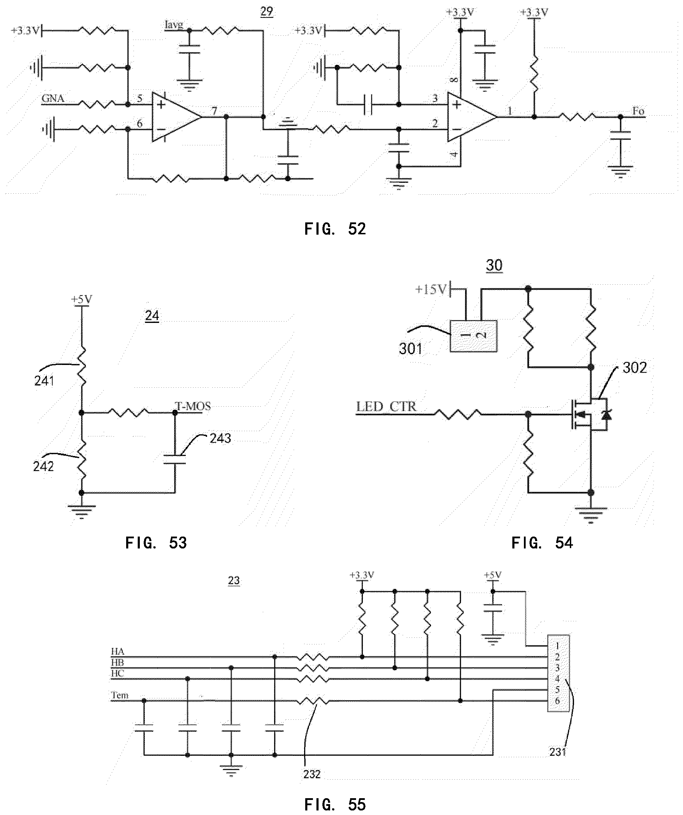

As shown in FIGS. 50 and 52, the fan drive circuit further includes a filter capacitor 253 and a sampling resistor 254 connected in series to the filter capacitor 253. The sampling resistor 254 is connected between the filter capacitor 253 and the ground. A node between the filter capacitor 253 and the sampling resistor 254 is electrically connected to the master control circuit 11. Further, the fan drive circuit further includes a signal amplification circuit 29. An input end of the signal amplification circuit 29 is connected to the node between the filter capacitor 253 and the sampling resistor 254. The signal amplification circuit 29 is configured to amplify a signal sampled by the sampling resistor 254 (i.e., a signal of the node between the filter capacitor 253 and the sampling resistor 254) and to provide the amplified signal to the master control circuit 11. In this way, the main control circuit 11 of the fan drive circuit may keenly detect an abnormal voltage or current signal when the fan drive circuit is abnormal and then perform protection against the abnormalities, such as stopping operating or reducing a fan speed. In this way, safety of using the fan drive circuit is improved.

As shown in FIG. 53, the transistor temperature detection circuit 24 of the third implementation is essentially the same as that of the second implementation and will not be repeated herein.

As shown in FIG. 54, a schematic view of a light control circuit 30 of the fan drive circuit in the third implementation is provided. The light control circuit 30 includes a light-emitting element 301 and a control switch 302. A positive electrode of the light-emitting element 301 receives a drive voltage. A negative electrode of the light-emitting element 301 is grounded via two conductive ends of the resistor and the control switch 302. A control end of the control switch 302 is electrically connected to the master control circuit 11, such that the master control circuit 11 outputs a light control signal to the control end of the control switch 302 to control the light-emitting element 301 to emit light.

As shown in FIG. 55, the fan drive circuit further includes a Hall detection circuit 23. The Hall detection circuit 23 is electrically connected to the master control circuit 11 to detect a magnetic field generated by the DC brushless fan motor and to output a Hall detection signal to the master control circuit 11. In this way, the master control circuit 11 may be informed, based on the Hall detection signal, of a position of a rotor of the DC brushless fan motor, such that the master control circuit 11 may provide a corresponding control signal to control the DC brushless fan motor to operate. In this case, a start-up time length of the fan using the fan drive circuit is shorter, and the fan may not shake during starting-up, and a better user experience is provided.

As shown in FIG. 55, the Hall detection circuit 23 further includes a motor temperature detection element 232 connected between a Hall element 231 of the Hall detection circuit 23 and the master control circuit 11. The motor temperature detection element 232 may be a sampling resistor. By arranging the motor temperature detection element 232, the master control circuit 11 may be informed of whether a temperature of the DC brushless fan motor is abnormal. When the temperature of the DC brushless fan motor is abnormal, the master control circuit 11 may control the fan drive circuit to stop operating or operate at a lower power, such that an over-temperature protection is provided for the fan drive circuit, and reliability and the service life of the fan drive circuit are improved.

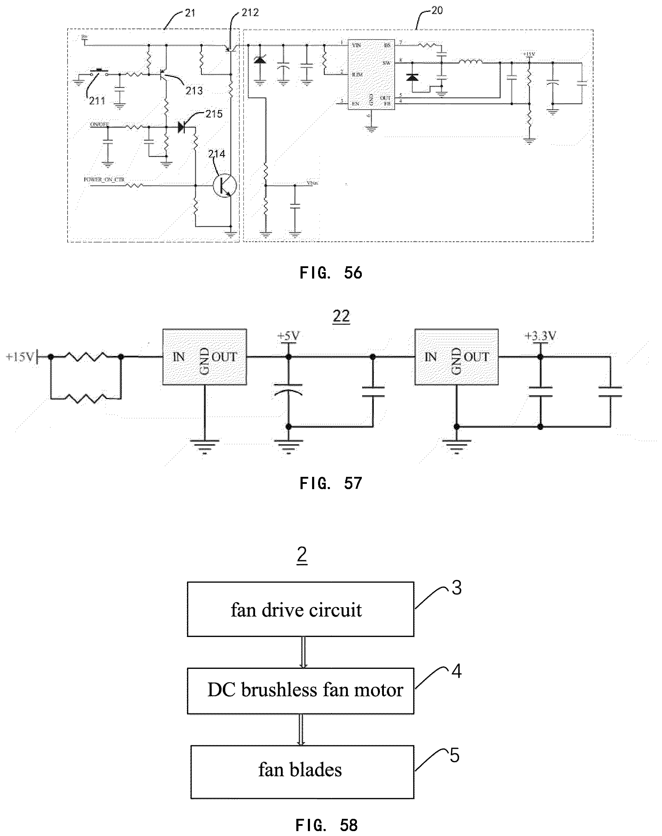

As shown in FIGS. 51 and 56, the fan drive circuit further includes a voltage conversion circuit 20. The voltage conversion circuit 20 is configured to receive a battery voltage (VB+), convert the battery voltage into a drive voltage (such as 15V), and provide the drive voltage to power supply ends of the three three-phase control chips 112. The master control chip 111 is configured to output a master control signal to the three three-phase control chips 112, such that each of the three three-phase control chips 112 outputs the respective control signal to the three-phase drive circuit 12.

The fan drive circuit further includes a switch control circuit 21. The switch control circuit 21 is electrically connected to the battery VBAT, the voltage conversion circuit 20, and the master control circuit 11 to control operation of the voltage conversion circuit 20. The switch control circuit 21 includes a keypad 211, a first switch transistor 212, a second switch transistor 213, and a third switch transistor 214. Two conductive ends of the first switch transistor 212 are respectively connected to the positive electrode of the battery VBAT and the input end of the voltage conversion circuit 20. A control end of the first switch transistor 212 is grounded via two conductive ends of the third switch transistor 214. The positive electrode of the battery VBAT is connected to the control end of the third switch transistor 214 via the two conductive ends of the first switch transistor 212 and a one-way diode 215. A control end of the second switch transistor 213 is grounded via the keypad 211. A control end of the third switch transistor 214 is electrically connected to the master control circuit 11. A node between the second switch transistor 213 and the one-way diode 215 is further electrically connected to a switch signal end of the master control circuit 11.

When the keypad 211 is pressed to be conductive, the second switch transistor 213 is turned on, the third switch transistor 214 is turned on, and the node between the second switch transistor 213 and the one-way diode 215 outputs a first switching signal (ON) to the switch signal end of the master control circuit 11. The first switch transistor 212 is turned on to enable the battery voltage of the battery VBAT to be supplied to the voltage conversion circuit 20. When the pressing on the keypad 211 is released, the second switch transistor 213 is turned off, the master control circuit 11 maintains the third switch transistor 214 to be turned on based on a power supply turn-on signal being output from the first switching signal to the control end of the third switch transistor 214, and the battery voltage of the battery VBAT is supplied to the voltage conversion circuit 20.

Further, when the battery voltage of the battery VBAT is supplied to the voltage conversion circuit 20, and when the keypad 211 is again pressed to be conductive, the node between the second switch transistor 213 and the one-way diode 215 outputs a second switching signal (OFF) to the switch signal end of the master control circuit 11, the master control circuit 11 controls the third switch transistor 214 to be turned off based on a power supply turn-off signal being output from the second switching signal to the control end of the third switch transistor 214. In this way, the first switch transistor 212 is turned off, the battery voltage of the battery VBAT cannot be supplied to the voltage conversion circuit 20 until the keypad 211 is again pressed.

The keypad 211, the first switch transistor 212, the second switch transistor 213 and the third switch transistor 214 operate together with the master control circuit 11 to control whether the battery voltage of the battery VBAT is supplied to the voltage conversion circuit 20. In this way, a simple control logic is provided, and the circuit has higher reliability.

As shown in FIG. 57, the fan drive circuit further includes a DC conversion circuit 22. The DC conversion circuit 22 is configured to receive the drive voltage (such as a DC voltage of 15V) and convert the drive voltage to other DC operating voltages, such as a DC operating voltage of 3.3V and 5V.

As shown in FIG. 58, the present disclosure further provides a portable fan 2. The portable fan 2 includes a fan drive circuit 3, a DC brushless fan motor 4, and fan blades 5 driven by the DC brushless fan motor. The fan drive circuit 3 may be arranged with the fan drive circuit as described in any of the above embodiments.

For the fan drive circuit and the portable fan 2 in the present embodiment, the master control circuit 11, the three-phase drive circuit 12, the inverted-phase electric potential detection circuit 14, and the DC brushless fan motor are arranged. The energy-saving performance and control performance of the fan motor are improved, such that reliability of the fan drive circuit and the fan 2 are improved. In addition, the service life of the fan drive circuit and the fan 2 is extended, the arrangement of the DC brushless fan motor allows the fan 2 to have a more compact and smaller configuration, and the fan 2 has increased competitiveness in the market.

### Embodiment 6

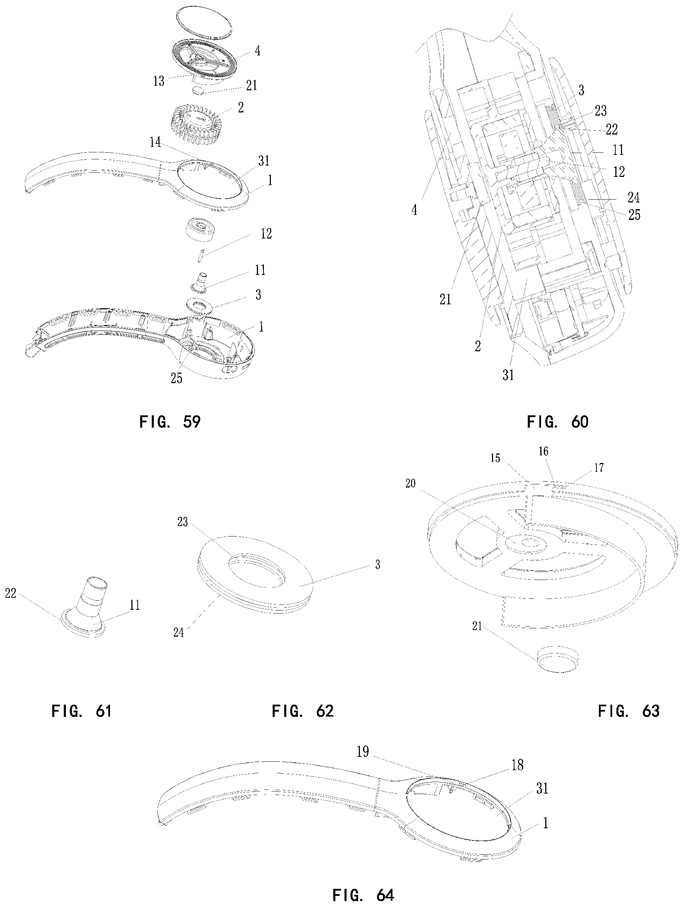

As shown in FIGS. 59 and 60, the portable fan of the present disclosure includes a housing 1, a shaft tube 11 arranged inside the housing 1, a rotation shaft 12 received in a shaft hole of the shaft tube 11 and rotatable with respect to the shaft tube 11, a stator sleeving an outside of the shaft tube 11, and an impeller 2 connected to a top of the rotation shaft 12. In the present embodiment, the portable fan is used as an example for illustration, and obviously, the portable fan may also be applied to a desktop fan, a handheld fan, a fan for a baby carriage, and other devices.

The housing 1 encloses to define a mounting channel 31 having an opening at an end. The mounting channel 31 is used for assembling and disassembling the impeller 2. The impeller 2 may be taken out from the mounting channel 31 for cleaning. A disassembling member 4 is arranged at the opening of the housing 1. The disassembling member 4 is detachably connected with the housing 1. Since a snap spring is arranged at an end of the rotation shaft 12, when the impeller 2 is rotating, the impeller 2 moves up and down and may hit the disassembling member 4 to produce a sound. An anti-hitting member 21 is arranged on a bottom surface of the disassembling member 4 facing towards the impeller 2, preventing the impeller 2 from hitting the disassembling member 4 to produce the sound.

In the art, the snap spring is usually arranged at the end of the rotation shaft 12, prevents the rotation shaft from moving out of the shaft tube, and prevents the impeller from falling off. In the present embodiment, the snap spring is not disposed at the end of the rotation shaft 12. When the fan is in use or being moved, the rotation shaft 12 may fall off from the shaft tube 11, and the impeller 2 may move. Therefore, the anti-hitting member 21 is arranged on the impeller 2 at a position corresponding to the disassembling member 4 to replace the snap spring, such that the impeller 2 is prevented from falling off.

The disassembling member 4 and the housing 1 may be rotatably disassembled from each other, or may be disassembled from each other by snap-fitting.

An anti-hitting recess 20 is formed in a bottom surface of a center of the disassembling member 4 facing towards the impeller 2, and the anti-hitting member 21 is arranged on the bottom surface of the disassembling member 4 facing towards the impeller 2. The anti-hitting member 21 is mounted at the anti-hitting recess 20, such that the impeller 2 is prevented from colliding, during rotating, with the disassembling member 4 to produce the sound, and therefore, the noise is reduced.

The anti-hitting member 21 is made of EVA foam or soft silicone. The anti-hitting member 21 is mounted between the impeller 2 and disassembling member 4, such that collision of the impeller 2 is effectively reduced, and a vibration absorption effect is achieved.

The anti-hitting member 21 may be cylindrical or square, as long as the impeller 2 can be prevented from falling off. It can be expected that in impact with the disassembling member 4 is also prevented, sch that an impact sound is reduced.

A distance from a side of the anti-hitting member 21 near the impeller 2 to a side of the impeller 2 near the anti-hitting member 21 is in a range of 0.1mm to 5 mm, and a diameter of the anti-hitting member 21 is in a range of 1mm to 20 mm.

An anti-vibration member and the shaft tube 11 are detachably connected to each other. The anti-vibration member and the housing 1 are detachably connected to each other. The shaft tube 11 is connected to the housing 1 through the anti-vibration member. When the portable fan is being used, the impeller 2 oscillates to drive the shaft tube 11 to vibrate. The anti-vibration member is connected between the housing 1 and the shaft tube 11. The anti-vibration member prevents vibration of the impeller 2 from being transmitted to the housing 1, such that noise is prevented, and the user experience is not affected.

As shown in FIG. 59, FIG. 60, FIG. 61, and FIG. 62, the anti-vibration member is an annular ring 3. An inner periphery of the annular ring 3 is snapped with the shaft tube 11. An end of the shaft tube 11 extends outwardly along a radial direction to form a first protrusion 22. An inner wall of the annular ring 3 defines an inner groove 23 that is recessed away from an axial center of the annular ring 3. The first protrusion 22 is inserted into the inner groove 23. A gap is reserved between the first protrusion 22 and the inner groove 23. When the shaft tube 11 vibrates, the first protrusion 22 is driven to vibrate. Since the gap is reserved between the first protrusion 22 and the inner groove 23, a vibration amplitude of the first protrusion 22 is smaller than the gap between the first protrusion 22 and the inner groove 23, such that vibration that can be transmitted to the housing 1 is reduced. In addition, the above configuration can be assembled conveniently and highly efficiently.

As shown in FIG. 59, FIG. 60, FIG. 61 and FIG. 62 and FIG. 65, an outer periphery of the annular ring 3 is snapped with the housing 1. An outer wall of the annular ring 3 defines an outer groove 24 recessed towards the axial center of the annular ring 3. The housing 1 is arranged with a second protrusion 25 snapped with the outer groove 24. The second protrusion 25 is inserted into the outer groove 24. A gap is reserved between the second protrusion 25 and the outer groove 24. When the shaft tube 11 vibrates, the second protrusion 25 is driven to vibrate. Since the gap is reserved between the second protrusion 25 and the outer groove 24, a vibration amplitude of the second protrusion 25 is smaller than the gap between the second protrusion 25 and the outer groove 24, such that vibration that can be transmitted to the housing 1 is reduced. In addition, the above configuration can be assembled conveniently and highly efficiently.

As shown in FIG. 59, FIG. 60, FIG. 61, FIG. 62, and FIG. 65, the annular ring 3 is made of a soft material. The annular ring 3 can be deformed. When the portable fan is started, and when the impeller 2 oscillates up and down and vibrates, the shaft tube 11 is driven to vibrate, and the vibration is transmitted to the annular ring 3. The vibration causes the annular ring 3 to be deformed. A part of the vibration is stored as energy in the deformed annular ring 3, and another part of the energy is transmitted to the housing 1. Therefore, vibration energy transmitted to the housing 1 is reduced, the generated noise and the vibration amplitude are also reduced.

As shown in FIG. 59, FIG. 60, FIG. 61 and FIG. 62, the housing 1 encloses to define a mounting channel 31 having an opening at an end. The opening is located corresponding to the impeller 2. The impeller 2 can be taken out from the mounting channel 31 to be cleaned. The disassembling member 4 is disposed at the opening of the housing 1. The disassembling member 4 is configured to cover the mounting channel 31 to prevent the hair from entering the mounting channel 31.

As shown in FIG. 63 and FIG. 64, the disassembling member 4 is detachably connected to the housing 1. When the disassembling member 4 is disassembled from the housing 1, the impeller may be conveniently taken out. One of the disassembling member 4 and the housing 1 is arranged with a rotation fastener 14, and the other one of the disassembling member 4 and the housing 1 defines a rotation fastener groove 13. Of course, in other embodiments, one of the disassembling member 4 and the housing 1 is arranged with outer threads, and the other one of the disassembling member 4 and the housing 1 is arranged with inner threads, such that the disassembling member 4 and the housing 1 are threadedly connected to each other. Of course, in other embodiments, one of the disassembling member 4 and the housing 1 is arranged with a first magnet, and the other one of the disassembling member 4 and the housing 1 is arranged with a second magnet, such that the disassembling member 4 and the housing 1 are magnetically connected to each other.

One of the disassembling member 4 and the housing 1 is arranged with the rotation fastener 14, and the other one of the disassembling member 4 and the housing 1 defines the rotation fastener groove 13. The rotation fastener groove 13 has a first position and a second position, and the rotation fastener 14 may rotate to switch between the position and the second position. The disassembling member 4 and the housing 1 rotate to switch between a first mating position and a second mating position. When the disassembling member 4 and the housing 1 are at the first mating position, the rotation fastener 14 is inserted into the first position of the rotation fastener groove 13. When the disassembling member 4 and the housing 1 are at the second mating position, the rotation fastener 14 is inserted into the second position of the rotation fastener groove 13. In this way, relative displacement of the disassembling member 4 and the housing 1 is limited. By sliding the disassembling member 4 and the housing 1 to switch between positions, the rotation fastener 14 is enabled to enter the second position of the rotation fastener groove 13. Due to relative movement of the rotation fastener groove 13 and the rotation fastener 14 being limited, connection between the disassembling member 4 and the housing 1 can be achieved.

The outer wall of the disassembling member 4 defines the rotation fastener groove 13, and the inner wall of the mounting channel 31 of the housing 1 having the opening is arranged with the rotation fastener 14. An outer wall of the disassembling member 4 defines a first recess 15 and a second recess 16 along the radial direction thereof. The first recess 15 and the second recess 16 are communicated to each other. The first position of the rotation fastener groove 13 is corresponding a position of the first recess 15, and the second position of the rotation fastener groove 13 is corresponding to a position of the second recess 16. A first resilient fastener 17 is arranged along the radial direction of the disassembling member 4 protruding into the second recess 16.

The inner wall of the opening of the housing 1 defines a third recess 19. The third recess 19 is extending in the radial direction of the housing 1. A second resilient fastener 18 is arranged at a side of the third recess 19 near the disassembling member 4. The second resilient fastener 18 is stretching along a direction extending from the opening of the housing 1 to a diameter of the housing 1. The second resilient fastener 18 is connected with the housing 1. When the second resilient fastener 18 slides from the first recess 15 to the second recess 16, an axial relative displacement of the disassembling member 4 and the housing 1 is limited.

### Embodiment 7

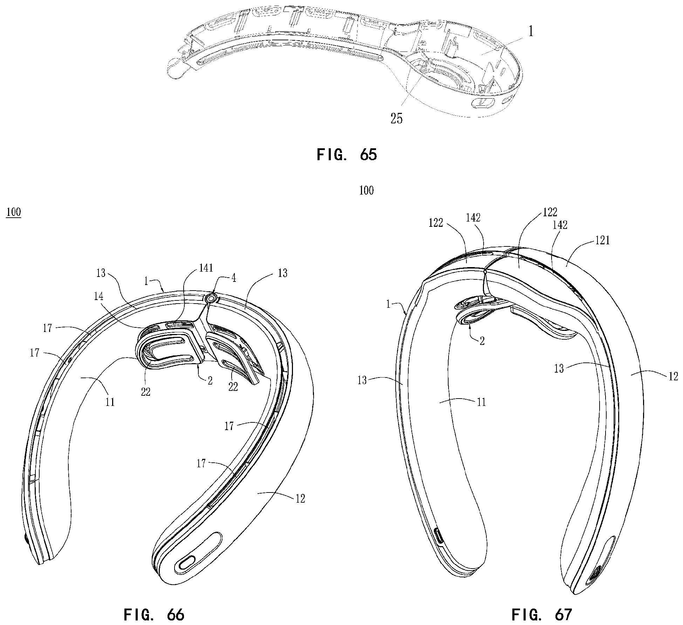

As shown in FIGS. 66 to 68, schematic views of a portable fan 100 of a first implementation are provided. The portable fan 100 includes a housing 1, a support member 2 and a fan assembly 3. The support member 2 is mounted on a side of the housing 1 facing the neck of the user. The support member 2 is rotatably connected to the housing 1 to be supported to the rear of the neck. The fan assembly 3 is arranged inside the housing 1. By arranging the support member 2 to be supported to the rear of the neck, the housing 1 dose not rub the neck. In addition, the support member 2 supports the housing 1 and the rear of the neck of the user, allowing a spacing to be defined between the housing 1 and the rear of the neck, such that a contact area between the housing 1 and the neck is reduced, and a cooling effect of the portable fan 100 is improved

As shown in FIGS. 66 to 68, the housing 1 includes a first side wall 11 facing towards the neck of the user and a second side wall 12 facing away from the neck. A first mating portion 18 is arranged on and protrudes from the first side wall 11. The support member 2 is arranged with a second mating portion 21 corresponding to the first mating portion 18. The second mating portion 21 is rotatably connected to the first mating portion 18. The support member 2 is rotatable relative to the first side wall 11.

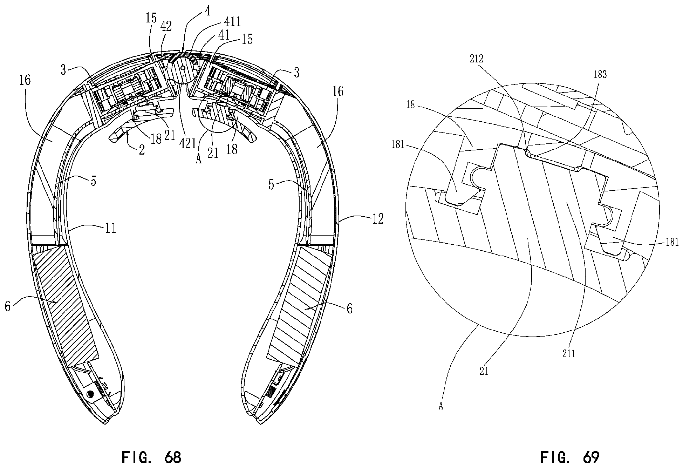

As shown in FIGS. 68 to 70, in the present embodiment, the first mating portion 18 includes two pivot portions 181 and an elastic arm 182 disposed between the two pivot portions 181. A protrusion 183 is arranged at a free end of the elastic arm 182. The second mating portion 21 includes a pivot column 211. A plurality of recesses 212 are defined in a surface of the pivot column 211. Two ends of the pivot column 211 are respectively pivotally connected to the two pivot portions 181. When the support member 2 is rotating upwardly and downwardly, the protrusion 183 is received in any one of the plurality of recesses 212 in order to position the support member 2 at various angles.

As shown in FIGS. 66 to 68, the housing 1 includes two clamp arms 13 that are disposed symmetrically to each other. The two the clamp arms 13 are rotatably connected to each other by a rotation connection member 4. A pivot angle of each clamp arm 13 is in a range of ±10°. The two clamp arms 13 are rotatably connected to each other to allow the user to adjust a distance between free ends of the two clamp arms 13, such that the portable fan can be worn to the neck and fixed. Each clamp arm 13 defines sequentially an air inlet 14, a receiving cavity 15, an air duct 16, and an air outlet 17, and the air inlet 14, the receiving cavity 15, the air duct 16, and the air outlet 17 are communicated to each other. The fan assembly 3 is received in the receiving cavity 15 to drive an airflow to flow from the air inlet 14 to flow through the air duct 16 to reach the air outlet 17.

As shown in FIGS. 66 to 68, the portable fan 100 is arranged with two support members 2 corresponding to the two clamp arms 13, respectively. When the two clamp arms 13 are pivoted, positions of the two support members 2 in contact with the user are changed. However, since the support members 2 are rotatably connected to the clamp arms 13, each support member 2 can be adjusted independently to be attached to the neck of the user, such that wearing comfort is improved.

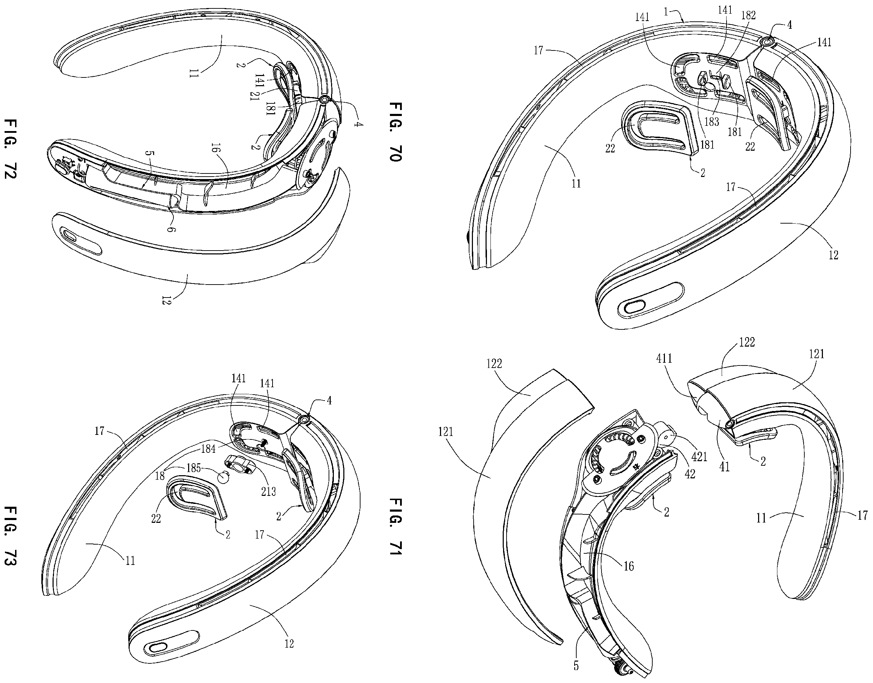

As shown in FIGS. 66 to 68, corresponding to each fan assembly 3, each clamp arm 13 defines a first air inlet 141 defined in the first side wall 11 and a second air inlet 142 defined in the second side wall 12. The receiving cavity 15 is located at two ends of the two clamp arms 13 that are connected to each other. The support member 2 is disposed corresponding to the receiving cavity 15. A spacing is defined between at least a portion of the support member 2 and the first air inlet 141. Therefore, when the portable fan 100 is in use, the support member 2 does not affect air intaken through the first air inlet 141. Moreover, as shown in FIG. 70, the support member 2 defines a through hole 22 corresponding to the first air inlet 141, such that sweat, generated by contact between the support member 2 and the neck, can be absorbed and evaporated in time, ensuring that the neck of the user is dry and fresh, and a wearing experience is improved.

As shown in FIGS. 67 and 71, the second side wall 12 includes a main wall 121 and an auxiliary wall 122. The main wall 121 and the auxiliary wall 122 are arranged in a stepped manner. The second air inlet 142 is formed between the main wall 121 and the auxiliary wall 122. The second air inlet 142 is hiddenly formed between the main wall 121 and the auxiliary wall 122, and therefore, an aesthetic appearance is provided, and the hair of the user may not enter the second air inlet 142 easily, safety of the portable fan is improved.

As shown in FIGS. 68 and 71, the rotation connection member 4 includes a first connection member 41 and a second connection member 42. The first connection member 41 is fixedly connected to one of the two clamp arms 13, and the second connection member 42 is fixedly connected to the other one of the two clamp arms 13. The first connection member 41 includes a first fixation portion 411, and the second connection member 42 includes a second fixation portion 421. The first fixation portion 411 and the second fixation portion 421 are rotatably connected to each other. The first fixation portion 411 and the second fixation portion 421 cooperatively form a spindle shape. The two the clamp arms 13, during rotating, rotate along the first fixation portion 411 and the second fixation portion 421 that are configured in the spindle shape, such that an aesthetic appearance is provided.

As shown in FIGS. 68, 71 and 72, a partition assembly 5 is further arranged. The partition assembly 5 separates the receiving cavity 15 and the air duct 16, which are communicated with each other, from the other space inside the housing 1. A width of an end of the air duct 16 away from the receiving cavity 15 is less than a width of an end of the air duct 16 near the receiving cavity 15, such that a space is reserved to receive a battery 6. In the present embodiment, the battery 6 is arranged inside each of the two clamp arms 13, and two fan assemblies 3 may operate simultaneously or separately. The air outlet 17 is formed at and extends through a side of the air duct 16 and extends substantially to cover the entirety of the air duct 16. The width of the end of the air duct 16 away from the receiving cavity 15 is narrower, such that the air may be gathered easily at the narrower end, and various portions of the air outlet 17 may have an even airflow.

As shown in FIG. 73, a schematic view of the portable fan 100 of a second implementation is shown. In the present implementation, the second mating portion 21 includes a mounting bracket 213. The first mating portion 18 includes a fixation member 184 secured to the first side wall 11 and a rotation ball 185 that extends through the mounting bracket 213 and is secured to the fixation member 184. The mounting bracket 213 is rotated with respect to the rotation ball 185 to enable the support bracket to rotate with respect to the rotation ball 185. Compared to the support bracket in the first implementation capable of rotating up and down, the support member 2 in the present implementation may rotate spherically, such that the portable fan can be worn comfortably. Other structures and performance of the present implementation are substantially the same as those in the first implementation, and will not be repeated herein.

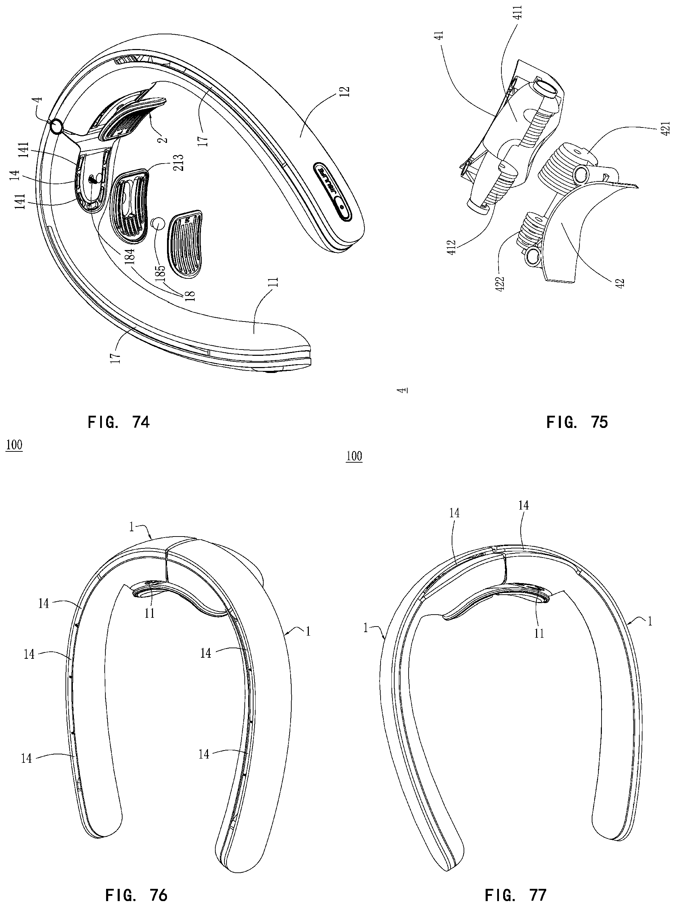

As shown in FIGS. 74 and 75, schematic views of the portable fan 100 of a third implementation are shown. In the present implementation, a shape of the mounting bracket 213 matches a shape of the second mating portion 21. In the rotation connection member 4, the first fixation portion 411 includes a plurality of first pieces 412, the second fixation portion 421 includes a plurality of second pieces 422. The plurality of the first pieces 412 and the plurality of the second pieces 422 are laminated and arranged alternately with each other. In this way, friction between the first fixation portion 411 and the second fixation portion 421 is greatly increased, such that, during rotating, the two clamp arms 13 may stop at any position, safety performance is improved. Other structures and performance of the present implementation are substantially the same as those in the first implementation, and will not be repeated herein.

It is understood that, in addition to the above embodiment, in other embodiments, the support member 2 is connected to the housing 1 by a bendable positioning member. The bendable positioning member may allow the support member 2 to be better attached to the rear of the neck.

### Embodiment 8

As shown in FIGS. 76 to 80, schematic views of a portable fan 100 of a first implementation are provided. The portable fan 100 includes a clamp arm 1, a fan assembly 2, and a rotation connection structure 3. Two clamp arms 1 are arranged, and each of the two clamp arms 1 defines an air inlet 11, a receiving cavity 12, an air duct 13, and an air outlet 14, all of which are communicated with each other. Correspondingly, two fan assemblies 2 are arranged. Each of the two fan assemblies 2 is located in a respective one accommodating cavity 12. The fan assembly 2 is configured to drive the air to flow from the air outlet 14 through the air duct 13 to reach the air outlet 14. The rotation connection structure 3 includes a first hinge member 31 and a second hinge member 32. The first hinge member 31 includes a plurality of first pieces 311 that are arranged in parallel to each other and are spaced apart from each other. The second hinge member 32 includes a plurality of second pieces 321 that are arranged in parallel to each other and are spaced apart from each other.

The first hinge member 31 is fixed to one of the two clamp arms 1, and the second hinge member 32 is fixed to the other one of the two clamp arms 1. The first hinge member 31 and the second hinge member 32 are fixedly mated to each other. The plurality of the first pieces 311 and the plurality of the second pieces 321 are laminated and are arranged alternately with each other. In this way, the two clamp arms 1 can be rotated relative to each other, and when rotating is stopped, the two clamp arms 1 can stay static at the stopped position. By arranging the first hinge member 31 including the plurality of the first pieces 311 and the second hinge member 32 including the plurality of the second pieces 321, the plurality of the first pieces 311 and the plurality of the second pieces 321 are laminated and are arranged alternately with each other, such that friction between the first hinge member 31 and the second hinge member 32 is greatly increased, allowing the two clamp arms 1 to stop rotating at any position during rotating, safety performance of the portable fan is improved.

In the present embodiment, the first hinge member 31 includes five first pieces 311, and the second hinge member 32 includes six second pieces 321. Each of the plurality of first pieces 311 has two contact surfaces respectively contacting two of the plurality of second pieces 321 adjacent to the respective one first piece 311. When the plurality of first pieces 311 and the plurality of second pieces 321 are laminated and are arranged alternately, a total of ten contact surfaces are formed, and therefore, the friction is ten times of friction generated by one contact surface. Therefore, the rotation connection structure 3, after being connected, rotates with a higher friction, the rotation connection structure 3 may stop rotating at any position. Of course, the number of the first pieces 311 and the number of the second pieces 321 are not limited herein.

It is understood that in other embodiments, three clamp arms 1 may be arranged. The rotation connection structure 3 is disposed between every two adjacent clamp arms 1. In this way, every two adjacent clamp arms 1 are rotatable with respect to each other, and the rotation may stop at any position.

As shown in FIGS. 76 to 78, in the present embodiment, the fan assembly 2 is a centrifugal fan. The clamp arm 1 defines two air inlets 11 corresponding to each fan assembly 2. The air is intaken from two sides, a stronger airflow is generated, and a better air outputting effect is achieved. A support portion is arranged on the side of the portable fan 100 facing the neck. The support portion supports the portable fan 100 to be attached to the neck to allow the portable fan to be worn more comfortably, and allows a spacing to be defined between the clamp arm 2 and the neck, enabling the air inlet 11 on the side facing the neck to better intake the air.

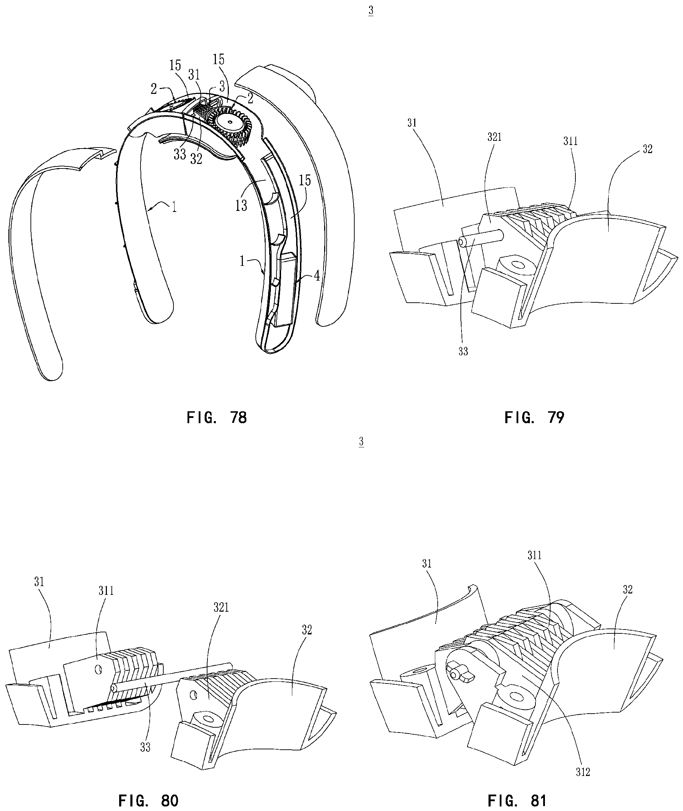

As shown in FIGS. 78 to 80, the plurality of first pieces 311 have a same thickness, the plurality of second pieces 321 have a same thickness, and the thickness of the plurality of first pieces 311 is the same as the thickness of the plurality of second pieces 321. The thickness of each of the plurality of first pieces 311 is 1.5 mm, and the thickness of each of the plurality of second pieces 321 is 1.5 mm. A spacing between two adjacent first pieces 311 is 0.01mm to 0.3mm greater than the thickness of the second piece 321. In the present embodiment, the spacing between two adjacent first pieces 311 is 0.1mm greater than the thickness of the second piece 321, and a spacing between two adjacent second pieces 321 is 0.1mm greater than the thickness of the first piece 311.

As shown in FIGS. 78 to 80, in the present embodiment, a pivot 33 is further arranged. The pivot 33 extends through the plurality of first pieces 311 and the plurality of the second pieces 321 that are laminated and arranged alternately, so as to secure the first hinge member 31 and the second hinge member 32. The receiving cavity 12 is located at an end of the clamp arm 1 near the rotation connection structure 3 and receives the fan assembly 2. The air duct 13 and a wire receiving chamber 15 are defined along a direction extending from the receiving cavity 12 towards the free end of the clamp arm 1. At least one wire receiving chamber 15 receives a battery 4. The battery 4 supplies power to the fan assembly 2. Two fan assemblies 2 may operate simultaneously or independently.

As shown in FIGS. 78 to 80, each first piece 311 and each second piece 321 have a same shape, which is irregular polygonal. When the free ends of the two clamp arms 1 are rotated to be away from each other, a contact area between the first piece 311 and the second piece 321 increases. Therefore, the friction between the first hinge member 31 and the second hinge member 32 increases. When the free ends of the two clamp arms 1 are rotated to be closer to each other, the contact area between the first piece 311 and the second piece 321 decreases, and therefore, the friction between the first hinge member 31 and the second hinge member 32 decreases. That is, the friction between the first hinge member 31 and the second hinge member 32 when the free ends of the two clamp arms 1 being rotated away from each other is greater than the friction between the first hinge member 31 and the second hinge member 32 when the free ends of the two clamp arms 1 being rotated to be closer to each other. In other words, it is easier to rotate the free ends of the two clamp arms 1 to be closer to each other than to rotate the free ends of the two clamp arms 1 to be away from each other. Therefore, when the two clamp arms 1 are worn around the neck, the two clamp arms 1 are less likely to slip away from the neck.

As shown in FIGS. 77 to 80, it is understood that, due to the pivot 33 being located close to the free ends of the first piece 311 and the second piece 321, and due to no limiting structure being disposed between the first piece 311 and the second piece 321, theoretically the first hinge member 31 and the second hinge member 32 may be rotated by more than 180° with respect to each other. However, according to the practical demands of the product, the spacing between shells of the two clamp arms 1 is small. Due to limitation of the spacing between the housings of the two clamp arms 1, the pivoting angle of the two the clamp arms 1 is in a range of from ±10°.

As shown in FIGS. 81 to 82, schematic views of the rotation connection structure 3 of the portable fan 100 in a second implementation are shown. In the present implementation, the pivot 33 that extends through the first pieces 311 and the second pieces 321 is omitted, and instead, one of the first hinge member 31 and the second hinge member 32 is arranged with a pivot portion 322, and the other one of the first hinge member 31 and the second hinge member 32 defines a pivot hole 312. The pivot portion 322 is fixed in the pivot hole 312 to secure the first hinge member 31 and the second hinge member 32. The pivot portion 322 and the pivot hole 312 are formed directly into the first hinge member 31 and the second hinge member 32 themselves without arranging any additional element, such that mounting operations are simplified. Two pivot portions 322 and two pivot holes 312 are arranged corresponding to each other. The two pivot portions 322 and the two pivot holes 312 are respectively arranged on opposite sides of the plurality of first pieces 311 and the plurality of second pieces 321. Other structures and performance of the present implementation are substantially the same as those in the first implementation and will not be repeated herein.

### Embodiment 9

The portable fan of the present disclosure includes a housing 1, a shaft tube 11 arranged inside the housing 1, a rotation shaft 12 received in a shaft hole of the shaft tube 11 and rotatable with respect to the shaft tube 11, a stator sleeving an outside of the shaft tube 11, and an impeller 2 connected to a top of the rotation shaft 12. In the present embodiment, the portable fan is used as an example for illustration, and obviously, the portable fan may also be applied to a desktop fan, a handheld fan, a fan for a baby carriage, and other devices.

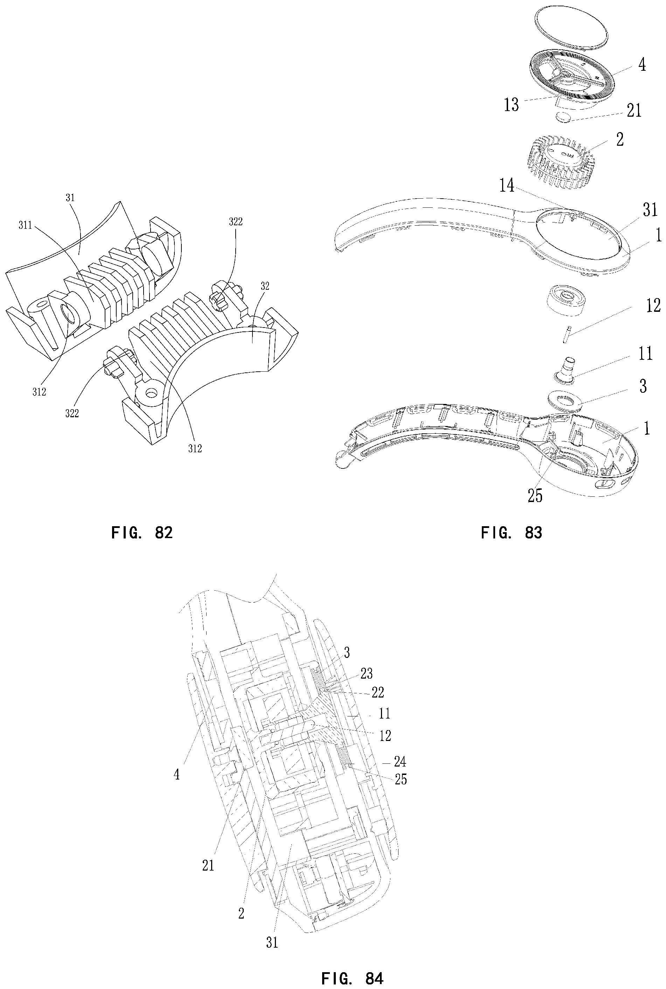

As shown in FIG. 83 and FIG. 84, the portable fan further includes an anti-vibration member disposed at the end of the shaft tube 11. The impeller 2 oscillates to drive the shaft tube 11 to vibrate. The anti-vibration member is connected between the housing 1 and the shaft tube 11. The anti-vibration member prevents the vibration of the impeller 2 from being transmitted to the housing 1, such that noise is avoided, and the user experience is not affected.

The anti-vibration member and the shaft tube 11 are detachably connected to each other. The anti-vibration member and the housing 1 are detachably connected to each other. The shaft tube 11 is connected to the housing 1 through the anti-vibration member. When the portable fan is being used, the impeller 2 oscillates to drive the shaft tube 11 to vibrate. Since the anti-vibration member is connected between the housing 1 and the shaft tube 11, the anti-vibration member prevents the vibration of the impeller 2 from being transmitted to the housing 1, such that noise is avoided, and the user experience is not affected.

As shown in FIG. 83, FIG. 84, FIG. 85, and FIG. 86, the anti-vibration member is an annular ring 3. An inner periphery of the annular ring 3 is snapped with the shaft tube 11. An end of the shaft tube 11 extends outwardly along a radial direction to form a first protrusion 22. An inner wall of the annular ring 3 defines an inner groove 23 that is recessed away from an axial center of the annular ring 3. The first protrusion 22 is inserted into the inner groove 23. A gap is reserved between the first protrusion 22 and the inner groove 23. When the shaft tube 11 vibrates, the first protrusion 22 is driven to vibrate. Since the gap is reserved between the first protrusion 22 and the inner groove 23, a vibration amplitude of the first protrusion 22 is smaller than the gap between the first protrusion 22 and the inner groove 23, such that vibration that can be transmitted to the housing 1 is reduced. In addition, the above configuration can be assembled conveniently and highly efficiently.

As shown in FIG. 83, FIG. 84, FIG. 85 and FIG. 86 and FIG. 89, an outer periphery of the annular ring 3 is snapped with the housing 1. An outer wall of the annular ring 3 defines an outer groove 24 recessed towards the axial center of the annular ring 3. The housing 1 is arranged with a second protrusion 25 snapped with the outer groove 24. The second protrusion 25 is inserted into the outer groove 24. A gap is reserved between the second protrusion 25 and the outer groove 24. When the shaft tube 11 vibrates, the second protrusion 25 is driven to vibrate. Since the gap is reserved between the second protrusion 25 and the outer groove 24, a vibration amplitude of the second protrusion 25 is smaller than the gap between the second protrusion 25 and the outer groove 24, such that vibration that can be transmitted to the housing 1 is reduced. In addition, the above configuration can be assembled conveniently and highly efficiently.

As shown in FIG. 83, FIG. 84, FIG. 85 and FIG. 86 and FIG. 89, the annular ring 3 is made of a soft material. The annular ring 3 can be deformed. When the portable fan is started, and when the impeller 2 oscillates up and down and vibrates, the shaft tube 11 is driven to vibrate, and the vibration is transmitted to the annular ring 3. The vibration causes the annular ring 3 to be deformed. A part of the vibration is stored as energy in the deformed annular ring 3, and another part of the energy is transmitted to the housing 1. Therefore, vibration energy transmitted to the housing 1 is reduced, the generated noise and the vibration amplitude are also reduced.

As shown in FIG. 83, FIG. 84, FIG. 85 and FIG. 86, the housing 1 encloses to define a mounting channel 31 having an opening at an end. The opening is located corresponding to the impeller 2. The impeller 2 can be taken out from the mounting channel 31 to be cleaned. The rotation plate 4 is disposed at the opening of the housing 1. The rotation plate 4 is configured to cover the mounting channel 31 to prevent the hair from entering the mounting channel 31.

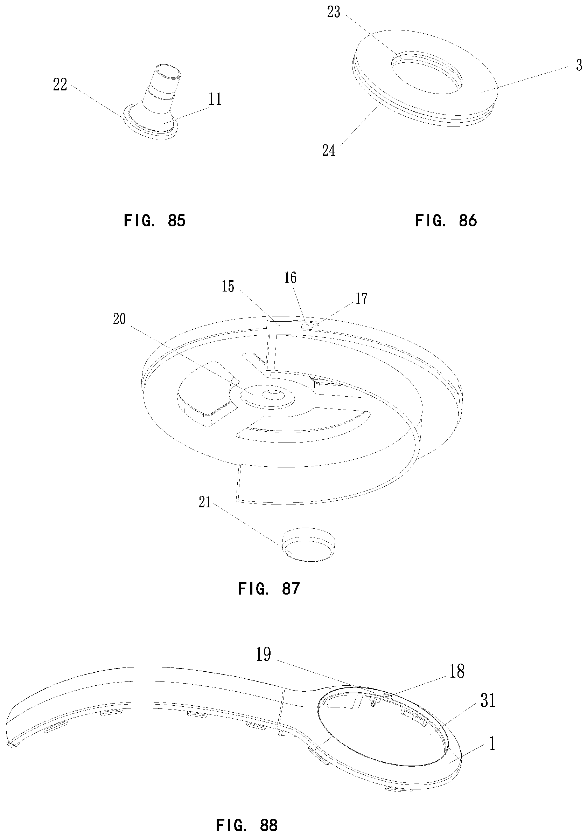

As shown in FIG. 87 and FIG. 88, the rotation plate 4 is detachably connected to the housing 1. When the rotation plate 4 is disassembled from the housing 1, the impeller may be conveniently taken out. One of the rotation plate 4 and the housing 1 is arranged with a rotation fastener 14, and the other one of the rotation plate 4 and the housing 1 defines a rotation fastener groove 13. Of course, in other embodiments, one of the rotation plate 4 and the housing 1 is arranged with outer threads, and the other one of the rotation plate 4 and the housing 1 is arranged with inner threads, such that the rotation plate 4 and the housing 1 are threadedly connected to each other. Of course, in other embodiments, one of the rotation plate 4 and the housing 1 is arranged with a first magnet, and the other one of the rotation plate 4 and the housing 1 is arranged with a second magnet, such that the rotation plate 4 and the housing 1 are magnetically connected to each other.

One of the rotation plate 4 and the housing 1 is arranged with the rotation fastener 14, and the other one of the rotation plate 4 and the housing 1 defines the rotation fastener groove 13. The rotation fastener groove 13 has a first position and a second position, and the rotation fastener 14 may rotate to switch between the position and the second position. The rotation plate 4 and the housing 1 rotate to switch between a first mating position and a second mating position. When the rotation plate 4 and the housing 1 are at the first mating position, the rotation fastener 14 is inserted into the first position of the rotation fastener groove 13. When the rotation plate 4 and the housing 1 are at the second mating position, the rotation fastener 14 is inserted into the second position of the rotation fastener groove 13. In this way, relative displacement of the rotation plate 4 and the housing 1 is limited. By sliding the rotation plate 4 and the housing 1 to switch between positions, the rotation fastener 14 is enabled to enter the second position of the rotation fastener groove 13. Due to relative movement of the rotation fastener groove 13 and the rotation fastener 14 being limited, connection between the rotation plate 4 and the housing 1 can be achieved.

An outer wall of the rotation plate 4 defines the rotation fastener groove 13, and an inner wall of the mounting channel 31 of the housing 1 having the opening is arranged with the rotation fastener 14. The outer wall of the rotation plate 4 defines a first recess 15 and a second recess 16 along the radial direction thereof. The first recess 15 and the second recess 16 are communicated to each other. The first position of the rotation fastener groove 13 is corresponding a position of the first recess 15, and the second position of the rotation fastener groove 13 is corresponding to a position of the second recess 16. A first resilient fastener 17 is arranged along the radial direction of the rotation plate 4 protruding into the second recess 16.

The inner wall of the opening of the housing 1 defines a third recess 19. The third recess 19 is extending in the radial direction of the housing 1. A second resilient fastener 18 is arranged at a side of the third recess 19 near the rotation plate 4. The second resilient fastener 18 is stretching along a direction extending from the opening of the housing 1 to a diameter of the housing 1. The second resilient fastener 18 is connected with the housing 1. When the second resilient fastener 18 slides from the first recess 15 to the second recess 16, an axial relative displacement of the rotation plate 4 and the housing 1 is limited.

A bottom surface of a center of the rotation plate 4 facing towards the impeller 2 defines a fourth recess 20, and an anti-hitting member 21 is arranged on the bottom surface of the rotation plate 4 facing towards the impeller 2. The anti-hitting member 21 is mounted at the fourth recess 20. The anti-hitting member 21 prevents the impeller 2 from hitting, during rotating, the rotation plate 4 to produce any sound, such that noise is reduced.

The anti-hitting member 21 is made of EVA foam or soft silicone. The anti-hitting member 21 is mounted between the impeller 2 and the rotation plate 4, such that collision of the impeller 2 is effectively reduced, and a vibration absorption effect is achieved.

### Embodiment 10

For ease of description, an up-down direction is defined as a first direction X, a positive direction of the direction X is an upward direction. A left-right direction is defined as a second direction Y, and a positive direction of the direction Y is a leftward direction. A front-rear direction is defined as a third direction Z, and a positive direction of the direction Z is a forward direction. Any two of the first direction X, the second direction Y, and the third direction Z are perpendicular to each other.

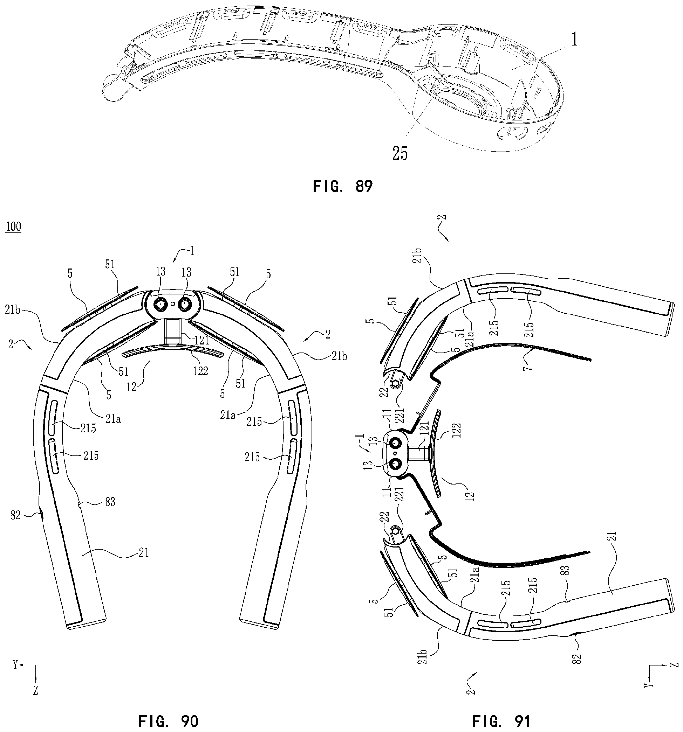

As shown in FIGS. 90, 93 and 94, schematic views of the portable fan 100 of a first implementation are shown.

The portable fan 100 includes a connection member 1 and two clamp arms 2 pivotally connected to the connection member 1. The two clamp arms 2 are respectively pivotally connected to a left end and a right end of the connection member 1, such that a distance between free ends of the two clamp arms 2 can be adjusted. By arranging the connection member 1 to be connected to the two clamp arms 2, the free ends of the two clamp arms 2 can be moved away from each other, enabling the user to put on and take off the portable fan 100 easily. In addition, the free ends of the two clamp arms 2 can be moved closer to each other, enabling the portable fan 100 to be stably fixed to the neck, such that the portable fan 100 may not fall off easily; and enabling the portable fan 100 to be stored easily.

As shown in FIGS. 90 to 92, at a mounting position between the connection member 1 and each clamp arm 2, the connection member 1 has a first mating surface 11 facing the clamp arm 2, and the clamp arm 2 has a second mating surface 22 facing the connection member 1. The first mating surface 11 and the second mating surface 22 are two curved surfaces parallel to each other. In the present embodiment, the second mating surface 22 is a concave surface, and the clamp arm 2 is arranged with a convex portion 221. The convex portion 221 is protruding from the second mating surface 22. The first mating surface 11 is a convex surface, and the connection member 1 defines a snap slot 111. The snap slot 111 is recessed from the first mating surface 11. The pivot 13 is connected to and extends through the convex portion 221 and the snap slot 111 in the first direction X, allowing the convex portion 221 and the snap slot 111 to be stably pivotally connected to each other. A size of the snap slot 111 is greater than a size of the convex portion 221. When the convex portion 221 is inserted into the snap slot 111, and when the pivot 13 is connected to and extends through the convex portion 221 and the snap slot 111 in the first direction X, the snap slot 111 leaves a space for the convex portion 221 to pivot, and an inner wall of the snap slot 111 limits a position of the convex portion 221 during pivoting.

In other embodiments, the first mating surface 11 may be a concave surface, and the connection member 1 may be arranged with the convex portion 221. The convex portion 221 is protruding from the first mating surface 11, and the second mating surface 22 is a convex surface. The clamp arm 2 defines the snap slot 111. The snap slot 111 is recessed from the second mating surface 22. The pivot 13 is connected to and extends through the convex portion 221 and the snap slot 111 in the first direction X, allowing the convex portion 221 and the snap slot 111 to be stably pivotally connected to each other. The size of the snap slot 111 is greater than the size of the convex portion 221. The inner wall of the snap slot 111 limits the position of the convex portion 221 during pivoting.

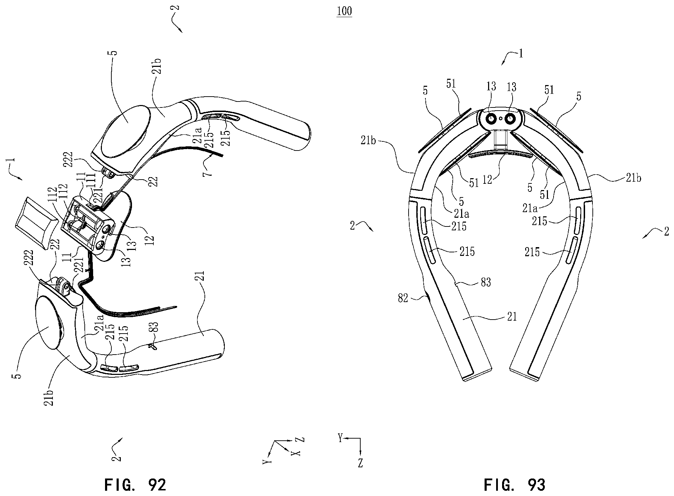

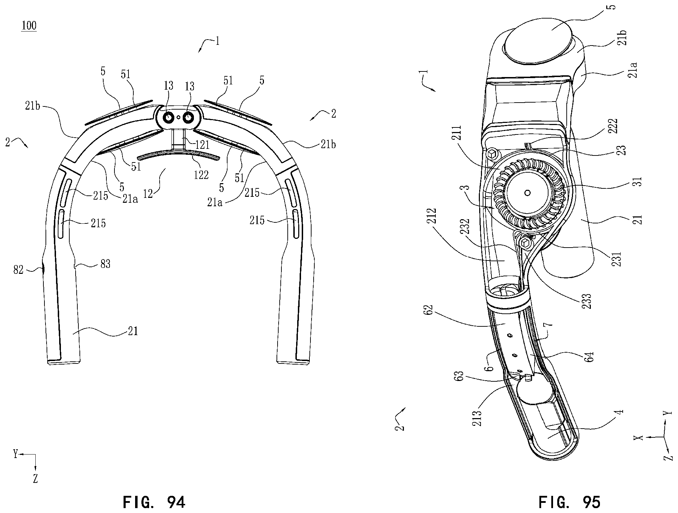

As shown in FIGS. 90 and 93, when two convex portion 221 are pivoted to enable the free ends of the two clamp arms 2 to be close to each other, the portable fan 100 occupies a smallest space, and the opening between the two clamp arms 2 is the smallest in size, facilitating the portable fan 100 to be fixedly worn to the neck. A distance between the free ends of the two clamp arms 2 is in a range of 0mm to 16mm. As shown in FIGS. 90 and 94, when the two convex portions 221 are pivoted to enable the free ends of the two clamp arms 2 to reach positions that are furthest away from each other, the opening between the two clamp arms 2 is the largest, facilitating the user to put on and take off the portable fan 100. The portable fan 100 can be worn to necks of various sizes. The distance between the free ends of the two clamp arms 2 is in a range of 160.3mm to 162.7 mm. It is understood that the two clamp arms 2 may be maintained at positions corresponding to positions where the two convex portions 221 stop pivoting. That is, a damping force is generated between the pivot 13 and the convex portions 221 and between the pivot 13 and the snap slot 111. When the convex portions 221 stop pivoting, the clamp arms 2 as a whole can be positioned at a corresponding angle. A pivot angle of the convex portions 221 is in a range of ±10°. Each convex portion 221 pivots infinitely within the range of the pivot angle. Therefore, the distance between the two clamp arms 2 is infinitely variable between an extremely closed position and an extremely furthest position.

As shown in FIGS. 91, 92, 95 and 98, a surface of the convex portion 221 is recessed to define a wire slot 222. The wire slot 222 extends through the second mating surface 22 to communicate with a space inside the housing 21. A wall of the snap slot 111 defines a wire hole 112. Two wire holes 112 are communicated with each other. The wire slot 222 and the wire holes 112 are communicated to each other to allow a wire 7 in one clamp arm 2 to extend through the wire slot 222 and the wire hole 112 to enter the other clamp arm 2. The wire slot 222 is recessed from the surface of the convex portion 211 and has a sufficient space, and the wire hole 112 also has a sufficiently space. In this way, when the clamp arm 2 is pivoting, the clamp arm 2 does not compress the wire 7. When the first mating surface 11 is a concave surface and the convex portion 221 is protruding from the first mating surface 11, the wire slot 222 extends through the first mating surface 11.

As shown in FIGS. 90, 93 and 94, the connection member 1 is arranged with a support portion 12 protruding from the connection member 1 corresponding to the rear neck of the user. The support portion 12 includes a support bar 121 and a support pad 122 connected to the support bar 121. The support pad 122 is attached to the neck to separate the connection member 1 from the rear neck, such that pivoting between the clamp arms 2 and the connection member 1 is prevented from affecting the rear neck, and the clamp arms 2 are prevented from clamping the rear neck during pivoting. A length of the support pad 122 in the second direction Y is greater than a length of the connection member 1 in the second direction Y. When a surface area of the support pad 122 is large, a contact area between the support pad 122 and the rear neck is large, and the user wears the portable fan more comfortably. In addition, the longer support pad 122 can abut against the two clamp arms 2, such that the two clamp arms 2 are prevented to be disposed too closed to each other, and the neck is prevented from being pinched painfully by the two clamp arms 2.

As shown in FIG. 95, FIG. 96, FIG. 98, and FIG. 99, each clamp arm 2 includes a housing 21. The housing 21 defines a receiving cavity 211, an air duct 212, and a battery receiving chamber 213 sequentially along an extension direction of the housing 21. The receiving cavity 211 is located close to the connection member 1. The receiving cavity 211 receives the fan assembly 3. The fan assembly 3 includes centrifugal fan blades 31 and a motor 32. The motor 32 drives the centrifugal fan blades 31 to rotate. The battery receiving chamber 213 is located at a free end. Each of two battery receiving chambers 213 of the two clamp arms 2 receives one battery 4. The battery 4 supplies power to the corresponding fan assembly 3. Two fan assemblies 3 in the two clamp arms 2 may operate simultaneously or independently.

As shown in FIGS. 95, 96, 98 and 99, an axis of the centrifugal fan blades 31 is perpendicular to the first direction X. That is, the centrifugal fan blades 31 are arranged vertically in the first direction X. In the present embodiment, the battery 4 is a cylindrical battery 4. An extending direction of the battery 4 is the same as an extending direction of the battery receiving chamber 213. The receiving cavity 211 needs to receive the centrifugal fan blades 31 that are arranged vertically, and therefore, the receiving cavity 211 needs to have a larger height. The battery receiving chamber 213 extends substantially along the third direction Z, and therefore, a height of the battery receiving chamber 213 may be less. The height of the receiving cavity 211 in the first direction X is 1.45 to 2 times of the height of the battery receiving chamber 213 in the first direction X. When the user is wearing the portable fan 100, corresponding to the rear neck, the support portion 12 is arranged to support the connection member 1 and the two receiving cavities 211; and corresponding to a left neck, a right neck, and a front chest, the battery receiving chamber 213 having a compact size is arranged. In this way, an aesthetic appearance is provided. In addition, when the portable fan is worn to the user, the two batteries 4 in the two battery receiving chambers 213 are located forward and downward, and centrifugal fan blades 31 and two motors 32 of the two fan assemblies are located backward and downward. A front and a rear of the portable fan 100 is gravitationally balanced to each other. The portable fan 100 is more closely attached to the neck of the user and can be worn more comfortably. The user may not be tired when wearing the fan 100 for a long period of time.

As shown in FIG. 95, FIG. 96, FIG. 98 and FIG. 99, the housing 21 defines two air inlets 214 respectively on two opposite sides of each receiving cavity 211 and defines the air outlet 215 on a side wall of the air duct 212. The air inlets 214, the receiving cavity 211, the air duct 212, and the air outlet 215 are communicated with each other sequentially. When the user is wearing the portable fan 100, the air outlet 215 is directed at least towards a chin and a face of the user and outputs airflow to cool down parts of the user that need cooling the most. Of course, the air outlet 215 may further be provided to face towards the left neck, the right neck, and a downward of the neck. Specifically, the housing 21 includes a first housing 21a facing towards the neck and a second housing 21b facing away from the neck. Each of the first housing 21a and the second housing 21b defines the air inlet 214 corresponding to the centrifugal fan blades 31. The air inlet 214 defined in the first housing 21a facing towards the neck may absorb the air from the neck, such that the rear neck where no air outlet 215 is defined can also be cooled. Vertical projections of the two second mating surfaces 22 in the first direction X forms two arcs. A radius of a circle where each of the two arcs is located is in a range of 7.5mm to 9.5 mm. A distance between centers of two circles where the two arcs are located is in a range of 15mm to 26 mm. In this way, a distance between the two fan assemblies 3 is limited, such that an area of air intaken and cooling for the rear neck is increased, and turbulence of airflows from the two fan assemblies 3 is prevented.

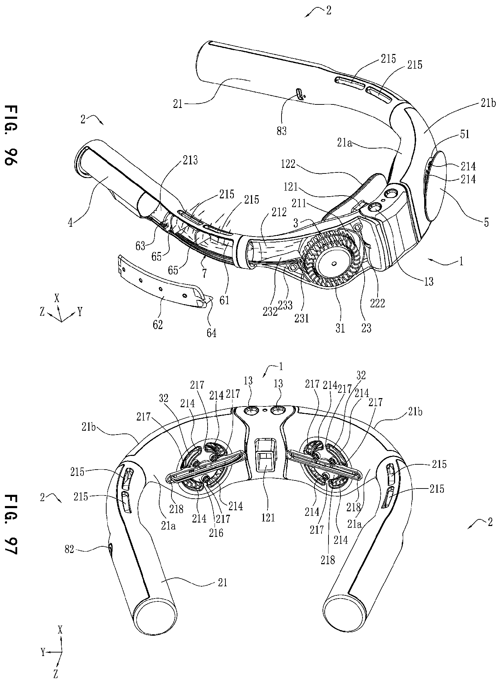

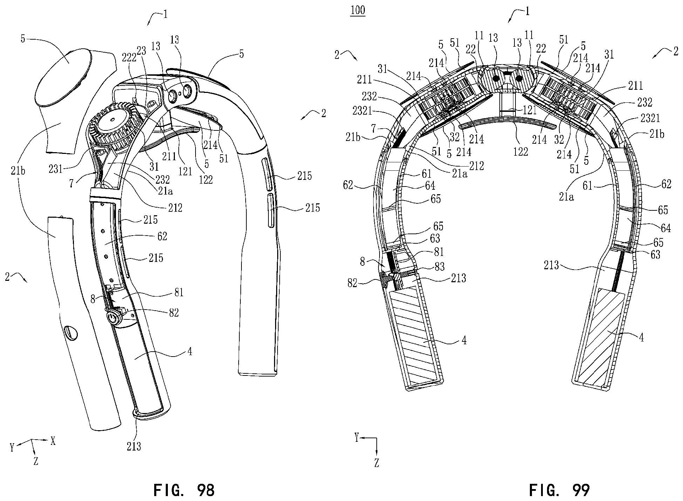

As shown in FIGS. 97 and 99, a mounting portion 216 and a connection portion 217 connected to the mounting portion 216 and the housing 21 are arranged at each air inlet 214. The fan assembly 3 is mounted in the mounting portion 216, and the motor 32 is disposed corresponding to the mounting portion 216. The mounting portion 216 and the connection portion 217 are protruding to form a wire space 218. The wire space 218 extends from two ends of the connection portion 217 to a portion of the housing 21. A shielding plate 5 is arranged at each air inlet 214, fixed to the mounting portion 216, and covers the air inlet 214 and the wire space 218. In this way, hair or other debris are prevented from entering the air inlet 214, and the wire 7 is not exposed, a neater and aesthetic appearance is provided. In the present embodiment, the wire space 218 is formed by the mounting portion 216 and the connection portion 217 protruding from a side of the first housing 21a. It is understood that the wire space 218 may alternatively be formed by the mounting portion 216 and the connection portion 217 protruding from a side of the second housing 21b. A gap 51 is formed between the shielding plate 5 and the housing 21 to allow external airflow to flow through the gap 51 to reach the air inlet 214. When the two clamp arms 2 are pivoted to be close to each other, the support pad 122 abuts against the two shielding plates 5 located on the side of the first housing 21 to limit the distance between the two fan assemblies 3, such that turbulence is prevented, and an airflow outputting effect is improved.

As shown in FIGS. 95, 96, 98 and 99, a partition plate 6 is received in the air duct 212. The partition plate 6 is connected to an inner wall of the air duct 212. The partition plate 6 includes a first plate 61 adjacent to the air outlet 215, a second plate 62 opposite to the first plate 61, and a third plate 63 connected to the first plate 61 and the second plate 62. The first plate 61 is attached to a portion of the inner wall of the air duct 212. A spacing is defined between at least a portion of the second plate 62 and the portion of the inner wall of the air duct 212. The third plate 63 separates the air duct 212 from the battery receiving chamber 213. The partition plate 6 further includes a fourth plate 64, which is connected to the first plate 61, the second plate 62, and the third plate 63, and is opposite to the air outlet 215. A spacing is formed between at least a portion of the fourth plate 64 and the portion of the inner wall of the air duct 212. Each housing 21 includes at least two air outlets 215. The partition plate 6 is arranged with an air guiding plate 65 corresponding to each air outlet 215. The air guiding plate 65 is connected to the first plate 61 and the second plate 62 and has a curved surface. It is understood that the partition plate 6 and a wall of the housing 21 may be formed as a one-piece and integral structure or may be separately moulded and then assembled to each other. The first plate 61, the second plate 62, the third plate 63, the fourth plate 64 and the air guiding plate 65 may be each individually moulded and then assembled to each other; or some of which are formed as a one-piece and integral structure, and the rest of which are separately moulded and then assembled to each other; or all of which are formed as a one-piece and integral structure.

From the taller receiving cavity 211, through the air duct 212, to the smaller battery receiving chamber 213, the air duct 212 has a smaller outer contour. In addition, the partition plate 6 is received in the air duct 212. The spacing is defined between at least a portion of the second plate 62 and the inner wall of the air duct 212. The spacing is defined between at least a portion of the fourth plate 64 and the inner wall of the air duct 212. In this way, the air duct 212 is divided into smaller air sub-ducts 212. The airflow blown from the fan assembly 3 flows through the smaller air sub-ducts 212 to be blown out of the housing through the air outlet 215. In this way, the airflow strength is increased, and the cooling effect is better.

As shown in FIGS. 95, 96, 98 and 99, the housing 21 is arranged with a cavity plate 23. In the present embodiment, the cavity plate 23 is integrally formed with the housing 21. A portion of the cavity plate 23 encloses to form the receiving cavity 211, and a portion of the cavity plate 23 forms a part of the air duct 212. In other embodiments, the cavity plate 23 may be separately moulded and mounted to the housing 21. The cavity plate 23 includes a shielding portion 231 and a guiding portion 232 formed at a connection between the receiving cavity 211 and the air duct 212. The shielding portion 231 forms at least a part of the receiving cavity 211, and the guiding portion 232 forms at least a part of the air duct 212. The shielding portion 231 and the guiding portion 232 are connected to each other. A wire receiving chamber 233 is formed by the shielding portion 231, the guiding portion 232 and the housing 21. The guiding portion 232 defines a through hole 2321 allowing the wire 7 to pass through.

As shown in FIGS. 98 and 99, the housing 21 is further arranged with a main control module 8 therein. The main control module 8 is received in one battery receiving chamber 213. The main control module 8 includes a main board 81, an adjustment key 82 electrically connected to the main board 81, and an interface 83 electrically connected to the main board 81. The adjustment key 82 is configured to turn on and off the two fan assemblies 3 and to control a wind speed of the two fan assemblies 3. The interface 83 is configured to be connected to an external power supply to charge of the battery 4.

As shown in FIGS. 95 to 99, the wire 7 is electrically connected to the two batteries 4. The wire 7 is electrically connected to one battery 4 and the main board 81. The wire 7 between the battery 4 and the main board 81 that are located in a same battery receiving chamber 213 does not need extend through any structure to achieve the connection. The wire 7 of the battery 4 located in another battery receiving chamber 213 needs to extends from one clamp arm 2, through the wire slot 222 and the wire hole 112, into the other clamp arm 2. Specifically, the wire 7 extends through the spacing between the fourth plate 64 and the inner wall of the air duct 212 to enter the air duct 212. The wire extends from the air duct 212 through the through hole 2321 of the guiding portion 232 to enter the wire receiving cavity 233. The wire extends out of the housing 21 through the wire receiving cavity 233 to enter the wire space 218. The wire extends from the wire space 218 of one clamp arm 2, passing through the wire slot 222, the wire hole 112, the wire hole 112, the wire slot 222, to enter the wire space 218 of the other clamp arm 2. The wire further extends into the housing 21 through the wire space 218 to enter the wire receiving cavity 233. The wire extends through the through hole 2321 to enter the air duct 212. The wire extends from the air duct 212 through the spacing between the fourth plate 64 and the inner wall of the air duct 212 to enter the battery receiving chamber 213. **In** this way, the wire is connected to the other battery 4.

As shown in FIGS. 95 to 99, the wire 7 is electrically connected to the motor 32 and the main board 81. The wire 7 is electrically connected to the main board 81 and the battery 4. Therefore, the motor 32 and the battery 4 are electrically connected to each other through the main board 81 and the wire 7. Specifically, the wire 7, which is connected to the motor 32 located in the same clamp arm 2 as the main board 81, sequentially extends out of the housing 21 into the wire space 218, extends into the housing 21 to enter the wire receiving chamber 233, extends through the through hole 2321 to enter the air duct 212, extends from the air duct 212 through the spacing between the fourth plate 64 and the inner wall of the air duct 212 to enter the battery receiving chamber 213, so as to be connected to the main board 81. The wire 7, which is connected to the motor 32 located in the other clamp arm 2, sequentially extends out of the housing 21 to enter the wire space 218, extends from the wire space 218 of one clamp arm 2 through the wire slot 222, the wire hole 112, the wire hole 112, and the wire slot 222 to enter the wire space 218 of the other clamp arm 2, extends into the housing 21 to enter the wire receiving chamber 233, extends through the through hole 2321 to enter the air duct 212, extends from the air duct 212 through the spacing between the fourth plate 64 and the inner wall of the air duct 212 to enter the battery receiving chamber 213, so as to be connected to the main board 81.

The shielding plate 5 covers the wire space 218. Therefore, the wire 7 is not exposed to the outside, and a neater and aesthetic appearance is provided. The wire 7 is prevented from external steams and sunshine exposure. Safety performance of the product is improved, and a service life of the wire 7 is extended.

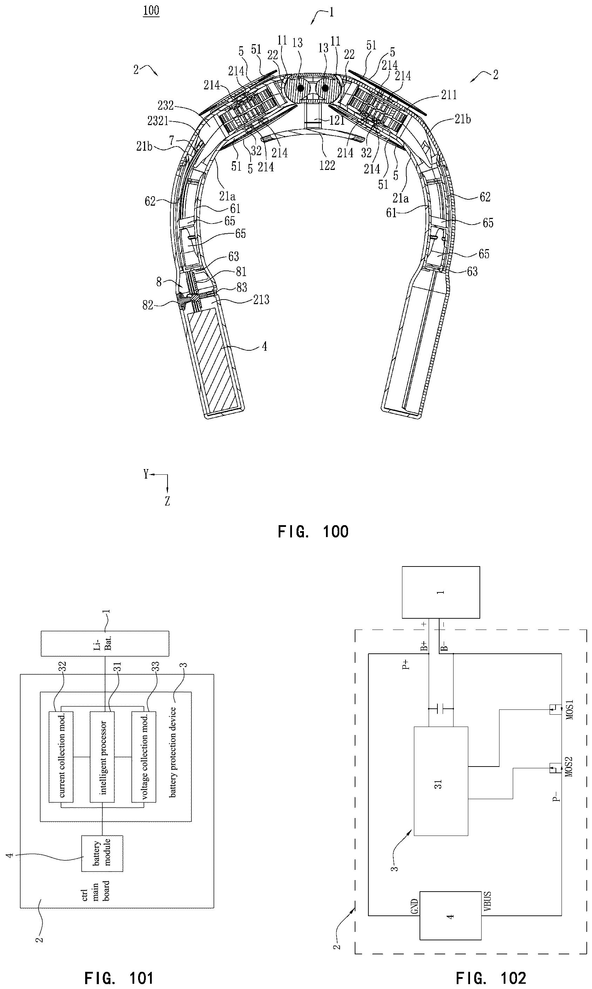

As shown in FIG. 100, a schematic view of the portable fan 100 of a second implementation is provided. In the present implementation, one of the two battery receiving chambers 213 receives one battery 4, and the one battery 4 is electrically connected to the two fan assemblies 3 at the same time. Compared to the first implementation, in the present implementation, the fourth plate 64 is omitted. **In** each clamp arm 2, the air guiding plate 65 near the battery receiving chamber is extending backward to be disposed near the other air guiding plate 65. In this way, the airflow is enabled to blow smoothly through the two air guiding plates 65 to be blown out of the housing through two air outlets 215. In the present implementation, only one battery 4 is arranged. The battery 4 and the main board 81 are located in the same battery receiving chamber 213 and are electrically connected to each other via the wire 7. The two motors 32 are electrically connected to the main board 81 via the wire 7. Other structures and performance in the present implementation are substantially the same as those in the first implementation and will not be repeated herein.

It is understood that one or two batteries 4 may be arranged in the portable fan 100. In the above implementations, the batteries 4 are electrically connected to the main board 81 via the wire 7, and the main board 81 is electrically connected to the motor 32 via the wire 7. In other embodiments, another order of the connection may be applied.

### Embodiment 11

As shown in FIG. 101 and FIG. 102, a partial schematic view and a partial circuit diagram of the portable fan 100 of a first implementation are shown, where the partial schematic view and the partial circuit diagram are simplified. For example, a drive circuit between the control main board 2 and the fan assembly 6 is omitted. A battery protection device 3 usually includes pins B+, B-, P+ and P-. The battery protection device 3 usually includes an intelligent processor 31, a current collection module 32 and a voltage collection module 33. The intelligent processor 31 is connected to the current collection module 32 and the voltage collection module 33. The intelligent processor 31 may be an IC control chip, or other types of chips that are not limited herein. The voltage collection module 33 operates as follows. The pins B+ and B- of the battery protection device 3 are respectively connected to a positive electrode and a negative electrode of a lithium ion battery 1. In this way, the battery protection device 3 monitors a voltage between the positive electrode and the negative electrode of the lithium ion battery 1. The current collection module 32 operates as follows. A MOS1 and a MOS2 are connected to each other in series on a circuit, such that the MOS1 and the MOS2 monitor a current of the circuit.

The intelligent processor 31 monitors, by the voltage collection module 33 and the current collection module 32, the voltage of the lithium ion battery 1 and the current of the circuit to control the MOS1 and the MOS2 to be on or off. The MOS1 and the MOS2 serve as switches in the circuit to respectively control a charging circuit and a discharging circuit to be conducted or disconnected. During normal operation, when the voltage of the lithium ion battery 1 is within a range of A to B, both the MOS1 and the MOS2 are in an on state. When the intelligent processor 31 determines, based on a monitoring result of the voltage collection module 33, that the voltage of the lithium ion battery 1 reaches a value of B, the intelligent processor 31 controls the MOS1 (the charging circuit) to be off, such that the charging circuit is disconnected, and an external power source cannot charge the lithium ion battery 1. In this way, an overcharging protection is provided. When the intelligent processor 31 determines, based on the monitoring result of the voltage collection module 33, that the voltage of the lithium ion battery 1 is lower than a value of A, the intelligent processor 31 controls the MOS2 (the discharging circuit) to be off, such that the discharging circuit is disconnected, and the lithium ion battery 1 is not discharged to any load. In this way, a discharging protection is provided. During the lithium ion battery 1 being normally discharged to a load, a discharge current passes through the MOS1 and the MOS2 that are connected to each other in series. Due to a conduction impedance of the MOS 1 and the MOS2, a voltage is generated at two ends of the MOS1 and the MOS2, and the intelligent processor 31 detects a value of the generated voltage. When the load is abnormal due to some reasons, resulting in the current of the circuit being increased, and when the current of the circuit is increased to enable the voltage between the ends of the MOS1 and the MOS2 to be greater than a value of C, the intelligent processor 31 controls the MOS1 and the MOS2 to be off, such that the discharging circuit is disconnected, the current of the circuit is turned to zero, and therefore, an overcurrent protection is provided. During the lithium ion battery 1 being discharged to the load, when the current of the circuit is increased to enable the voltage between the ends of the MOS1 and the MOS2 to be greater than a value of D (D>C), the intelligent processor 31 determines that the load is short-circuited and controls the MOS2 (the discharging circuit) to be off, such that the discharging is disconnected, and therefore, a short-circuit protection is provided. The current collection module 32 and the voltage collection module 33 are both connected to the lithium ion battery 1 to monitor the current and the voltage of the lithium ion battery 1. Since the intelligent processor 31, the current collection module 32, and the voltage collection module 33 operate together, the lithium ion battery 1 is prevented from overvoltage, undervoltage, overcurrent, and short circuits, such that an operation state of the lithium ion battery 1 is intelligently controlled.

The battery protection device 3 is arranged on the control main board 2. Therefore, when the lithium ion battery 1 needs to be disassembled, the lithium ion battery 1 can be directly removed from the portable fan 100, and another lithium ion battery 1 may be reassembled or replaced. In this way, disassembling and assembling of the battery protection device 3 with the lithium ion battery 1 can be reduced, facilitating inspection and replacement of the lithium ion battery 1.

As shown in FIGS. 101 to 102, the control main board 2 is arranged with a power supply module 4. The pins P- and P+ of the battery protection device 3 are respectively connected to pins of the power supply module 4, such that the current collection module 32 and the voltage collection module 33 are connected to the power supply module 4 to monitor a current and a voltage of the power supply module 4. Specifically, the power supply module 4 includes a charging interface 41, and in the present embodiment, the charging interface 41 is a TYPE-C female port. Of course, in other embodiments, the charging interface 41 may be a port in other connection types. During charging, pins VBUS and GND are respectively connected to the pins P- and P+ of the battery protection device 3, a conducted TYPE-C male terminal is inserted into the charging interface 41 to allow the charging interface 41 to intake power from an external power source. The charging interface 41 supplies power to the battery protection device 3 via the pins VBUS and GND, and the battery protection device 3, after being supplied with the power, charges power to the lithium ion battery via the pins B - and B+. During discharging, the lithium-ion battery 1, after being charged, supplies power to the control board 2 through the pins P- and P+ of the battery protection device 3. Therefore, a wire, which is connected between the lithium ion battery 1 and the control main board 2 for power supplying, can be eliminated. In this way, a circuit configuration of the portable fan 100 is simplified. The control main board 2 is further arranged with a switch 7 and a motor (not shown, the same hereinafter). The switch 7 is exposed outwardly to be operated by the user. Only when the switch 7 is turned on, the lithium ion battery 1 can supply power to the motor of the control main board 2. The motor drives the fan assembly 6 to rotate to activate the portable fan 100, otherwise, the portable fan 100 cannot be activated.

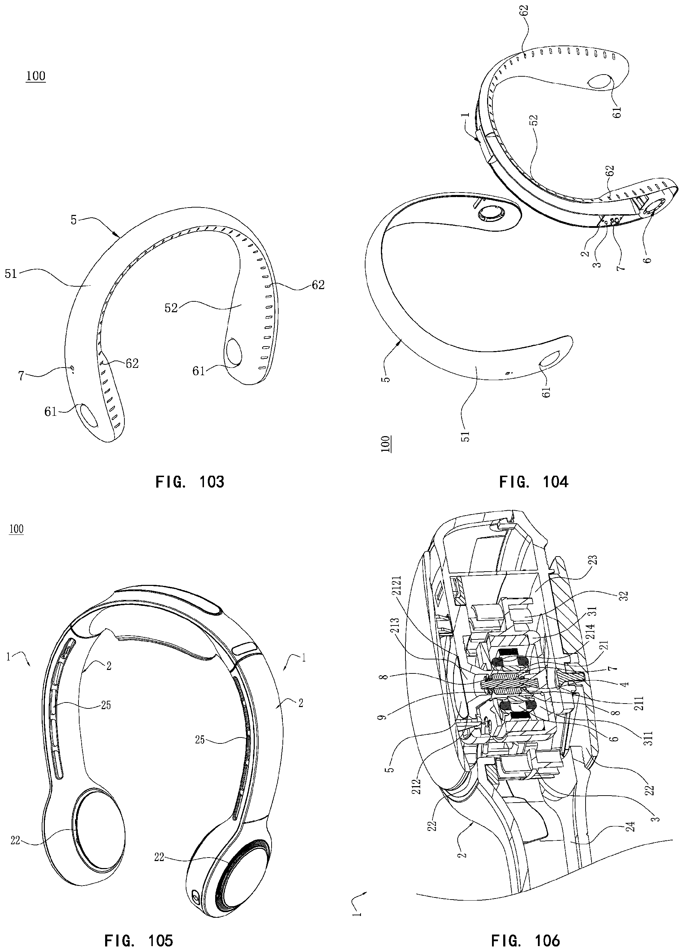

As shown in FIG. 103 and FIG. 104, wires between various components are also omitted in FIG. 104. The portable fan 100 is arranged with a housing 5, a fan assembly 6 arranged inside the housing 5, the lithium-ion battery 1 arranged inside the housing 5, the control main board 2 arranged inside the housing 5, and the battery protection device 3 arranged inside the housing 5. The housing 5 is arranged with an air inlet portion 61 and an air outlet portion 62. The housing 5 includes a first housing 51 and a second housing 52 mated with the first housing 51. The lithium ion battery 1 is configured to supply power to the portable fan 100. The fan assembly 6 rotates to intake air from the air inlet portion 61 and drives the air to be output from the air outlet portion 62. The control main board 2 is configured to control the portable fan 100. The battery protection device 3 is configured to protect the lithium ion battery 1. Further, the power supply module 4 is not shown in FIG. 104. It is to be understood that the power supply module 4 may also be arranged on the control main board 2. Other structures and functions of the present embodiment are the same as those in the first embodiment and will not be repeated herein.

Of course, the portable fan 100 is not limited to a portable fan, and the portable fans 100 applicable in other application scenarios fall within the scope of the present disclosure.

In summary, the portable fan 100 herein has the following beneficial effects:
The battery protection device 3 is arranged on the control main board 2, such that the lithium ion battery 1 can be directly removed from the portable fan 100 when the lithium ion battery 1 needs to be disassembled, and then another lithium ion battery 1 can be reassembled or replaced. Disassembling and assembling of the battery protection device 3 with the lithium ion battery 1 can be reduced, facilitating inspection and replacement of the lithium ion battery 1.

When the battery protection device 3 is electrically connected to the control main board 2, the battery protection device 3 may be integrated in the control main board 2, the wire between the battery protection device 3 and the control main board 2 can be eliminated, and space utilization is improved.

After charging, the lithium ion battery 1 supplies power to the control main board 2 through the pins P- and P+ of the battery protection device 3. Any wire, which is connected between the lithium ion battery 1 and the control main board 2 for power supplying, can be eliminated. In this way, a circuit configuration of the portable fan 100 is simplified.

### Embodiment 12

As shown in FIGS. 105 and 106, schematic views of a first implementation are shown. The fan assembly 100 is a neck fan 3 and includes two air outlet members 1 that are disposed symmetrically to each other. Each of the two air outlet members 1 includes a housing 2, a fan 3 arranged inside the housing 2, a motor 6 arranged inside the housing 2, and a rotation shaft 4 arranged inside the housing 2. The housing 2 includes an annular body 21 protruding from the housing 2. The annular body 21 is hollow. The motor 6 drives the fan 3 to rotate, and the fan 3 rotates around the rotation shaft 4 to generate an airflow. The rotation shaft 4 is inserted to the annular body 21 from a first end 211 of the annular body 21 to secure the fan 3 to the annular body 21. A sealing member 5 is further arranged. The sealing member 5 is arranged at a second end 212 of the annular body 21 opposite to the first end 211 and is configured to seal the second end 212 of the annular body 21.

By arranging the sealing member 5, the second end 212 of the annular body 21 is sealed, such that a relatively sealed space is formed between the rotation shaft 4 and the annular body 21, preventing a lubricant of the rotation shaft 4 from spilling outwards from the second end 212, and preventing external dust and impurities from entering the annular body 21 through the second end 212. Therefore, the lubricant is prevented from being dried out, and the rotation shaft 4 is prevented from being excessively rubbed, ensuring that the rotation shaft 4 stably and smoothly rotate, and the service life of the air outlet members 1 is extended. The lubricant may be a lubricating fluid, a lubricating oil or any lubricating substance that can reduce friction of rotation of the rotation shaft.

In the present embodiment, the fan 3 is a centrifugal fan 3, the housing 2 defines an air inlet 22, a receiving cavity 23, an air duct 24 and an air outlet 25; and the air inlet 22, the receiving cavity 23, the air duct 24 and the air outlet 25 are sequentially connected to each other. The annular body 21 extends and protrudes from the housing 2 to the receiving cavity 23. The fan 3, the motor 6 and the rotation shaft 4 are received in the receiving cavity 23. The housing 2 defines two air inlets 22 respectively corresponding to two opposite air inlet negative pressure zones of the fan 3. The fan 3 rotates to drive the air at the air inlets 22 to flow through the air duct 24 to be output out of the housing through the air outlet 25.

When the rotation shaft 4 is inserted in the annular body 21, an oil storage portion 213 is formed between the rotation shaft 4 and the sealing member 5. The oil storage portion 213 is configured to store the lubricant. The lubricant carried by the rotation shaft 4 itself is inevitably partially consumed during rotation. By arranging the oil storage portion 213 for storing the lubricant, the lubricant can be supplied to the rotation shaft 4 in time, ensuring the rotation shaft 4 to rotate smoothly. At least part of the oil storage portion 213 is radially increased in a direction extending towards the second end 212, such that more lubricant can be stored, and the rotation shaft 4 is ensured to be in a lubricated state for a period of long time.

In the present embodiment, an end surface of the second end 212 is recessed relative to a surface of the housing 2 to form a recess portion 2121. The sealing member 5 is received in the recess portion 2121. A surface of the seal 5 is flush with the surface of the housing 2. The sealing member 5 is made of PE, which is a low cost material. A rear adhesive is formed on the sealing member 5 made of PE to firmly adhere the sealing member 5 to the recess portion 2121. Therefore, a simple assembling is achieved. A thickness of the sealing member 5 is 0.05mm-0.3mm, which is a thickness suitable for the portable fan assembly 100 to be used, wear and tear can be avoided, and the fan is thin and light and can be easily assembled. A process in the art which requires alignment snapping is eliminated, and an assembling efficiency is increased. Since the surface of the sealing member 5 is flush with the surface of the housing 2, an overall appearance is more aesthetic. Of course, in other embodiments, the surface of the sealing member 5 may be recessed or protruded with respect to the surface of the housing 2. Alternatively, the end surface of the second end 212 protrudes from the surface of the housing 2, and the sealing member 5 is arranged on the end surface of the second end 212. The thickness of the sealing member 5 is not limited to 0.05mm-0.3 mm, as long as the sealing member 5 seals the end surface of the second end 212. A manner in which the sealing member 5 is secured to the second end 212 is not limited to adhering, but any other securing means may be applied.

A bearing 7 is further arranged. The bearing 7 is disposed between the rotation shaft 4 and a radial direction of the annular body 21 to secure the rotation shaft 4 to the annular body 21 and to enable the rotation shaft 4 to rotate in the bearing 7. By arranging the bearing 7, the friction between the rotation shaft 4 and the annular body 21 is reduced, and the rotation shaft 4 is more securely fixed in the annular body 21. The lubricant is filled between the rotation shaft 4 and the bearing 7 to reduce the friction between the rotation shaft 4 the bearing 7, generated during the rotation shaft 4 rotating at high speeds, and vibration of the rotation shaft 4 is reduced. Therefore, the rotation shaft 4 rotates smoothly and steadily, with very low operating noise, and the bearings has low wear.

Two oil rings 8 are further arranged. The two oil rings 8 are respectively arranged on two axial sides of connection between the rotation shaft 4 and the bearing 7. The two oil rings 8 are configured to seal and maintain the lubricant between the rotation shaft 4 and the bearing 7 to ensure the rotation shaft 4 to stably and smoothly rotate.

A snap ring 9 is further arranged. The annular body 21 is hollow, and the second end 212 is communicated to the outside environment. The snap ring 9 may be received into the annular body 21 from the second end 212 and is snapped in a snap slot of the rotation shaft 4. The annular body 21 is arranged with a stopping portion 214 that is inwardly protruding. An outer diameter of the snap ring 9 is greater than an inner diameter of the stopping portion 214, such that the rotation shaft 4 is prevented from being detached off from the annular body 21. In addition, the snap ring 9 prevents the oil ring 8 from being detached off from the rotation shaft 4. The snap ring 9 is spaced apart, in the axial direction, from the stopping portion 214 to allow the lubricant to flow through. That is, the lubricant, which is stored in the oil storage portion 213, may flow through a spacing between the snap ring 9 and the stopping portion 214 in the axial direction to reach the rotation shaft 4, the bearing 7 and the interior of the annular body 21, such that the lubricant can be replenished in time. In addition, at least part of the oil storage portion 213 is radially increased to enable the snap ring 9 to be assembled from the second end 212 to the rotation shaft 4.

The fan 3 includes a hub 31 and fan blades 32 connected to the hub 31. The rotation shaft 4 is fixedly connected to the hub 31, a manner in which the rotation shaft 4 and the hub 31 are fixed to each other is not specifically limited herein. The rotation shaft 4 and the hub 31 may be fixed to each other by any one or more of: threads, screws, snaps and other fixing means. When the rotation shaft 4 is inserted in the annular body 21, a receiving portion 311 is formed between the hub 31 and the housing 2, the receiving portion 311 receives the motor 6.

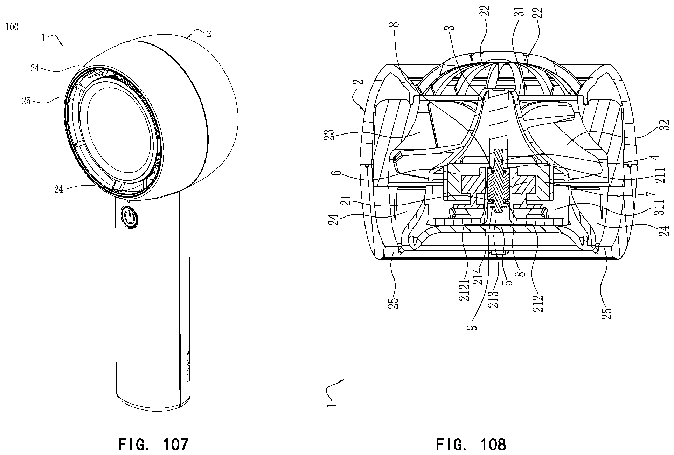

FIGS. 107 and 108 show schematic views of the fan of a second implementation. In the present implementation, the fan assembly 100 is a handheld fan 3, the fan assembly 100 includes one air outlet member 1. The fan 3 is a diagonal flow fan 3. The housing 2 defines one air inlet 22 corresponding to the fan 3. The fan 3 rotates to drive the air at the air inlet 22 to flow through the air duct 24 to be output out of the housing through the air outlet 25. The outer diameter of the snap ring 9 is not greater than the inner diameter of the stopping portion 214, and the snap ring 9 is configured only to prevent the oil ring 8 from being detached off from the rotation shaft 4.

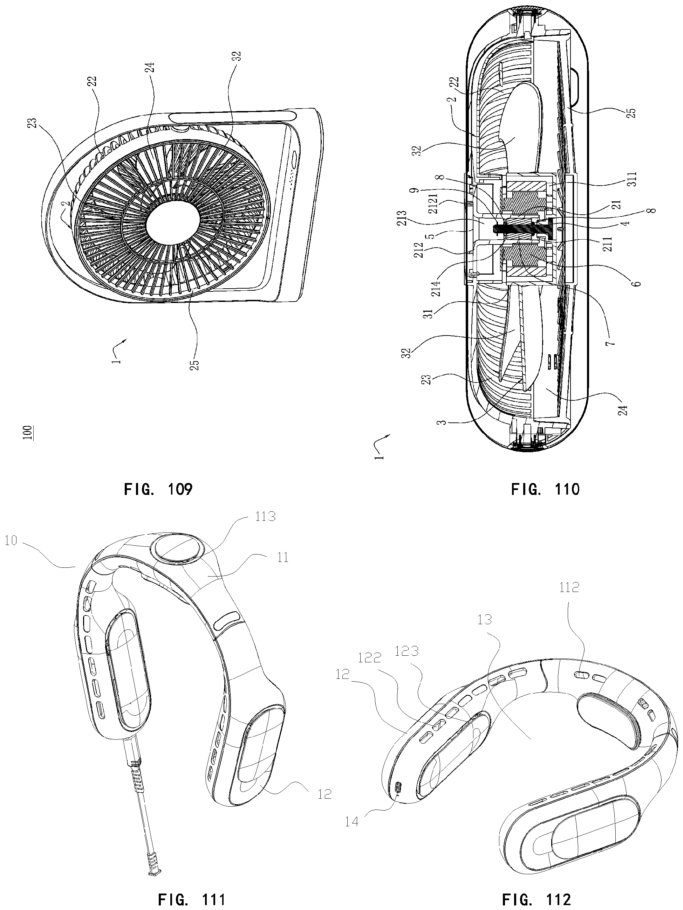

FIGS. 109 and 110 show schematic views of a third implementation. In the present implementation, the fan assembly 100 is a desktop fan 3, the fan assembly 100 includes one air outlet member 1. The fan 3 is an axial flow fan 3. The housing 2 defines one air inlet 22 corresponding to the fan 3. The fan 3 rotates to drive the air at the air inlet 22 to flow through the air duct 24 to be output out of the housing through the air outlet 25. The outer surface of the sealing member 5 is not flush with the surface of the housing 2, and the sealing member 5 is arranged with a protective cover (not labelled) to further improve fixation of the sealing member 5. The outer diameter of the snap ring 9 is not greater than the inner diameter of the stopping portion 214, and the snap ring 9 is configured only to prevent the oil ring 8 from being detached off from the rotation shaft 4.

In addition to the specific implementations illustrated above, it is understood that the present application is not limited to the above embodiments. Specifically, the fan assembly 100 may be any type of fan assembly 100, such as a neck fan 3, a handheld fan 3, a desktop fan 3, a stand fan 3, a ceiling fan, and so on. The number of air outlet members 1 may be one or two or more. The fan 3 may be any one of: a centrifugal fan 3, a diagonal flow fan 3, an axial flow fan 3, a cross flow fan 3, and so on. The sealing member 5 is required to seal the second end 212 of the annular body 21 to ensure that the rotation shaft 4 in the annular body 21 is provided with sufficient lubricate, such that the rotation shaft 4 can constantly rotate steadily and smoothly, the service life of the air outlet member 1 and the fan assembly 100 can be extended.

### Embodiment 13

As shown in FIGS. 111 to 120, in order to solve the above problem, the present disclosure provides a fan, including: a housing 10. The housing 10 includes a connection section 11 and two neck side sections 12 respectively connected to two sides of the connection section 11. The connection section 11 and the two neck side sections 12 cooperatively define a neck space 13. The connection section 11 and/or at least one of the two neck side sections 12 are arranged with an airflow portion 20. Each airflow portion 20 is configured to blow an airflow towards the neck space 13. In the present embodiment, since the two neck side sections are arranged respectively on the two sides of the connection section 11, the connection section 11 and the two neck side sections 12 cooperatively define the neck space 13, enabling the user to put on the fan through the neck space 13, and two hand of the user can be effectively freed. In addition, since the connection section 11 and/or the at least one of the two neck side sections 12 are arranged with the airflow portion 20, the fan of the present embodiment can blow the airflow towards the neck space 13, enabling the user to have a better airflow cooling experience.

For portable fans in the art, a single-phase motor is arranged for driving, an airflow strength is less, and a cooling effect is poor. Therefore, a better user experience cannot be provided.

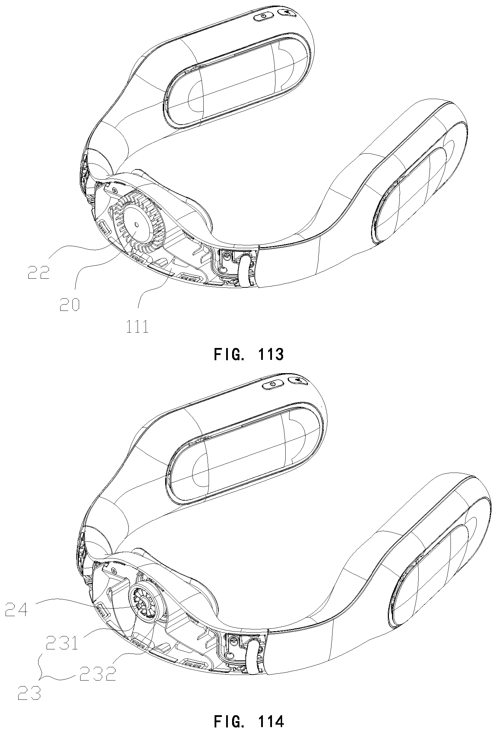

As shown in FIGS. 113 to 120, in order to solve the above problem, the airflow portion 20 of the present embodiment includes a positioning protruding post 21, rotation air blades 22 and a three-phase motor drive assembly 23 that is driveably connected to the rotation air blades 22. The three-phase motor drive assembly 23 includes a stator 231 and a rotor 232 sleeving an outside of the stator 231. The rotor 232 is fixedly mounted on the rotation air blades 22 and is coaxially arranged with the rotation air blades 22. The stator 231 fixedly sleeves the positioning protruding post 21. By arranging the three-phase motor drive assembly 23 to drive the rotation air blades 22 in the present embodiment, the airflow portion 20 is enabled to output a stronger airflow to the neck space 13, such that the user may have an improved cooling effect.

As shown in FIGS. 113 to 115, in an embodiment, the airflow portion 20 is arranged on the connection section 11. The connection section 11 defines a first air guiding cavity 111 therein. The connection section 11 defines a first air outlet 112 communicated with the first air guiding cavity 111. The first air outlet 112 faces towards the neck space 13. The positioning protruding post 21 is received in the first air guiding cavity 111. The rotation air blades 22 are rotatably received in the first air guiding cavity 111 and configured to blow the airflow to the first air outlet 112.

In an embodiment, the rotation air blades are blades of a radial flow fan.

As shown in FIGS. 111 to 115, in an embodiment, in order to enable the fan of the present embodiment to effectively blow the air into the neck space 13, the connection section 11 defines a first air inlet 113 communicated to the first air guiding cavity 111, and the first air inlet 113 faces towards an end of the rotation air blades 22. The first air outlet 112 faces towards a side of the rotation air blades 22. By defining the first air inlet 113 facing towards the end of the rotation air blades 22 and defining the first air outlet 112 facing towards the side of the rotation air blades 22, the fan can efficiently drive the external ambient air to flow towards the neck space 13, such that the user can obtain a better cooling effect.

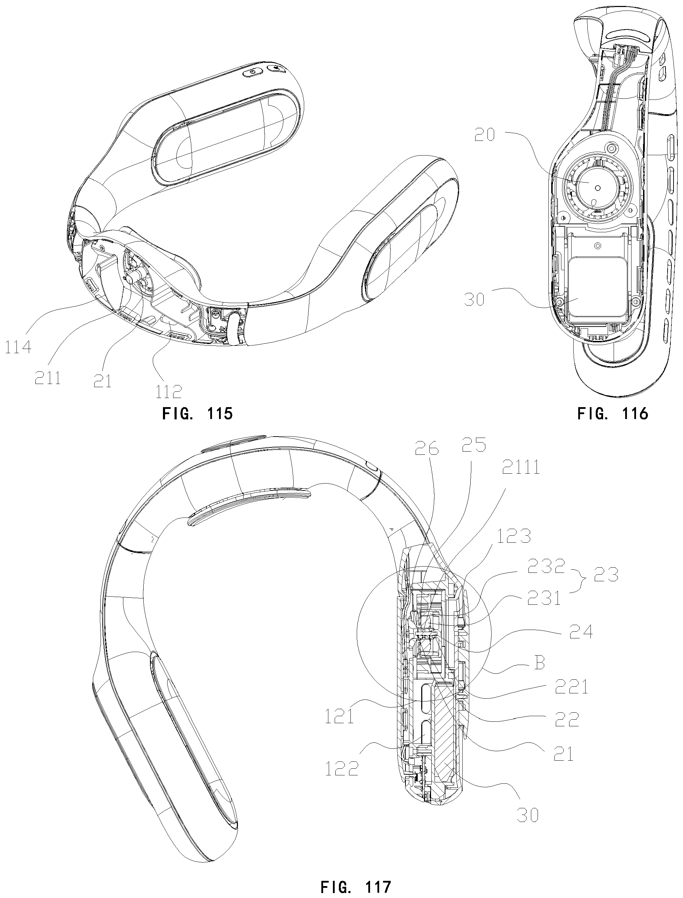

As shown in FIGS. 117 to 120, in an embodiment, the rotation air blades 22 defines a fixation hole 221, and an axis of the fixation hole 221 coincides with an axis of the rotation air blades 22. The airflow portion 20 further includes a rotation shaft 24. A first end of the rotation shaft 24 is fixedly threaded into the fixation hole 221. A positioning protruding post 21 defines a positioning hole 211. An axis of the positioning hole 211 coincides with the axis of the rotation shaft 24. A second end of the rotation shaft 24 is rotatably received in the positioning hole 211.

As shown in FIGS. 113 to 115, in order to improve the cooling effect of the fan provided in the present embodiment, a plurality of first air guiding ribs 114 are arranged in the connection section 11, and the plurality of first air guiding ribs 114 cooperatively define the first air guiding cavity 111. A plurality of first air outlets 112 are defined. The plurality of first air outlets 112 are spaced apart from each other and are located along an extension direction of the connection section 11. An end of the first air guiding cavity 111 is communicated with the plurality of first air outlets 112. The plurality of first air outlets 112 are defined, the plurality of first air outlets 112 are spaced apart from each other and are located along the extension direction of the connection section 11, and the end of the first air guiding cavity 111 is communicated with the plurality of first air outlets 112. Therefore, the air in the air guiding cavity can be uniformly output, by the airflow portion 20, from the plurality of first air outlets 112 to the neck space 13, such that the cooling effect of the fan is improved.

As shown in FIGS. 117 to 120, since the rotation air blades 22 of the present embodiment are driven by the three-phase motor assembly having a higher rotational speed, in order to avoid sharp wear between the rotation shaft 24 and the positioning hole 211 due to the high-speed rotation and to ensure the service life of the fan, at least one airflow portion 20 includes a bearing portion 25. An outer ring of the bearing portion 25 is fixed in the positioning hole 211, and an inner ring of the bearing portion 25 sleeves the second end of the rotation shaft 24. Due to the outer ring of the bearing portion 25 being fixed in the positioning hole 211 and the inner ring of the bearing portion 25 sleeving the second end of the rotation shaft 24, the friction between the positioning hole 211 and the rotation shaft 24 can be converted from sliding friction to friction inside the bearing portion 25. In this way, wear and tear between the positioning hole 211 and the rotation shaft 24 can be effectively avoided, the service life of the airflow portion 20 of the present embodiment is extended.

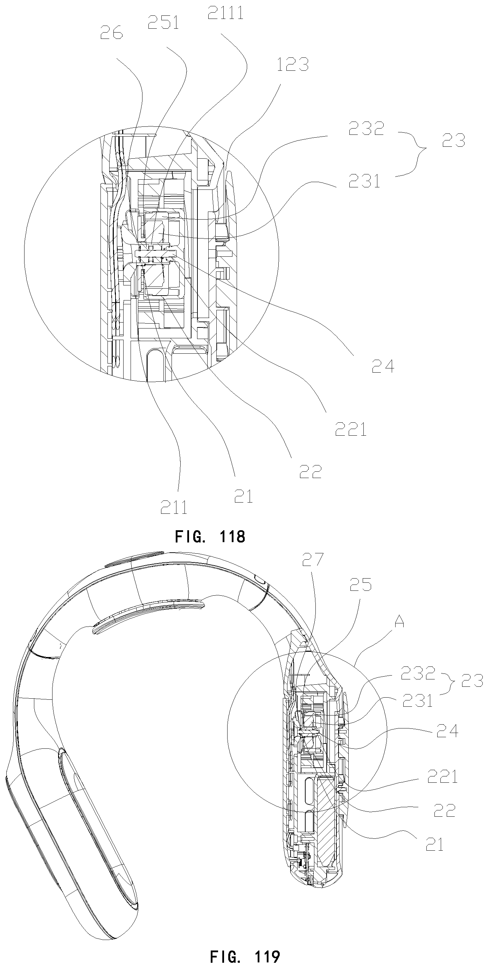

As shown in FIGS. 117 and 118, in an embodiment, the bearing portion 25 includes a rolling bearing 251. The airflow portion 20 further includes a limiting member 26. The limiting member 26 is mounted on the second end of the rotation shaft 24. The bearing portion 25 is disposed between the limiting member 26 and the first end of the rotation shaft 24. A plurality of bearing portions 25 are arranged. An inner flange 2111 is arranged on an inner wall of the positioning hole 211, and the inner flange 2111 is disposed between two adjacent bearing portions 25, such that the two adjacent bearing portions 25 are spaced apart from each other. By arranging the limiting member 26 on the second end of the rotation shaft 24 and arranging the inner flange 2111 on an inner wall of the positioning hole 211, the inner ring and the outer ring of the rolling bearing 251 of the present embodiment can be effectively limited, such that the rolling bearing 251 can be effectively mounted.

In an embodiment, the airflow portion 20 of the present embodiment further includes an elastic member. The elastic member of the present embodiment is disposed between the rotation air blades 22 and the rolling bearing 251 near the rotation air blades 22 and sleeves the rotation shaft 24. Two ends of the elastic member of the present embodiment respectively abut against the inner ring of the rolling bearing 251 and the rotation air blades 22. In this way, an elastic force, in a direction away from the air outlet, is applied on the inner ring of the rolling bearing 251. Due to the elastic member of the present embodiment, the rolling bearing 251 can be preloaded, such that the service life of the rolling bearing 251 is extended.

In an embodiment, the bearing portion 25 of the present embodiment is a ball bearing, and the lubricating oil is arranged in the ball bearing.

In an embodiment, the bearing portion 25 of the present embodiment is a ceramic bearing, and the lubricating oil is arranged in the ceramic bearing.

In an embodiment, the bearing portion 25 of the present embodiment is a magnetic levitation bearing.

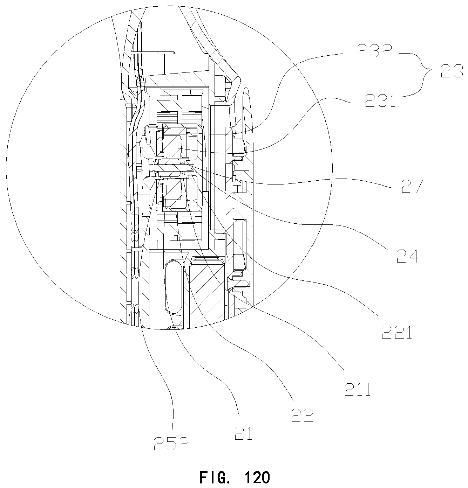

As shown in FIGS. 119 and 120, in another embodiment, the bearing portion 25 includes a slide bearing 252, and the airflow portion 20 further includes two sealing rings 27. The two sealing rings 27 both sleeve the rotation shaft 24 and are respectively located on two sides of the slide bearing 252. In the present embodiment, the slide bearing 252 between the rotation shaft 24 and the positioning hole 211, the inner ring of the slide bearing 252 is fixedly connected to the second end of the rotation shaft 24, and the outer ring is fixedly connected to the fixation hole 221. Therefore, the friction between the rotation shaft 24 and the positioning hole 211 can be converted into the friction between the inner ring and the outer ring of the slide bearing 252, wear and tear between the rotation shaft 24 and the positioning hole 211 is avoided, and the service life of the fan of the present embodiment is extended.

In an embodiment, the lubricate oil is arranged in the slide bearing 252. The sealing rings 27, which sleeve the rotation shaft 24 and are located on the two sides of the slide bearing 252 respectively, are configured to seal the lubricate oil.

As shown in FIGS. 116 to 120, in an embodiment, each of the two neck side sections 12 is arranged with one respective airflow portion 20. A second air guiding cavity 121 is defined in each neck side section 12. The neck side section 12 defines a second air outlet 122 communicated with the second air guiding cavity 121. The second air outlet 122 faces towards the neck space 13. The positioning protruding post 21 is received in the second air guiding cavity 121. The rotation air blades 22 are rotatably mounted in the second air guiding cavity 121 and are configured to blow the airflow to the second air outlet 122.

As shown in FIGS. 117 to 120, in an embodiment, the neck side section 12 defines a second air inlet 123 communicated to the second air guiding cavity 121. The second air inlet 123 faces an end of the rotation air blades 22, and the second air outlet 122 faces a side of the rotation air blades 22.

In an embodiment, a plurality of second air outlets 122 are defined. The plurality of first air outlets 122 are spaced apart from each other and are located along an extension direction of the neck side section 12.

In an embodiment, the airflow portion 20 is arranged in one of the two neck side sections 12, and the airflow portion 20 is arranged with the bearing portion 25.

In an embodiment, the airflow portion 20 is arranged in one of the connection section 11 and the two neck side sections 12, and the airflow portion 20 is arranged with the bearing portion 25.

In an embodiment, each of the connection section 11 and the two neck side sections 12 is arranged with one airflow portion 20 respectively, and the airflow portion 20 is arranged with the bearing portion 25.

As shown in FIG. 112 and FIG. 116, in order to enable the fan of the present embodiment to operate without being connected to any external power source, the fan further includes a power supply portion 30, arranged inside the housing 10. The power supply portion 30 is electrically connected to each airflow portion 20. The housing 10 is arranged with a charging port 14 that is electrically connected to the power supply portion 30. By arranging the power supply portion 30 in the housing 10 and enabling the power supply portion 30 to be electrically connected to each airflow portion 20, the fan of the present embodiment can blow the airflow without being connected to any external power source, such that application versatility of the fan of the present embodiment is improved. In addition, by arranging the charging port 14 in the housing 10 to be electrically connected to the power supply portion 30, the power supply portion 30 may be connected to the external power source through the charging port 14, such that the power supply section 30 can be charged.

In an embodiment, the power supply portion 30 of the present embodiment is a storage battery.

In an embodiment, at least one of the first air inlet and the first air outlet is arranged with a filter mesh. By arranging the filter mesh on at least one of the first air inlet and the first air outlet, debris in the external environment can be effectively avoided from entering the fan.

In an embodiment, at least one of the second air inlet and the second air outlet is arranged with a filter mesh. By arranging the filter mesh on at least one of the second air inlet and the second air outlet, debris in the external environment can be effectively avoided from entering the fan.

In an embodiment, the fan of the present embodiment further includes a gel receiving portion. The gel receiving portion is detachably arranged in the first air guiding cavity and/or the second air guiding cavity. The gel receiving portion of the present embodiment is configured to hold a gel-type aroma agent. By arranging the gel-type aroma agent inside the gel receiving portion, the airflow blown by the fan may have an aroma odour, and a better user experience is provided.

In an embodiment, the gel receiving portion of the present embodiment includes a box body and a cover plate covering on the box body. The box body is detachably mounted in a first air guiding cavity. The box body defines a receiving chamber to receive the gel-type aroma agent. The cover plate assembly includes a first cover plate and a second cover plate. The first cover plate is detachably mounted on the box body, and the second cover plate is pivoted to the first cover plate. The first cover plate has a first opening, and the second cover plate has a second opening. The second cover plate has a covering state in which the second cover plate covers the entire first opening and has an open state in which the first opening and the second opening coincide with each other. By rotating the second cover plate, the second cover plate can be switched between the covering state and the open state.

In summary, the fan of the present embodiment has at least the following beneficial technical effect. Two neck side sections are respectively arranged on the two sides of the connection section 11, such that the connection section 11 and the two neck side sections 12 cooperatively define the neck space 13, enabling the user to put on the fan through the neck space 13, and the hands of the user are freed. **In** addition, the airflow portion 20 is arranged in the connection section 11 and/or the at least one neck side section 12, such that airflow can be blown towards the neck space 13, a better cooling effect is provided.

The above detailed description is only an illustration of preferred embodiments of the present disclosure. The present disclosure is not limited thereto. Any equivalent technical changes made based on the contents of the specification and the drawings of the present invention are included in the scope of the present disclosure.

## Claims

1. A portable fan, comprising:
a housing, defining an air inlet, a receiving cavity, an air duct, and an air outlet, wherein the air inlet, the receiving cavity, the air duct, and the air outlet are sequentially communicated with each other; and the housing comprises a first side wall defining the air outlet;
a fan assembly, received in the receiving cavity and configured to blow an airflow at the air inlet to flow through the air duct to reach the air outlet;
a negative-ion module, arranged in the housing, wherein the negative-ion module comprises a negative-ion generator, a positive-electrode release portion, and a negative-electrode release portion; when the negative-ion generator is operating, the positive-electrode release portion and the negative-electrode release portion are configured to discharge to a space between the positive-electrode release portion and the negative-electrode release portion to ionize air in the space to produce negative ions, and the negative ions are capable of being discharged out of the housing.

2. The portable fan according to claim 1, further comprising a partition assembly, wherein the partition assembly comprises a longitudinal plate, a first cross plate, and a second cross plate; the longitudinal plate, the first cross plate, the second cross plate, and an inner wall of the housing cooperatively define the receiving cavity and the air duct; and
a first space is defined between the first cross plate and the inner wall of the housing, the negative-ion module is received in the first space; the housing has a recess exposed outwardly at a position corresponding to the first space; a first release port and a second release port, which communicate the first space to an outside environment, are defined in the recess; the negative-electrode release portion is arranged at the first release port, and the positive-electrode release portion is received in the second release port; or
a first space is defined between the first cross plate and the inner wall of the housing, the negative-ion module is received in the first space; the negative-electrode release portion and the positive-electrode release portion are both received in the first space, the housing defines a first release port that communicates the first space with an outside environment; and the negative-electrode release portion is arranged at the first release port; or
the first cross plate defines a first through hole communicating the air duct with the first space; a portion of the airflow generated by the fan assembly is blown to flow through the first through hole towards the first space; or the first cross plate does not define the first through hole communicating the air duct with the first space; or
a first space is defined between the first cross plate and the inner wall of the housing, the negative-ion module is received in the first space; the first cross plate defines a first through hole that communicates the air duct with the first space, the negative-electrode release portion is disposed corresponding to the first through hole; a second space is defined between the second cross plate and the housing, the positive-electrode release portion is received in the second space, the second cross plate defines a second through hole that communicates the air duct with the second space, and the positive-electrode release portion is disposed corresponding to the second through hole; or
the first through hole and the second through hole are located staggered to each other in a front-to-rear direction, and the positive-electrode release portion and the negative-electrode release portion are disposed staggered to each other in a front-to-rear direction; or
the housing defines a first release port that communicates the first space with an outside environment, the negative-electrode release portion is located close to the first release port, a part of the airflow generated by the fan assembly is capable of flowing through the first through hole to enter the first space to blow a part of the negative ions out of the housing through the air outlet; or
the housing comprises a first portion, a second portion, and a third portion pivotally connected to the first portion and the second portion; the first portion and the second portion are disposed symmetrically to each other; each of the first portion, the second portion, and the third portion defines the air inlet, the receiving cavity, the air duct, and the air outlet; and the air inlet, the receiving cavity, the air duct, and the air outlet are sequentially located and are communicated with each other; the fan assembly is received in each respective receiving cavity; or
the receiving cavity of the first portion is located at a free end of the first portion, the receiving cavity of the second portion is located at a free end of the second portion, the receiving cavity of the third portion is located at a middle portion of the third portion; each of two sides of the receiving cavity of the third portion is arranged with one respective air duct; and each of the first portion and the second portion is arranged with one respective negative-ion module.

3. The portable fan according to claim 1, further comprising a partition assembly, wherein the partition assembly comprises a longitudinal plate, a first cross plate, and a second cross plate; the longitudinal plate, the first cross plate, the second cross plate, and an inner wall of the housing cooperatively define the receiving cavity and the air duct; and
the first cross plate comprises a shielding plate that extends longitudinally and abuts against the second cross plate; the shielding plate is located between the receiving cavity and the air duct; an end of the shielding plate abuts against the first side wall, an air vent is defined between the other end of the shielding plate and the longitudinal plate; the airflow generated by the fan assembly is capable of flowing through the air vent to enter the air duct; the shielding plate has a projecting area onto the first side wall, the air outlet is defined in the projecting area; and
the first cross plate comprises a first flat plate portion received in the receiving cavity, a first inclined surface received in the air duct, a second inclined surface received in the air duct, and a second flat plate portion received in the air duct; the second inclined surface is connected to both the first inclined surface and the second flat plate portion; the second inclined surface is disposed adjacent to the air outlet; the shielding plate is disposed between the first flat plate portion and the second inclined surface; or
the shielding plate comprises a curved portion located at a free end thereof; an end of the curved portion is disposed on the second inclined surface; an inner diameter of the curved portion is in a range of 0.25mm to 1.25 mm; or
the housing comprises a second side wall facing towards a neck of a user and a third side wall facing away from the neck of the user; each of the second side wall and the third side wall defines the respective air inlet corresponding to the fan assembly; a negative-ion cavity is defined between the first cross plate and the second side wall; the first side wall defines a release port communicated with the negative-ion cavity; the negative-ion generator is arranged inside the housing; a release end of the negative-ion generator directly faces the release port; the release port is disposed adjacent to and side-by-side with the air outlet; or
the first cross plate defines a through hole communicated with the negative-ion cavity, a part of the airflow generated by the fan assembly is capable of flowing through the through hole to reach a release end of the negative-ion generator and flowing out of the housing through the release port; or
a light strip is embedded in the third side wall, the light strip and the third side wall extend along a same direction; the air inlet defined in the third side wall is disposed on two sides of the light strip; the second cross plate is arranged with a filter member that faces towards the air inlet defined in the third side wall; the filter member has a plurality of filter holes; or
the housing comprises a first portion, a second portion, and a third portion pivotally connected to the first portion and the second portion; the first portion and the second portion are disposed symmetrically to each other; each of the first portion, the second portion, and the third portion defines the air inlet, the receiving cavity, the air duct, and the air outlet; and the air inlet, the receiving cavity, the air duct, and the air outlet are sequentially located and are communicated with each other; the fan assembly is received in each respective receiving cavity; or
the receiving cavity of the first portion is located at a free end of the first portion, the receiving cavity of the second portion is located at a free end of the second portion, the receiving cavity of the third portion is located at a middle portion of the third portion; each of two sides of the receiving cavity of the third portion is arranged with one respective air duct; or
the housing comprises a second side wall facing towards a neck of a user; the third portion is further arranged with a support portion facing towards a rear of the neck; the support portion defines a first notch; a center of the second side wall of the third portion is arranged with a logo portion that is exposed from the first notch; the support portion further defines a second notch extending parallel to the air outlet; or
the logo portion is surrounded by the air inlet, and a part of the air inlet is exposed from the first notch.

4. The portable fan according to claim 1, wherein the housing comprises a first side wall facing towards a neck of a user and a second side wall facing away from the neck; the second side wall comprises a main wall and an auxiliary wall; the main wall and the auxiliary wall are arranged in a stepped manner; the second air inlet is formed between the main wall and the auxiliary wall; and
the air inlet comprises a first air inlet defined in the first side wall and a second air inlet defined in the second side wall, the second air inlet is formed between the main wall and the auxiliary wall; or
the housing comprises two clamp arms that are disposed symmetrically to each other, the two the clamp arms are rotatably connected to each other by a rotation connection member; or
each of the two clamp arms defines the air inlet, the receiving cavity, the air duct, and the air outlet; and the air inlet, the receiving cavity, the air duct, and the air outlet are sequentially communicated to each other, each receiving cavity receives respective one fan assembly; or
each of the two clamp arms defines the first air inlet defined in the first side wall and the second air inlet defined in the second side wall; or
a pivot angle of each clamp arm is in a range of ±10°; or
the main wall is longer than the auxiliary wall; and when the user is wearing the portable fan, the main wall is located above the auxiliary wall; or
a cover plate having a through hole is disposed between the second air inlet and the fan assembly; or
a cover plate having a through hole is disposed between the second air inlet and the fan assembly; or
a partition assembly is further arranged to separate the receiving cavity and the air duct, which are communicated with each other, from the remaining space inside the housing; a width of an end of the air duct away from the receiving cavity is less than a width of an end of the air duct near the receiving cavity, and a space is formed to receive a battery.

5. The portable fan according to claim 1, wherein,
the housing comprises a rear neck section located at a middle of the housing, the receiving cavity is defined in the rear neck section, the rear neck section is arranged with a mounting portion at a position corresponding to the receiving cavity and facing towards a rear neck;
a support member is mounted on the mounting portion and is configured to support the rear neck;
a cushion member is arranged inside the mounting portion and abuts against the support member, and the cushion member is elastically deformable; and
the support member comprises a contact portion and a connection member, the connection member comprises a first mating portion mating with the contact portion and two second mating portions mating with the mounting portion; and when the two second mating portions are pressed, the two second mating portions are moved close to each other to extend into or move out of the mounting portion; when pressing on the two second mating portions is released, the two second mating portions are secured to the mounting portion or moved out of the mounting portion; or
the mounting portion comprises a mounting cavity, each of the two second mating portions comprises a hook portion that enters the mounting cavity and hooks with the mounting cavity, the cushion member abuts against the hook portion, a height of the hook portion and the cushion member is greater than or equal to a height of the mounting cavity; or
a spacing is defined between the mounting portion and a surface of the first mating portion facing towards the mounting portion, the cushion member is made of silicone, and a size of the cushion member is capable of being compressed to be 15% to 25% of its original size, the support member is made of silicone; or
the housing comprises a first side wall facing towards the neck of the user and a second side wall facing away from the neck of the user, the first side wall is arranged with the mounting portion; or
the housing comprises two clamp arms that are disposed symmetrically to each other; the two the clamp arms are rotatably connected to each other by a rotation connection member; a pivot angle of each of the two clamp arms is in a range of ±10°; or
the portable fan is arranged with two support members corresponding to the two clamp arms; when the two clamp arms are pivoted, the two support members are configured to contact the neck of the user; or
each of the two clamp arms defines the air inlet, the receiving cavity, the air duct, and the air outlet; and the air inlet, the receiving cavity, the air duct, and the air outlet are sequentially arranged and are communicated to each other; corresponding to each of fan assembly, each clamp arm defines a first air inlet defined in the first side wall and a second air inlet defined in the second side wall; a spacing is defined between at least a portion of the support member and the first air inlet; or
the support member comprises a contact portion and a connection member, the contact portion defines a through hole at position corresponding to the first air inlet; the second side wall comprises a main wall and an auxiliary wall; the main wall and the auxiliary wall are arranged in a stepped manner; the second air inlet is formed between the main wall and the auxiliary wall; or
a partition assembly is further arranged, the partition assembly separates the receiving cavity and the air duct, which are communicated with each other, from the remaining space inside the housing, a width of an end of the air duct away from the receiving cavity is less than a width of an end of the air duct near the receiving cavity, and a space is reserved to receive a battery.

6. The portable fan according to claim 1, further comprising a fan drive circuit; wherein the fan drive circuit:
a master control circuit;
a three-phase drive circuit, comprising at least three signal input ends and three drive signal output ends; wherein each of the at least three signal input ends is electrically connected to the master control circuit to receive control signals; each of the three drive signal output ends is electrically connect to a respective one of three signal ends of a direct-current (DC) brushless fan motor to respectively output a three-phase drive signal to drive the DC brushless fan motor to rotate; and
an inverted-phase electric potential detection circuit, comprising three detection branches; wherein each of the three detection branches comprises a detection end and a detection output end electrically connected to the detection end; three detection ends of the three detection branches are respectively electrically connected to the three drive signal output ends; three detection output ends of the three detection branches are electrically connected to the master control circuit to respectively output a first detection signal, a second detection signal, and a third detection signal; the master control circuit is informed of a phase of the three-phase drive signal based on the first detection signal, the second detection signal, and the third detection signal to adjust the control signals;
wherein the detection branch comprises a first detection resistor, a second detection resistor, and a third detection resistor; the first detection resistor and the second detection resistor are connected to each other in series; an end of the first detection resistor away from the second detection resistor is the detection end, and an end of the second detection resistor away from the first detection resistor is grounded; a node between the first detection resistor and the second detection resistor is the detection output end; or
the three-phase drive circuit comprises a first transistor, a second transistor, a third transistor, a fourth transistor, a fifth transistor, a sixth transistor, a seventh transistor, an eighth transistor, and a ninth transistor; a first conductive end of the first transistor, a first conductive end of the second transistor, and a first conductive end of the third transistor are connected to a power supply end; a first conductive end of the fourth transistor is connected to the power supply end; a first conductive end of the fifth transistor is connected to the power supply end; a first conduction end of the sixth transistor is connected to the power supply end; a control end of the fourth transistor, a control end of the fifth transistor, and a control end of the sixth transistor are electrically connected to the master control circuit, a control end of the seventh transistor is electrically connected to the control end of the fourth transistor, a control end of the eighth transistor is electrically connected to the control end of the fifth transistor, and a control end of the ninth transistor is electrically connected to the control end of the sixth transistor, so as to receive the control signals; second conduction ends of the fourth transistor, the fifth transistor, and the sixth transistor are grounded; a first conductive end of the seventh transistor is connected to a second conductive end of the first transistor; a second conductive end of the seventh transistor is grounded; a first conductive end of the eighth transistor is connected to a second conductive end of the second transistor; a second conductive end of the eighth transistor is grounded; a first conductive end of the ninth transistor is connected to the second conductive end of the third transistor; a second conductive end of the ninth transistor is grounded; a node between the first conductive of the seventh transistor and the second conductive of the first transistor, a node between the first conductive of the eighth transistor and the second conductive of the second transistor, and a node between the first conductive of the ninth transistor and the second conductive of the third transistor respectively serve as the three drive signal output ends; the at least three signal input ends are three PWM signal input ends; the control signals comprise three PWM signals; and the fan drive circuit further comprises a current detection circuit; the second conductive ends of the seventh transistor, the eighth transistor, and the ninth transistor are all grounded via the current detection circuit; the current detection circuit is electrically connected to the master control circuit; the current detection circuit comprises a sense resistor and a sense capacitor; the second conductive ends of the seventh transistor, the eighth transistor, and the ninth transistor are grounded via the sense resistor and the sense capacitor sequentially; a node between the sense resistor and the sense capacitor is electrically connected to the master control circuit; or
the three-phase drive circuit comprises a first transistor, a second transistor, a third transistor, a fourth transistor, a fifth transistor, and a sixth transistor; a first conductive end of the first transistor, a first conductive end of the second transistor, and a first conductive end of the third transistor are connected to a power supply end; a first conductive end of the fourth transistor is connected to a second conductive end of the first transistor, a first conductive end of the fifth transistor is connected to a second conductive end of the second transistor, a first conduction end of the sixth transistor is connected to a second conductive end of the third transistor; a node between the first conductive of the fourth transistor and the second conductive of the first transistor, a node between the first conductive of the fifth transistor and the second conductive of the second transistor, and a node between the first conductive of the sixth transistor and the second conductive of the third transistor respectively serve as the three drive signal output ends; control ends of the first transistor, the second transistor, the third transistor, the fourth transistor, the fifth transistor, and the sixth transistor are electrically connected to the master control circuit to receive the control signals; and the control signals comprise six PWM signals; or
and the fan drive circuit further comprises a current detection circuit, the second conductive end of the sixth transistor is grounded via the current detection circuit, the current detection circuit is electrically connected to the master control circuit; the current detection circuit comprises a sense resistor and a sense capacitor; the second conductive end of the sixth transistor is grounded via the sense resistor; the sense capacitor is connected in parallel with the sense resistor; a node between the sense resistor and the second conduction end of the sixth transistor is electrically connected to the master control circuit; the current detection circuit further comprises a first series resistor, a second series resistor, a parallel resistor; the parallel resistor is connected in parallel with the sense resistor; the first series resistance is connected between an end of the sense capacitor and an end of the sense resistor; the second series resistance is connected between the other end of the sense capacitor and the other end of the sense resistor; or
the master control circuit comprises a master control chip and three three-phase control chips, each of the three three-phase control chips is electrically connected to the master control chip and the three-phase drive circuit; the fan drive circuit further comprises a voltage conversion circuit; the voltage conversion circuit is configured to receive a battery voltage, convert the battery voltage into a drive voltage, and provide the drive voltage to power supply ends of the three three-phase control chips; the master control chip is configured to output a master control signal to the three three-phase control chips to enable each of the three three-phase control chips to output the respective control signal to the three-phase drive circuit; the fan drive circuit further comprises a switch control circuit; the switch control circuit is electrically connected to the battery, the voltage conversion circuit, and the master control circuit to control operation of the voltage conversion circuit; the switch control circuit comprises a keypad, a first switch transistor, a second switch transistor, and a third switch transistor; two conductive ends of the first switch transistor are respectively connected to the positive electrode of the battery and the input end of the voltage conversion circuit; a control end of the first switch transistor is grounded via two conductive ends of the third switch transistor; a positive electrode of the battery is connected to the control end of the third switch transistor via the two conductive ends of the first switch transistor and a one-way diode; a control end of the second switch transistor is grounded via the keypad; a control end of the third switch transistor is electrically connected to the master control circuit; a node between the second switch transistor and the one-way diode is further electrically connected to the master control circuit; the fan drive circuit further comprises a direct current (DC) conversion circuit, the DC conversion circuit is configured to receive the drive voltage and convert the drive voltage to a DC operating voltage; or
the fan drive circuit further comprises a battery voltage detection circuit, the battery voltage detection circuit comprises a second voltage divider resistor and a third voltage divider resistor that is connected in series to the second voltage divider resistor; a node between the second voltage divider resistor and the third voltage divider resistor is electrically connected to the master control circuit; or the battery voltage detection circuit comprises a filter capacitor and a sampling resistor connected in series to the filter capacitor; the sampling resistor is connected between the filter capacitor and the ground; a node between the filter capacitor and the sampling resistor is electrically connected to the master control circuit; the fan drive circuit further comprises a signal amplification circuit; the node between the filter capacitor and the sampling resistor is connected to the master control circuit via the signal amplification circuit; the signal amplification circuit is configured to amplify a signal sampled by the sampling resistor and provide the amplified signal to the master control circuit; or
the fan drive circuit further comprises a Hall detection circuit; the Hall detection circuit is electrically connected to the master control circuit to detect a magnetic field generated by the DC brushless fan motor and to output a Hall detection signal to the master control circuit; the master control circuit is informed, based on the Hall detection signal, of a position of a rotor of the DC brushless fan motor; the Hall detection circuit further comprises a motor temperature detection element connected between a Hall element of the Hall detection circuit and the master control circuit; the fan drive circuit further comprises a transistor temperature detection circuit; the transistor temperature detection circuit comprises a voltage regulated capacitor connected in parallel with the thermistor; the thermistor is configured to sense a temperature of each transistor of the three-phase drive circuit; a node between the first voltage divider resistor and the thermistor is electrically connected to the master control circuit and is configured to output a temperature signal, enabling the master control circuit to control, based on the temperature signal, the fan drive circuit to enter or not enter a temperature protection state; the thermistor is connected between the first voltage divider resistor and the ground; the transistor temperature detection circuit further comprises a voltage regulated capacitor connected in parallel with the thermistor; or
the master control circuit comprises a master control chip and an auxiliary chip; two three-phase drive circuits, two inverted-phase electric potential detection circuits, and two DC brushless fan motors are arranged in one-to-one correspondence to each other; the master control circuit is electrically connected to one of the two three-phase drive circuits to output the control signals to one of the two three-phase drive circuits to drive the respective one of the two brushless DC fan motors; the inverted-phase electric potential detection circuit is electrically connected to the respective one three-phase drive circuit and outputs the first detection signal, the second detection signal, and the third detection signal to the master control chip to enable the master control chip to be informed of the phase of the three-phase drive signal of the respective one three-phase drive circuit to adjust the control signals output to the respective one three-phase drive circuit; the auxiliary chip is electrically connected to the other one of the two three-phase drive circuits to output the control signal to the other one of the two three-phase drive circuits to drive the other one of the two DC brushless fan motors; the other one of the two inverted-phase electric potential detection circuits is electrically connected to the respective three-phase drive circuit and outputs a corresponding first detection signal, a corresponding second detection signal and a corresponding third detection signal to the auxiliary chip to enable the auxiliary chip to be informed of the phase of the three-phase drive signal of the other one of the two three-phase drive circuits to adjust the control signals output to the other one of the two three-phase drive circuits.

7. The portable fan according to claim 1, further comprising: a shaft tube arranged inside the housing, a rotation shaft received in a shaft hole of the shaft tube and rotatable with respect to the shaft tube, a stator sleeving an outside of the shaft tube, and an impeller connected to a top of the rotation shaft; wherein the housing encloses to define a mounting channel having an opening at an end, the impeller is capable of being assembled in and disassembled from the mounting channel; a disassembling member 4 arranged on the housing; the disassembling member is detachably connected with the housing; an anti-hitting member is arranged on a bottom surface of the disassembling member facing towards the impeller to prevent the impeller from hitting the disassembling member; and
an anti-hitting recess is formed in a bottom surface of a center of the disassembling member facing towards the impeller; the anti-hitting member is mounted at the anti-hitting recess; or
the anti-hitting member is made of EVA foam or soft silicone; or
the anti-hitting member is cylindrical; or
one of the disassembling member and the housing is arranged with a rotation fastener and/or outer threads and/or a first magnet, and the other one of the disassembling member and the housing is arranged with a rotation fastener groove and/or inner threads and/or a second magnet; or
one of the disassembling member and the housing is arranged with a rotation fastener, and the other one of the disassembling member and the housing is arranged with a rotation fastener groove; the rotation fastener groove has a first position and a second position, and the rotation fastener is configured to rotate to switch between the position and the second position;
the disassembling member and the housing are configured to rotate to switch between a first mating position and a second mating position; when the disassembling member and the housing are at the first mating position, the rotation fastener is inserted into the first position of the rotation fastener groove; when the disassembling member and the housing are at the second mating position, the rotation fastener is inserted into the second position of the rotation fastener groove, so as to limit relative displacement of the disassembling member and the housing; or
the disassembling member is arranged with a rotation fastener, and the housing is arranged with a rotation fastener groove;
the disassembling member defines a first recess and a second recess along a radial direction thereof; the first recess and the second recess are communicated to each other; the first position of the rotation fastener groove is corresponding to a position of the first recess; the second position of the rotation fastener groove is corresponding to a position of the second recess; a first resilient fastener is arranged on the disassembling member and protruding from the disassembling member into the second recess;
the housing defines a third recess along the radial direction thereof; a second resilient fastener is arranged at a side of the third recess near the disassembling member; the second resilient fastener is connected with the housing; when the second resilient fastener slides from the first recess to the second recess, an axial relative displacement of the disassembling member and the housing is limited; or
the disassembling member defines a through hole at a center thereof for screws to pass through, and a decorative cover is arranged at an outside of through hole; or
a distance between the anti-hitting member and the impeller is in a range of 0.1mm to 5mm; or
a diameter of the anti-hitting member is in a range of 1mm to 20mm.

8. The portable fan according to claim 1, further comprising: a support member mounted on a side of the housing facing towards the neck; wherein the support member is rotatably connected to the housing and/or connected to the housing via a bendable positioning member; the support member is configured to be supported to a rear of the neck; and
the housing comprises a first side wall facing towards the neck and a second side wall facing away from the neck; a first mating portion is arranged on and protrudes from the first side wall; the support member is arranged with a second mating portion corresponding to the first mating portion; the second mating portion is rotatably connected to the first mating portion; the support member is rotatable relative to the first side wall; or
the first mating portion comprises two pivot portions and an elastic arm disposed between the two pivot portions; a protrusion is arranged at a free end of the elastic arm; the second mating portion comprises a pivot column; a plurality of recesses are defined in a surface of the pivot column; two ends of the pivot column are respectively pivotally connected to the two pivot portions; when the support member is rotating upwardly and downwardly, the protrusion is received in any one of the plurality of recesses in order to position the support member at various angles; or
the second mating portion comprises a mounting bracket; the first mating portion comprises a fixation member secured to the first side wall and a rotation ball that extends through the mounting bracket and is secured to the fixation member; the mounting bracket is rotated with respect to the rotation ball to enable the support member to rotate with respect to the rotation ball; or
the housing comprises two clamp arms that are disposed symmetrically to each other; the two the clamp arms are rotatably connected to each other by a rotation connection member; a pivot angle of each of the two clamp arms is in a range of ±10°; or
the portable fan is arranged with two support members corresponding to the two clamp arms; when the two clamp arms are pivoted, the two support members are configured to be in contact with the neck; or
each of the two clamp arms defines the air inlet, the receiving cavity, the air duct, and the air outlet, wherein the air inlet, the receiving cavity, the air duct, and the air outlet are sequentially communicated with each other; corresponding to each fan assembly, each clamp arm defines a first air inlet defined in the first side wall and a second air inlet defined in the second side wall; the receiving cavity is located at two ends of the two clamp arms that are connected to each other; the support member is disposed corresponding to the receiving cavity; a spacing is defined between at least a portion of the support member and the first air inlet; or
the support member defines a through hole corresponding to the first air inlet; the second side wall comprises a main wall and an auxiliary wall; the main wall and the auxiliary wall are arranged in a stepped manner; the second air inlet is formed between the main wall and the auxiliary wall; or
the rotation connection member comprises a first connection member and a second connection member; the first connection member is fixedly connected to one of the two clamp arms, and the second connection member is fixedly connected to the other one of the two clamp arms; the first connection member comprises a first fixation portion, the second connection member comprises a second fixation portion; the first fixation portion and the second fixation portion are rotatably connected to each other; the first fixation portion and the second fixation portion cooperatively form a spindle shape; the first fixation portion comprises a plurality of first pieces, the second fixation portion comprises a plurality of second pieces; the plurality of the first pieces and the plurality of the second pieces are laminated and arranged alternately with each other; or
a partition assembly is further arranged; the partition assembly separates the receiving cavity and the air duct, which are communicated with each other, from the other space inside the housing; a width of an end of the air duct away from the receiving cavity is less than a width of an end of the air duct near the receiving cavity, and a space is reserved to receive a battery.

9. The portable fan according to claim 1, wherein the housing comprises at least one clamping arm; the air inlet, the receiving cavity, the air duct, and the air outlet are defined in the at least one clamping arm;
a rotation connection structure comprises a first hinge member and a second hinge member; the first hinge member comprises a plurality of first pieces that are arranged in parallel to each other and are spaced apart from each other; the second hinge member comprises a plurality of second pieces that are arranged in parallel to each other and are spaced apart from each other;
the number of the at least one clamping arm is two; and two fan assemblies are arranged corresponding to the two clamping arms; the first hinge member is fixed to one of the two clamp arms; the second hinge member is fixed to the other one of the two clamp arms; the first hinge member and the second hinge member are fixedly mated to each other; the plurality of the first pieces and the plurality of the second pieces are laminated and are arranged alternately with each other, enabling the two clamp arms to be rotated relative to each other and enabling the two clamp arms, when rotating is stopped, to stay static at any stopped position; and
a pivot is further arranged; the pivot extends through the plurality of first pieces and the plurality of the second pieces that are laminated and arranged alternately, so as to secure the first hinge member and the second hinge member; or
one of the first hinge member and the second hinge member is arranged with a pivot portion, and the other one of the first hinge member and the second hinge member defines a pivot hole; the pivot portion is fixed in the pivot hole to secure the first hinge member and the second hinge member; or
two pivot portions and two pivot holes are arranged corresponding to each other; the two pivot portions and the two pivot holes are respectively arranged on opposite sides of the plurality of first pieces and the plurality of second pieces; or
the receiving cavity is located at an end of the clamp arm near the rotation connection structure and receives the fan assembly; the air duct and a wire receiving chamber are defined along a direction extending from the receiving cavity towards a free end of the clamp arm; at least one wire receiving chamber receives a battery; the battery supplies power to the fan assembly; two fan assemblies are configured to operate simultaneously or independently; or
a pivot angle of each clamp arm is in a range of ±10°; or
each of the plurality of first pieces and each of the plurality of second pieces have a same shape, which is irregular polygonal; friction between the first hinge member and the second hinge member when free ends of the two clamp arms being rotated away from each other is greater than friction between the first hinge member and the second hinge member when the free ends of the two clamp arms being rotated to be closer to each other.

10. The portable fan according to claim 1, wherein a shaft tube is arranged inside the housing; a rotation shaft is received in a shaft hole of the shaft tube and rotatable with respect to the shaft tube; a stator sleeves an outside of the shaft tube, and an impeller is connected to a top of the rotation shaft; the portable fan further comprises an anti-vibration member disposed at an end of the shaft tube; the anti-vibration member is configured to absorb vibration of the impeller 2; and
the housing encloses to define a mounting channel having an opening at an end; the impeller is capable of being taken out from the mounting channel; a rotation plate is disposed at the opening of the housing; the rotation plate is detachably connected to the housing;
one of the rotation plate and the housing is arranged with a rotation fastener and/or outer threads and/or a first magnet, and the other one of the rotation plate and the housing is arranged with a rotation fastener groove and/or inner threads and/or a second magnet; or
one of the rotation plate and the housing is arranged with a rotation fastener, and the other one of the rotation plate and the housing is arranged with a rotation fastener groove; the rotation fastener groove has a first position and a second position, and the rotation fastener is configured to slide to switch between the position and the second position;
the rotation plate and the housing are configured to rotate to switch between a first mating position and a second mating position; when the rotation plate and the housing are at the first mating position, the rotation fastener is inserted into the first position of the rotation fastener groove; when the rotation plate and the housing are at the second mating position, the rotation fastener is inserted into the second position of the rotation fastener groove, so as to limit relative displacement of the disassembling member and the housing; or
the rotation plate defines a rotation fastener groove, and the housing is arranged with a rotation fastener;
the rotation plate defines a first recess and a second recess along a radial direction thereof; the first recess and the second recess are communicated to each other; the first position of the rotation fastener groove is corresponding to a position of the first recess; the second position of the rotation fastener groove is corresponding to a position of the second recess; a first resilient fastener is arranged on the rotation plate and protruding from the rotation plate into the second recess;
the housing defines a third recess along the radial direction thereof; a second resilient fastener is arranged at a side of the third recess near the rotation plate; the second resilient fastener is connected with the housing; when the second resilient fastener slides from the first recess to the second recess, an axial relative displacement of the rotation plate and the housing is limited; or
an inner diameter of the anti-vibration member is in a range from 7mm to 13mm, and an outer diameter of the anti-vibration member is in a range from 17mm to 40mm; or
the anti-vibration member and the shaft tube are detachably connected to each other, the anti-vibration member and the housing are detachably connected to each other, the shaft tube is connected to the housing via the anti-vibration member, the anti-vibration member is a circular body; or
an inner periphery of the circular body is snapped with the shaft tube, an end of the shaft tube extending outwardly in a radial direction to form a first protrusion, an inner recess is formed in an inner wall of the circular body, the first protrusion is inserted into the inner recess; or
an outer periphery of the circular body is snapped with the housing, an outer recess is formed in an outer wall of the circular body, a second protrusion is formed from the housing to be snapped with the outer recess, the second protrusion is inserted into the outer recess; or
the circular body is made of a soft material and the circular body is deformable; or
a thickness of the circular body is in a range from 0.9mm to 3mm.

11. The portable fan according to claim 1, wherein
the housing comprises two clamp arms that are disposed symmetrically to each other;
the fan assembly comprises two fan assemblies that are arranged respectively in the two clamp arms and corresponding to a rear of the neck;
a connection member, pivotally connected to the two clamp arms to adjust a distance between free ends of the two clamp arms; wherein
at a mounting position between the connection member and each clamp arms, the connection member has a first mating surface facing the clamp arm, and the clamp arm has a second mating surface facing the connection member; the first mating surface and the second mating surface are two curved surfaces parallel to each other; perpendicular projections of two second mating surfaces of the two clamp arms in a first direction form two arcs, a radius of a circle where each of the two arcs is located is in a range of 7.5mm to 9.5 mm, a distance between centers of two circles where the two arcs are located is in a range of 15mm to 26 mm, so as to limit a distance between the two fan assemblies; an area of air intaken and cooling for the rear of the neck is increased, and turbulence of airflows from the two fan assemblies is prevented; and
the second mating surface is a concave surface, and the clamp arm is arranged with a convex portion; the convex portion is protruding from the second mating surface; the first mating surface is a convex surface, and the connection member defines a snap slot; the snap slot is recessed from the first mating surface; a pivot is connected to and extends through the convex portion and the snap slot, allowing the convex portion and the snap slot to be stably pivotally connected to each other; a size of the snap slot is greater than a size of the convex portion; an inner wall of the snap slot is configured to limit a position of the convex portion during pivoting; or
the first mating surface is a concave surface, and the connection member is arranged with a convex portion; the convex portion is protruding from the first mating surface; the second mating surface is a convex surface; the clamp arm defines a snap slot; the snap slot is recessed from the second mating surface; a pivot is connected to and extends through the convex portion and the snap slot, allowing the convex portion and the snap slot to be stably pivotally connected to each other; a size of the snap slot is greater than a size of the convex portion; an inner wall of the snap slot is configured to limit a position of the convex portion during pivoting; or
a surface of the convex portion is recessed to define a wire slot; the wire slot extends through the second mating surface or the first mating surface; a wall of the snap slot defines a wire hole; the wire slot and the wire hole are communicated to each other to allow a wire in one of the two clamp arms to extend through the wire slot and the wire hole to enter the other one of the two clamp arms; or
each clamp arm comprises a housing; the housing defines a receiving cavity, an air duct, and a battery receiving chamber sequentially along an extension direction of the housing; the receiving cavity is located close to the connection member; the receiving cavity receives the fan assembly; the battery receiving chamber is located at a free end of the housing; at least one battery receiving chamber receives a battery; the battery is configured to supply power to the fan assembly; two fan assemblies in the two clamp arms are configured to operate simultaneously or independently; or
the housing comprises a first housing facing towards the neck and a second housing facing away from the neck; the fan assembly comprises centrifugal fan blades and a motor; each of the first housing and the second housing defines the air inlet 214 corresponding to the centrifugal fan blades; the housing defines the air outlet in a side wall of the air duct; the air inlet, the receiving cavity, the air duct, and the air outlet are sequentially communicated with each other; or
the connection member is arranged with a support portion protruding towards a rear of the neck; the support portion comprises a support bar and a support pad connected to the support bar; the support pad is attached to the neck to separate the connection member from the rear of the neck; or
a shielding plate is arranged at each air inlet; a gap is formed between the shielding plate and the housing to allow external airflow to flow through the gap to reach the air inlet; when the two clamp arms are pivoted to be close to each other, the support pad abuts against the two shielding plates located on a side of the first housing; or
a second direction is perpendicular to the first direction, a length of the support pad in the second direction is greater than a length of the connection member in the second direction; or
a mounting portion and a connection portion connected to the mounting portion and the housing are arranged at each air inlet; the fan assembly is mounted in the mounting portion; the motor is disposed corresponding to the mounting portion; the mounting portion and the connection portion are protruding to form a wire space; or
a wire is electrically connected to the motor to the battery, the wire extends from the motor out of the housing into the wire space and extends from the wire space into the housing.

12. The portable fan according to claim 1, further comprising:
a lithium ion battery, configured to supply power to the portable fan;
a control main board, configured to control the portable fan; and
a battery protection device, provided on the control main board;
wherein the battery protection device is electrically connected to the control main board, pins B+ and B-of the battery protection device are respectively connected to a positive electrode and a negative electrode of the lithium ion battery; or
the battery protection device is integrated into the control main board; or
the battery protection device is mechanically connected to the control main board, a pin P+ of the battery protection device is connected to a pin of the control main board, a pin P- of the battery protection device is connected to another pin of the control main board; the pins B+ and B- of the battery protection device are respectively connected to the positive electrode and the negative electrode of the lithium ion battery; or
the battery protection device is embedded in the control main board; or
the battery protection device is welded to the control main board; or
the battery protection device is screwed to the control main board; or
the battery protection device is riveted to the control main board; or
the battery protection device comprises an intelligent processor, a current collection module and a voltage collection module; the intelligent processor is connected to the current collection module and the voltage collection module; the current collection module and the voltage collection module are connected to a power supply module; or
the power supply module is provided in the control collection, and the pins P+ and P- of the battery protection device are respectively connected to pins of the power supply module.

13. The portable fan according to claim **1,** wherein
the fan assembly comprises: a fan arranged inside the housing and a rotation shaft arranged inside the housing; the fan is configured to rotate around the rotation shaft to produce the airflow; the rotation shaft is inserted into the annular body from a first end of the annular body;
a sealing member is arranged at a second end of the annular body opposite to the first end and is configured to seal the second end of the annular body; and
an oil storage portion is formed between the rotation shaft and the sealing member when the rotation shaft is inserted in the annular body; the oil storage portion is configured to store a lubricant; or
at least a portion of the oil storage portion is radially enlarged in a direction facing the second end; or
a surface of the second end is recessed with respect to a surface of the housing to form a concave portion; the sealing member is arranged in the concave portion; a surface of the sealing member is flush with the surface of the housing; or
the sealing member is made of PE; the sealing member has a thickness of 0.05mm to 0.3 mm; or
a bearing is further arranged; the bearing is disposed between the rotation shaft and the annular body in a radial direction for securing the rotation shaft to the annular body and for enabling the rotation shaft to rotate within the bearing; or
two oil rings are arranged; the two oil rings are arranged on two sides of an axial connection between the rotation shaft and the bearing to seal and maintain the lubricant between the rotation shaft and the bearing to allow the rotation shaft to stably and smoothly rotate; or
a snap ring is further arranged; the snap ring enters the annular body from the second end and is snapped in a snap slot of the rotation shaft; the annular body is arranged with a stopping portion that is inwardly protruding; an outer diameter of the snap ring is greater than an inner diameter of the stopping portion, and the rotation shaft is prevented from being detached off from the annular body; the snap ring is spaced apart, in the axial direction, from the stopping portion to allow the lubricant to flow through; or
a motor driving the fan to rotate is further arranged, the fan comprises a hub and fan blades connected to the hub; when the rotation shaft is inserted in the annular body, a receiving portion is formed between the hub and the housing; the receiving portion receives the motor.

14. The portable fan according to claim 1, wherein,
the housing comprises a connection section and two neck side sections respectively connected to two sides of the connection section; the connection section and the two neck side sections cooperatively define a housing space;
an airflow portion is arranged in each of the connection section and/or the at least one of the two neck side sections; each airflow portion is configured to blow the airflow towards the housing space; and
the airflow portion comprises a positioning protruding post, rotation air blades and a three-phase motor drive assembly that is driveably connected to the rotation air blades; the three-phase motor drive assembly comprises a stator and a rotor sleeving an outside of the stator; the rotor is fixedly mounted on the rotation air blades and is coaxially arranged with the rotation air blades; the stator fixedly sleeves the positioning protruding post; or
the airflow portion is arranged on the connection section; the connection section defines a first air guiding cavity therein; the connection section defines a first air outlet communicated with the first air guiding cavity; the first air outlet faces towards the housing space; the positioning protruding post is received in the first air guiding cavity; the rotation air blades are rotatably received in the first air guiding cavity and configured to blow the airflow to the first air outlet; or
the connection section defines a first air inlet communicated to the first air guiding cavity; the first air inlet faces towards an end of the rotation air blades; the first air outlet faces towards a side of the rotation air blades; or
the rotation air blades define a fixation hole; an axis of the fixation hole coincides with an axis of the rotation air blades; the airflow portion further comprises a rotation shaft; a first end of the rotation shaft is fixedly threaded into the fixation hole; the positioning protruding post defines a positioning hole; an axis of the positioning hole coincides with the axis of the rotation shaft; a second end of the rotation shaft is rotatably received in the positioning hole; or
a plurality of first air guiding ribs are arranged in the connection section; the plurality of first air guiding ribs cooperatively define the first air guiding cavity; a plurality of first air outlets are defined; the plurality of first air outlets are spaced apart from each other and are located along an extension direction of the connection section; an end of the first air guiding cavity is communicated with the plurality of first air outlets; or
at least one airflow portion comprises a bearing portion; an outer ring of the bearing portion is fixed in the positioning hole; an inner ring of the bearing portion sleeves the second end of the rotation shaft;
the bearing portion comprises a rolling bearing; the airflow portion comprises a limiting member; the limiting member is mounted on the second end of the rotation shaft; the bearing portion is disposed between the limiting member and the first end of the rotation shaft; a plurality of bearing portions are arranged; an inner flange is arranged on an inner wall of the positioning hole; the inner flange is disposed between two adjacent bearing portions to enable the two adjacent bearing portions to be spaced apart from each other; or the bearing portion comprises a slide bearing; the airflow portion comprises two sealing rings; the two sealing rings both sleeve the rotation shaft and are respectively located on two sides of the slide bearing; or
each of the two neck side sections is arranged with one respective airflow portion; a second air guiding cavity is defined in each neck side section; the neck side section defines a second air outlet communicated with the second air guiding cavity; the second air outlet faces towards the housing space; the positioning protruding post is received in the second air guiding cavity; the rotation air blades are rotatably mounted in the second air guiding cavity and are configured to blow the airflow to the second air outlet; or
the neck side section defines a second air inlet communicated to the second air guiding cavity; the second air inlet faces an end of the rotation air blades, and the second air outlet faces a side of the rotation air blades; or
the fan further comprises a power supply portion arranged inside the housing; the power supply portion is electrically connected to each airflow portion; the housing is arranged with a charging port that is electrically connected to the power supply portion.
